# EUROPÄISCHE PATENTANMELDUNG

(11) **EP 3 838 902 A1**
(43) Veröffentlichungstag der Anmeldung: **23.06.2021**
(21) Anmeldenummer: 20208213.7
(22) Anmeldetag: 22.06.2012
(51) Int. Cl.: C07D 475/14, A61P 31/00, A61Q 11/00, A61Q 17/00

(54) **10H-BENZO[G]PTERIDIN-2,4-DION-DERIVATE, VERFAHREN ZUR HERSTELLUNG UND VERWENDUNG DERSELBEN**

(30) Priorität: 22.06.2011 DE 102011105657
(62) Teilanmeldung aus: 12728621.9
(71) Anmelder: TriOptoTec GmbH, 93053 Regensburg (DE)
(72) Erfinder: MAISCH, Tim, 90425 Nürnberg (DE); SPÄTH, Andreas, 93055 Regensburg (DE)
(74) Vertreter: Walcher, Armin

(57) **Zusammenfassung**

Die vorliegende Erfindung betrifft 10H-Benzo[g]pteridin-2,4-dion-Derivate, deren Herstellung und Verwendung.

## Beschreibung

Die vorliegende Erfindung betrifft 10H-Benzo[g]pteridin-2,4-dion-Derivate, deren Herstellung und Verwendung.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erreger-bedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Neue Ansatzpunkt zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei verschiedene photooxidative Prozesse eine entscheidende Rolle. Voraussetzung für den Ablauf einer photooxidativen Inaktivierung ist einerseits das Vorhandensein einer ausreichenden Menge Sauerstoff und andererseits die Lokalisierung eines so genannten Photosensibilisators, welcher durch Licht einer entsprechenden Wellenlänge angeregt wird. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff alle Moleküle angegriffen. Besonders anfällig für eine Schädigung sind allerdings ungesättigte Fettsäuren in den Membranen von Bakterien. Gesunde, körpereigene Zellen verfügen über eine zelluläre Abwehr gegen Angriffe von freien Radikalen, die so genannten Katalasen oder Superoxiddismutasen. Daher können gesunde, körpereigene Zellen eine Schädigung durch reaktive Sauerstoffspezies (ROS), beispielsweise Radikale oder Singulett-Sauerstoff, entgegenwirken.

Aus dem Stand der Technik sind zahlreiche Photosensibilisatoren bekannt, die beispielsweise aus der Gruppe der Porphyrine und deren Derivate oder Phthalocyanine und deren Derivate oder Fullerene und deren Derivate oder Derivate der Phenothiazininium-Struktur, wie beispielsweise Methylenblau oder Toluidinblau, oder Vertreter der Phenoxazinium-Reihe, wie beispielsweise Nile blue, stammen. Die Photodynamik von Methylenblau bzw. Toluidinblau gegenüber Bakterien wird beispielsweise bereits in der Zahnheilkunde eingesetzt.

Bei den aus dem Stand der Technik bekannten Photosensibilisatoren handelt es sich zumeist um Substanzen mit einer relativ komplexen Molekülstruktur und daher aufwendigen Herstellungsverfahren.

Die Nicht-Patentliteratur Svoboda et al. (Chem.Eur.J. 2008, 14, Seiten 1854 - 1864) beschreibt den Einfluß von Thioharnstoff auf die Photooxidation von Benzylalkohol durch Flavin und verschiedenen Flavinderivaten, darunter die Verbindung mit der Formel (20):

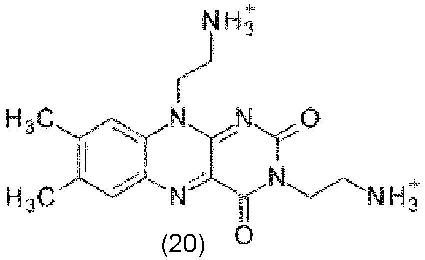

Allerdings wird in Svoboda et al. keine Wirkung der untersuchten Flavinderivate auf die Lebensfähigkeit von Mikroorganismen beschrieben.

Es ist weiterhin bekannt, dass 10-Methyl-10H-benzo[g]pteridine-2,4-dion-Derivate, Riboflavin und Tetraacetylriboflavin hohe Ausbeuten an Singulett-Sauerstoff haben, wobei jedoch die Affinität zu Mikroorganismen gering ist. Es ist weiterhin bekannt, dass Singulett-Sauerstoff lediglich über eine geringe Entfernung diffundieren kann, bevor er reagiert oder abgebaut wird. Daher ist die Inaktivierung von Mikroorganismen durch 10-Methyl-10H-benzo[g]pteridine-2,4-dion-Derivate, Riboflavin und Tetraacetylriboflavin unzureichend.

Weiterhin sind aus der WO 2010/019208 A1 und der WO 2011/008247 A1 zahlreiche Flavin-, Roseoflavin- und Riboflavin-Derivate bekannt, die an Flavinmononucleotid (FMN) Riboswiches binden können. Riboswiches sind RNA-Elemente in untranslatierten Regionen der mRNA von Prokaryoten, Pilzen und Pflanzen, die niedermolekulare Metabolite, beispielsweise FMN, binden und daraufhin die Genexpression regulieren. Beispielsweise wird nach Bindung von FMN an FMN-Riboswiches von Prokaryoten die Expression von Enzymen, die für die Riboflavin- und FMN-Biosynthese verantwortlich sind, reprimiert, wodurch die Riboflavin- und FMN-Biosynthese zum Erliegen kommt. Riboflavin nimmt im Stoffwechsel eine zentrale Rolle ein, da es als Vorstufe für Flavin-Koenzyme dient. Daher führt eine unterdrückte Riboflavin- und FMN-Biosynthese zu einer reduzierten Überlebensfähigkeit.

Allerdings kann es bei dieser Form der Bekämpfung von pathogenen Mikroorganismen ebenfalls zum Auftreten von Resistenzen kommen, die beispielsweise durch Mutationen der entsprechenden RNA-Elemente entstehen können.

Aufgabe der vorliegenden Erfindung ist es daher, neue Photosensibilisatoren bereitzustellen, die Mikroorganismen effizienter inaktivieren.

Die Aufgabe der der vorliegenden Erfindung wird gelöst durch die Bereitstellung einer Verbindung mit der Formel (1): 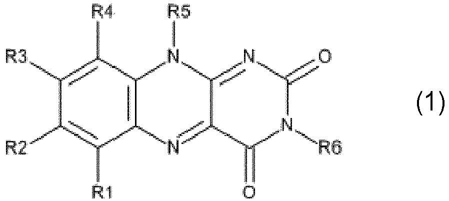
wobei I) nur 1 Rest R1, R2, R3, R4, R5 oder R6 ein organischer Rest mit:
   a) wenigstens zwei neutralen, protonierbaren Stickstoffatomen, die nicht direkt an den Isoalloxazinring gebunden sind, oder
   b) wenigstens zwei positiv geladenen, vorzugsweise quartären, Stickstoffatomen, die nicht direkt an den Isoalloxazinring gebunden sind, bedeutet
   und wobei jeder der Reste R1, R2, R3 oder R4, der kein organischer Rest mit a) wenigstens zwei neutralen, protonierbaren Stickstoffatomen, die nicht direkt an den Isoalloxazinring gebunden sind, oder b) wenigstens zwei positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens zwei neutralen, protonierbaren Stickstoffatomen, die nicht direkt an den Isoalloxazinring gebunden sind, oder b) wenigstens zwei positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
   mit der Maßgabe, dass der Rest R3 kein Aminomethyl-Rest, wobei das Stickstoffatom unsubstituiert oder substituiert sein kann, bedeutet, und
   wobei Verbindungen ausgenommen sind, bei denen die Reste R1, R4 und R10 Wasserstoff sind, der Rest R2 Wasserstoff, Alkyl mit 1 bis 8 C-Atomen oder Cycloalkyl mit 3 bis 7 C-Atomen bedeutet, der Rest R3 Wasserstoff, Halogen, Alkyl mit 1 bis 8 C-Atomen, O-Alkyl mit 1 bis 8 C-Atomen oder ein heterocyclischer Rest mit 4 bis 7 C-Atomen bedeutet und der Rest R5 ein Alkylrest mit 1 bis 8 C-Atomen ist, der mit wenigstens mit einem Rest der Formel -N(R³⁵)(R³⁶) oder einem Rest der Formel -OR³⁵ substituiert ist, wobei der Rest R³⁵ ein Rest der Formel -C₁₋₈Alkyl(amin)-OR³⁷ oder ein 7,8-dimethyl-isoalloxazin-10-yl-C₁₋₈alkylrest bedeutet und wobei der Rest R³⁶ Wasserstoff oder ein Rest der Formel -C₁₋₈Alkyl, der unsubstituiert oder substituiert sein kann, bedeutet und wobei der Rest R³⁷ Wasserstoff, Aryl mit 6 bis 7 C-Atomen oder Alkyl, das unsubstituiert oder substituiert sein kann, mit 1 bis 8 C-Atomen bedeutet, und
   wobei weiterhin Verbindungen ausgenommen sind, bei denen die Reste R1 und R4 Wasserstoff bedeuten, der Rest R10 Wasserstoff oder ein Rest mit der allgemeinen Formel -C₁₋₄Alkyl-OC(O)CH₃ bedeutet, der Rest R2 Wasserstoff oder Alkyl mit 8 bis 8 C-Atomen bedeutet und der Rest R5 ein Rest mit der allgemeinen Formel -C₁₋₆Alkyl-N(R³¹)-C₀₋₃Alkyl-(R³²) bedeutet, wobei R³¹ Wasserstoff oder ein Rest mit der Formel -C₁₋₄Alkyl bedeutet und wobei R³² ein Rest mit der Formel -C₁₋₄Alkyl-N(R³³)(R³⁴), ein Rest mit der Formel -C₀₋₄Alkyl-Aryl, ein Rest mit der Formel -C₀₋₄Alkyl-Heterocycloalkyl oder ein Rest mit der Formel -C₀₋₄Alkyl-Heteroaryl bedeutet, und wobei die Reste R³³ und R³⁴ unabhängig voneinander Wasserstoff oder -C₁₋₄Alkyl bedeuten, und
   wobei weiterhin 10-Butyl-7,8-dimethyl-3-[2-oxo-2-(1,4,7,10-tetrazacyclododec-1-yl)ethyl]benzo[g]pteridin-2,4-dion und 10-[2-(2-Methoxyethoxy)ethyl]-7,8-dimethyl-3-[2-oxo-2-(1,4,7,10-tetrazacyclododec-1-yl)ethyl]benzo[g]pteridin-2,4-dion ausgenommen sind,
   oder
wobei II) nur 1 Rest R1, R2, R3, R4, R5 oder R6 ein organischer Rest mit:
   a) wenigstens einem neutralen, protonierbaren Stickstoffatom, das nicht direkt an den Isoalloxazinring gebunden ist, und
   b) wenigstens einem positiv geladenen, vorzugsweise quartären, Stickstoffatom, das nicht direkt an den Isoalloxazinring gebunden ist, bedeutet
   und wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens einem neutralen, protonierbaren Stickstoffatom, das nicht direkt an den Isoalloxazinring gebunden ist, und b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
wobei III) wenigstens 2 Reste R1, R2, R3, R4, R5 oder R6 ein organischer Rest mit:
   a) wenigstens einem neutralen, protonierbaren Stickstoffatom, das nicht direkt an den Isoalloxazinring gebunden ist, und/oder
   b) wenigstens einem positiv geladenen, vorzugsweise quartären, Stickstoffatom, das nicht direkt an den Isoalloxazinring gebunden ist, bedeutet und
   wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und/oder b) wenigstens einem positiv geladenen , vorzugsweise quartären, Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens einem neutralen, protonierbaren Stickstoffatom, das nicht direkt an den Isoalloxazinring gebunden ist, und/oder b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet
   und wobei die Verbindung mit der Formel (20): 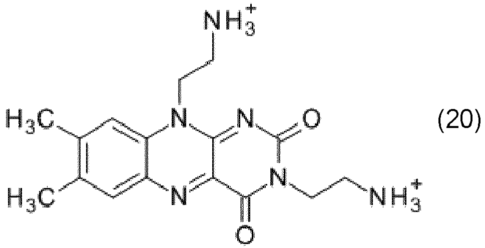 ausgenommen ist.

Die Aufgabe der der vorliegenden Erfindung wird ebenfalls gelöst durch die Verwendung einer Verbindung mit der Formel (1): 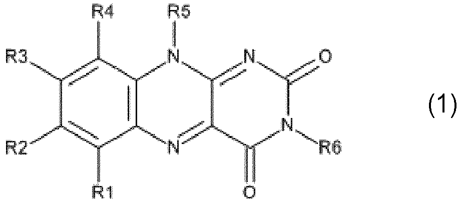 als Photosensibilisator. vorzugsweise bei der photodynamischen Inaktivierung von Mikroorganismen, weiter bevorzugt bei der photodynamischen Therapie,
wobei I) nur 1 Rest R1, R2, R3, R4, R5 oder R6 ein organischer Rest mit:
   a) wenigstens zwei neutralen, protonierbaren Stickstoffatomen, die nicht direkt an den Isoalloxazinring gebunden sind, oder
   b) wenigstens zwei positiv geladenen, vorzugsweise quartären, Stickstoffatomen, die nicht direkt an den Isoalloxazinring gebunden sind, bedeutet
   und wobei jeder der Reste R1, R2, R3 oder R4, der kein organischer Rest mit a) wenigstens zwei neutralen, protonierbaren Stickstoffatomen, die nicht direkt an den Isoalloxazinring gebunden sind, oder b) wenigstens zwei positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens zwei neutralen, protonierbaren Stickstoffatomen, die nicht direkt an den Isoalloxazinring gebunden sind, oder b) wenigstens zwei positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
wobei II) nur 1 Rest R1, R2, R3, R4, R5 oder R6 ein organischer Rest mit:
   a) wenigstens einem neutralen, protonierbaren Stickstoffatom, das nicht direkt an den Isoalloxazinring gebunden ist, und
   b) wenigstens einem positiv geladenen, vorzugsweise quartären, Stickstoffatom, das nicht direkt an den Isoalloxazinring gebunden ist, bedeutet
   und wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens einem neutralen, protonierbaren Stickstoffatom, das nicht direkt an den Isoalloxazinring gebunden ist, und b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
wobei III) wenigstens 2 Reste R1, R2, R3, R4, R5 oder R6 ein organischer Rest mit:
   a) wenigstens einem neutralen, protonierbaren Stickstoffatom, das nicht direkt an den Isoalloxazinring gebunden ist, und/oder
   b) wenigstens einem positiv geladenen, vorzugsweise quartären, Stickstoffatom, das nicht direkt an den Isoalloxazinring gebunden ist, bedeutet und
   wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und/oder b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens einem neutralen, protonierbaren Stickstoffatom, das nicht direkt an den Isoalloxazinring gebunden ist, und/oder b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet.

Bei der Verbindung mit der Formel (1) handelt es sich um ein 10H-Benzo[g]pteridin-2,4-dion- oder Flavin-Derivat, das im Folgenden auch so bezeichnet wird.

Als Gegenion zu wenigstens einen positiv geladenen, vorzugsweise quartären, Stickstoffatom kann jedes geeignete Anion verwendet werden. Vorzugsweise werden als Gegenion(en) zu(m) positiv geladenen, vorzugsweise quartären, Stickstoffatom(en) Anionen verwendet, die die Bereitstellung eines pharmakologisch verträglichen Salzes ermöglichen.

Bei einer bevorzugten Ausführungsform der vorliegenden Erfindung weisen positiv geladene, vorzugsweise quartäre, Stickstoffatome als Gegenion Fluorid, Chlorid, Bromid, lodid, Sulfat, Hydrogensulfat, Phosphat, Dihydrogenphosphat, Hydrogenphosphat, Tosylat, Mesylat, Formiat, Acetat, Oxalat, Benzoat, Citrat und/oder Mischungen davon aufweist.

Die vorliegende Anmeldung offenbart ebenfalls ein Verfahren zur Herstellung des erfindungsgemäßen 10H-Benzo[g]pteridin-2,4-dion-Derivats der Formel (1).

Weitere bevorzugte Ausführungsformen der vorliegenden Erfindung sind in den abhängigen Ansprüchen beschrieben.

Als "Photosensibilisator" werden erfindungsgemäß Verbindungen verstanden, die elektromagnetische Strahlung, vorzugsweise sichtbares Licht, UV-Licht und/oder Infrarotlicht, absorbieren und sodann reaktive Sauerstoffspezies (ROS), vorzugsweise freie Radikale und/oder Singulett-Sauerstoff, aus Triplett-Sauerstoff erzeugen.

Unter dem Begriff "photodynamische Therapie" wird erfindungsgemäß die lichtinduzierte, photodynamische, Inaktivierung von Zellen oder Mikroorganismen verstanden.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäß verwendeten Verbindung mit der Formel (1) bestimmt werden.

Erfindungsgemäß wird unter einer Reduzierung der Lebensfähigkeit verstanden, dass die Anzahl der Mikroorganismen um wenigstens 99,0 %, vorzugsweise um wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 %, verringert.

Vorzugsweise wird die Reduzierung der Anzahl der Mikroorganismen gemäß Boyce, J.M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.Infect.Control 30 (8), 2002, Seite 1 - 46) als log₁₀-Reduktionsfaktor angegeben.
Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens einer erfindungsgemäß verwendeten Verbindung mit der Formel (1) verstanden.

Geeignete Methoden zur Bestimmung des log₁₀-Reduktionsfaktors sind beispielsweise in der DIN EN 14885:2007-01 "Chemische Desinfektionsmittel und Antiseptika - Anwendung Europäischer Normen für chemische Desinfektionsmittel und Antiseptika" oder in Rabenau, H.F. und Schwebke, I. ("Leitlinie der Deutschen Vereinigung zur Bekämpfung der Viruskrankheiten (DVV) e.V. und des Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitschutz 51(8), (2008), Seiten 937 - 945) beschrieben.

Vorzugsweise beträgt der log₁₀-Reduktionsfaktor nach einer Bestrahlung von Mikroorganismen mit elektromagnetischer Strahlung in Gegenwart wenigstens einer erfindungsgemäß verwendeten Verbindung mit der Formel (1) mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀, noch weiter bevorzugt mindestens 7,5 log₁₀.

Beispielsweise bedeutet eine Reduktion der Anzahl der Mikroorganismen nach einer Bestrahlung dieser Mikroorganismen mit elektromagnetischer Strahlung in Gegenwart wenigstens einer erfindungsgemäß verwendeten Verbindungen mit der Formel (1) um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen mit elektromagnetischer Strahlung in Gegenwart wenigstens einer erfindungsgemäß verwendeten Verbindung mit der Formel (1) um mindestens 1 Zehnerpotenz, weiter bevorzugt um mindestens 2 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Bakterien und Bakteriensporen, und eukaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Ein erfindungsgemäßes 10H-Benzo[g]pteridin-2,4-dion-Derivat weist die Formel (1) auf, 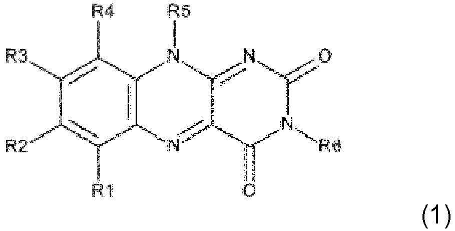
wobei I) nur 1 Rest R1, R2, R3, R4, R5 oder R6 ein organischer Rest mit:
   a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
   b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet oder
wobei II) nur 1 Rest R1, R2, R3, R4, R5 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet oder
wobei III) wenigstens 2 Reste R1, R2, R3, R4, R5 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und/oder
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), bedeutet.

Bei einer bevorzugten Ausführungsform weist ein erfindungsgemäßes 10H-Benzo[g]pteridin-2,4-dion-Derivat der Formel (1) kein neutrales, protonierbares Stickstoffatom, das direkt an den Isoalloxazinring gebunden ist, beispielsweise als Amino-Rest, Methylamino-Rest oder Dimethylamino-Rest, und kein positiv geladenes Stickstoffatom, das direkt an den Isoalloxazinring gebunden ist, beispielsweise als Pyridin-1-ium-1-yl-Rest oder Trimethylammonio-Rest, auf.

Bei der Variante I) des erfindungsgemäßen 10H-Benzo[g]pteridin-2,4-dion-Derivats der Formel (1), 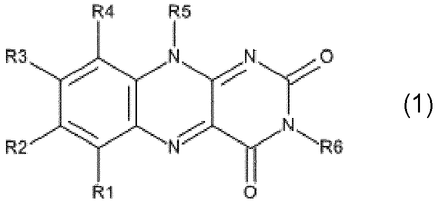
ist nur 1 Rest R1, R2, R3, R4, R5 oder R6 ein organischer Rest mit:
   a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
   b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind,und
wobei jeder der Reste R1, R2, R3 oder R4, der kein organischer Rest mit a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
wobei jeder der Reste R5 oder R6, der kein organischer Rest mit a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
mit der Maßgabe, dass der Rest R3 kein Aminomethyl-Rest, wobei das Stickstoffatom unsubstituiert oder substituiert sein kann, bedeutet, und
wobei Verbindungen ausgenommen sind, bei denen die Reste R1, R4 und R10 Wasserstoff sind, der Rest R2 Wasserstoff, Alkyl mit 1 bis 8 C-Atomen oder Cycloalkyl mit 3 bis 7 C-Atomen bedeutet, der Rest R3 Wasserstoff, Halogen, Alkyl mit 1 bis 8 C-Atomen, O-Alkyl mit 1 bis 8 C-Atomen oder ein heterocyclischer Rest mit 4 bis 7 C-Atomen bedeutet und der Rest R5 ein Alkylrest mit 1 bis 8 C-Atomen ist, der mit wenigstens mit einem Rest der Formel -N(R³⁵)(R³⁶) oder einem Rest der Formel -OR³⁵ substituiert ist, wobei der Rest R³⁵ ein Rest der Formel -C₁₋₈Alkyl(amin)-OR³⁷ oder ein 7,8-dimethyl-isoalloxazin-1 0-yl-C₁₋₈alkylrest bedeutet und wobei der Rest R³⁶ Wasserstoff oder ein Rest der Formel -C₁₋₈Alkyl, der unsubstituiert oder substituiert sein kann, bedeutet und wobei der Rest R³⁷ Wasserstoff, Aryl mit 6 bis 7 C-Atomen oder Alkyl, das unsubstituiert oder substituiert sein kann, mit 1 bis 8 C-Atomen bedeutet, und
wobei weiterhin Verbindungen ausgenommen sind, bei denen die Reste R1 und R4 Wasserstoff bedeuten, der Rest R10 Wasserstoff oder ein Rest mit der allgemeinen Formel -C₁₋₄Alkyl-OC(O)CH₃ bedeutet, der Rest R2 Wasserstoff oder Alkyl mit 8 bis 8 C-Atomen bedeutet und der Rest R5 ein Rest mit der allgemeinen Formel -C₁₋₆Alkyl-N(R³¹)-C₀₋₃Alkyl-(R³²) bedeutet, wobei R³¹ Wasserstoff oder ein Rest mit der Formel -C₁₋₄Alkyl bedeutet und wobei R³² ein Rest mit der Formel -C₁₋₄Alkyl-N(R³³)(R³⁴), ein Rest mit der Formel -C₀₋₄Alkyl-Aryl, ein Rest mit der Formel -C₀₋₄Alkyl-Heterocycloalkyl oder ein Rest mit der Formel -C₀₋₄Alkyl-Heteroaryl bedeutet, und wobei die Reste R³³ und R³⁴ unabhängig voneinander Wasserstoff oder -C₁₋₄Alkyl bedeuten und
wobei 10-Butyl-7,8-dimethyl-3-[2-oxo-2-(1,4,7,10-tetrazacyclododec-1-yl)ethyl]benzo[g]pteridin-2,4-dion und 10-[2-(2-Methoxyethoxy)ethyl]-7,8-dimethyl-3-[2-oxo-2-(1,4,7,10-tetrazacyclododec-1-yl)ethyl]benzo[g]pteridin-2,4-dion ausgenommen sind.

Bei einer bevorzugten Ausführungsform der Variante I) des 10H-Benzo[g]pteridin-2,4-dion-Derivats der Formel (1) ist der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet und wobei
A) nur 1 Rest R1, R2, R3 oder R4 ein organischer Rest mit:
   a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
   b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet,
   wobei jeder der Reste R1, R2, R3 oder R4, der kein organischer Rest mit a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei der Reste R6 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
   oder
B) nur der Reste R6 in organischer Rest mit:
   a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
   b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet,
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform der Variante I) des erfindungsgemäßen 10H-Benzo[g]pteridin-2,4-dion-Derivats der Formel (1) ist
A) nur 1 Rest R1, R2, R3 oder R4 ein organischer Rest der allgemeinen Formel (2), (3) oder (4): 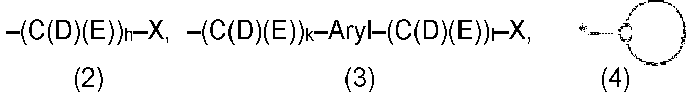
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest mit
      a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
      b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet, vorzugsweise mit der Maßgabe, dass der Rest R3 kein Aminomethyl-Rest, wobei das Stickstoffatom unsubstituiert oder substituiert sein kann, bedeutet, und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) wenigstens zwei, vorzugsweise wenigstens drei, neutrale, protonierbare Stickstoffatome, die nicht direkt an den Isoalloxazinring gebunden sind, oder b) wenigstens zwei, vorzugsweise wenigstens drei, positiv geladene, vorzugsweise quartären, Stickstoffatome, die nicht direkt an den Isoalloxazinring gebunden sind, enthält, bedeutet,
   wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei jeder der Reste R5 oder R6 jeweils unabhängig voneinander gleich oder verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet,
      oder
B) nur 1 Rest R5 oder R6 ein organischer Rest der allgemeinen Formel (2), (3) oder (4): 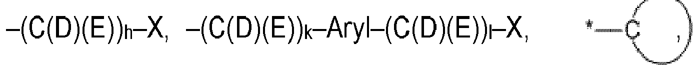 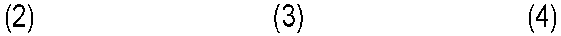
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit
      a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
      b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) wenigstens zwei, vorzugsweise wenigstens drei, neutrale, protonierbare Stickstoffatome, die nicht direkt an den Isoalloxazinring gebunden sind, oder b) wenigstens zwei, vorzugsweise wenigstens drei, positiv geladene, vorzugsweise quartären, Stickstoffatome, die nicht direkt an den Isoalloxazinring gebunden sind, enthält, bedeutet und
   wobei der Rest R5 oder R6, der nicht ein organischer Rest der allgemeinen Formel (2), (3) oder (4) ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten, und
   wobei jeweils Verbindungen ausgenommen sind, bei denen die Reste R1, R4 und R10 Wasserstoff sind, der Rest R2 Wasserstoff, Alkyl mit 1 bis 8 C-Atomen oder Cycloalkyl mit 3 bis 7 C-Atomen bedeutet, der Rest R3 Wasserstoff, Halogen, Alkyl mit 1 bis 8 C-Atomen, O-Alkyl mit 1 bis 8 C-Atomen oder ein heterocyclischer Rest mit 4 bis 7 C-Atomen bedeutet und der Rest R5 ein Alkylrest mit 1 bis 8 C-Atomen ist, der mit wenigstens mit einem Rest der Formel -N(R³⁵)(R³⁶) oder einem Rest der Formel -OR³⁵ substituiert ist, wobei der Rest R³⁵ ein Rest der Formel -C₁₋₈Alkyl(amin)-OR³⁷ oder ein 7,8-dimethyl-isoalloxazin-10-yl-C₁₋₈alkylrest bedeutet und wobei der Rest R³⁶ Wasserstoff oder ein Rest der Formel -C₁₋₈Alkyl, der unsubstituiert oder substituiert sein kann, bedeutet und wobei der Rest R³⁷ Wasserstoff, Aryl mit 6 bis 7 C-Atomen oder Alkyl, das unsubstituiert oder substituiert sein kann, mit 1 bis 8 C-Atomen bedeutet, und
   wobei weiterhin jeweils Verbindungen ausgenommen sind, bei denen die Reste R1 und R4 Wasserstoff bedeuten, der Rest R10 Wasserstoff oder ein Rest mit der allgemeinen Formel -C₁₋₄Alkyl-OC(O)CH₃ bedeutet, der Rest R2 Wasserstoff oder Alkyl mit 8 bis 8 C-Atomen bedeutet und der Rest R5 ein Rest mit der allgemeinen Formel -C₁₋₆Alkyl-N(R³¹)-C₀₋₃Alkyl-(R³²) bedeutet, wobei R³¹ Wasserstoff oder ein Rest mit der Formel -C₁₋₄Alkyl bedeutet und wobei R³² ein Rest mit der Formel -C₁₋₄Alkyl-N(R³³)(R³⁴), ein Rest mit der Formel -C₀₋₄Alkyl-Aryl, ein Rest mit der Formel -C₀₋₄Alkyl-Heterocycloalkyl oder ein Rest mit der Formel -C₀₋₄Alkyl-Heteroaryl bedeutet, und wobei die Reste R³³ und R³⁴ unabhängig voneinander Wasserstoff oder -C₁₋₄Alkyl bedeuten.

Bei einer weiter bevorzugten Ausführungsform ist der organische Rest mit a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, ein Rest der Formel (2) oder (3): 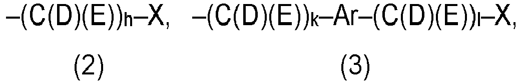 wobei h eine ganze Zahl von 6 bis 20, vorzugsweise von 6 bis 19, vorzugsweise von 8 bis 17, weiter bevorzugt von 8 bis 15, weiter bevorzugt von 8 bis 13, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) wenigstens zwei, vorzugsweise wenigstens drei, vorzugsweise wenigstens vier, weiter bevorzugt wenigstens fünf, neutrale, protonierbare Stickstoffatome, oder b) wenigstens zwei, vorzugsweise wenigstens drei, vorzugsweise wenigstens vier, weiter bevorzugt wenigstens fünf, positiv geladene, vorzugsweise quartäre, Stickstoffatome, enthält, und der Rest Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, wobei vorzugsweise der Rest R3 kein Aminomethyl-Rest, wobei das Stickstoffatom unsubstituiert oder substituiert sein kann, bedeutet und wobei jeweils Verbindungen ausgenommen sind, bei denen die Reste R1, R4 und R10 Wasserstoff sind, der Rest R2 Wasserstoff, Alkyl mit 1 bis 8 C-Atomen oder Cycloalkyl mit 3 bis 7 C-Atomen bedeutet, der Rest R3 Wasserstoff, Halogen, Alkyl mit 1 bis 8 C-Atomen, O-Alkyl mit 1 bis 8 C-Atomen oder ein heterocyclischer Rest mit 4 bis 7 C-Atomen bedeutet und der Rest R5 ein Alkylrest mit 1 bis 8 C-Atomen ist, der mit wenigstens mit einem Rest der Formel -N(R³⁵)(R³⁶) oder einem Rest der Formel -OR³⁵ substituiert ist, wobei der Rest R³⁵ ein Rest der Formel -C₁₋₈Alkyl(amin)-OR³⁷ oder ein 7,8-dimethyl-isoalloxazin-10-yl-C₁₋₈alkylrest bedeutet und wobei der Rest R³⁶ Wasserstoff oder ein Rest der Formel -C₁₋₈Alkyl, der unsubstituiert oder substituiert sein kann, bedeutet und wobei der Rest R³⁷ Wasserstoff, Aryl mit 6 bis 7 C-Atomen oder Alkyl, das unsubstituiert oder substituiert sein kann, mit 1 bis 8 C-Atomen bedeutet, und wobei weiterhin jeweils Verbindungen ausgenommen sind, bei denen die Reste R1 und R4 Wasserstoff bedeuten, der Rest R10 Wasserstoff oder ein Rest mit der allgemeinen Formel -C₁₋₄Alkyl-OC(O)CH₃ bedeutet, der Rest R2 Wasserstoff oder Alkyl mit 8 bis 8 C-Atomen bedeutet und der Rest R5 ein Rest mit der allgemeinen Formel -C₁₋₆Alkyl-N(R³¹)-C₀₋₃Alkyl-(R³²) bedeutet, wobei R³¹ Wasserstoff oder ein Rest mit der Formel -C₁₋₄Alkyl bedeutet und wobei R³² ein Rest mit der Formel -C₁₋₄Alkyl-N(R³³)(R³⁴), ein Rest mit der Formel -C₀₋₄Alkyl-Aryl, ein Rest mit der Formel -C₀₋₄Alkyl-Heterocycloalkyl oder ein Rest mit der Formel -C₀₋₄Alkyl-Heteroaryl bedeutet, und wobei die Reste R³³ und R³⁴ unabhängig voneinander Wasserstoff oder -C₁₋₄Alkyl bedeuten.

Bei einer weiteren bevorzugten Ausführungsform der Variante I) des 10H-Benzo[g]pteridin-2,4-dion-Derivats der Formel (1) ist der Rest R5 ein nichtcyclischer Polyolrest der allgemeinen Formel-CH₂(CH(OH))_{g}CH₂OH oder ein Ether, Ester oder Acetal davon, wobei g eine ganze Zahl von 1 bis 10, vorzugsweise 1 bis 4, und wobei entweder
A) nur 1 Rest R1, R2, R3 oder R4 ein organischer Rest der allgemeinen Formel (2), (3) oder (4): 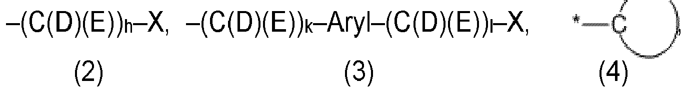 bedeutet,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest mit
      a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
      b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet, vorzugsweise mit der Maßgabe, dass der Rest R3 kein Aminomethyl-Rest, wobei das Stickstoffatom unsubstituiert oder substituiert sein kann, bedeutet, und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) wenigstens zwei, vorzugsweise wenigstens drei, neutrale, protonierbare Stickstoffatome, die nicht direkt an den Isoalloxazinring gebunden sind, oder b) wenigstens zwei, vorzugsweise wenigstens drei, positiv geladene, vorzugsweise quartären, Stickstoffatome, die nicht direkt an den Isoalloxazinring gebunden sind, enthält, bedeutet,
   wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei der Rest R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet,
      oder
B) nur der Rest R6 ein organischer Rest der allgemeinen Formel (2), (3) oder (4): 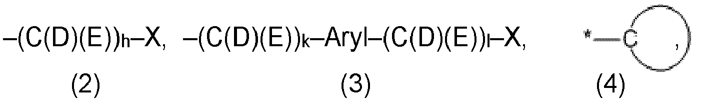 bedeutet,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest mit
      a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
      b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) wenigstens zwei, vorzugsweise wenigstens drei, neutrale, protonierbare Stickstoffatome, die nicht direkt an den Isoalloxazinring gebunden sind, oder b) wenigstens zwei, vorzugsweise wenigstens drei, positiv geladene, vorzugsweise quartären, Stickstoffatome, die nicht direkt an den Isoalloxazinring gebunden sind, enthält, bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Bei einer weiter bevorzugten Ausführungsform der Variante I) des 10H-Benzo[g]pteridin-2,4-dion-Derivats der Formel (1) ist der Rest R5 ein nichtcyclischer Polyolrest der allgemeinen Formel-CH₂(CH(OH))_{g}CH₂OH oder ein Ether, Ester oder Acetal davon, wobei g eine ganze Zahl von 1 bis 10, vorzugsweise 1 bis 4, und wobei entweder
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X, bedeutet
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei X ein organischer Rest ist, der a) wenigstens zwei, vorzugsweise wenigstens drei, vorzugsweise wenigstens vier, weiter bevorzugt wenigstens fünf, neutrale, protonierbare Stickstoffatome, die nicht direkt an den Isoalloxazinring gebunden sind, oder b) wenigstens zwei, vorzugsweise wenigstens drei, vorzugsweise wenigstens vier, weiter bevorzugt wenigstens fünf, positiv geladene, vorzugsweise quartäre,Stickstoffatome, die nicht direkt an den Isoalloxazinring gebunden sind, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
   wobei die Reste R1, R2, R3 oder R4, die nicht ein organischer Rest der allgemeinen Formel -(C(D)(E))ₕ-X oder -(C(D)(E))ₖ-Aryl-(C(D)(E))ₗ-X sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet,
   oder
B) nur der Rest R6 ein organischer Rest der allgemeinen Formel -(C(D)(E))ₕ-X oder -(C(D)(E))ₖ-Aryl-(C(D)(E))ₗ-X bedeutet,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet,oder Thiol, weiter bevorzugt Wasserstoff oder Hydroxyl, bedeuten, wobei X ein organischer Rest ist, der a) wenigstens zwei, vorzugsweise wenigstens drei, vorzugsweise wenigstens vier, weiter bevorzugt wenigstens fünf, neutrale, protonierbare Stickstoffatome, die nicht direkt an den Isoalloxazinring gebunden sind, oder b) wenigstens zwei, vorzugsweise wenigstens drei, vorzugsweise wenigstens vier, weiter bevorzugt wenigstens fünf, positiv geladene, vorzugsweise quartäre, Stickstoffatome, die nicht direkt an den Isoalloxazinring gebunden sind, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform der Variante I) des 10H-Benzo[g]pteridin-2,4-dion-Derivats der Formel (1) ist nur der Rest R5 ein nichtcyclischer Polyolrest der allgemeinen Formel -CH₂(CH(OZ))_{f}CH₍₃₋ₑ₎(OZ)ₑ, wobei e 0, 1, oder 2 ist und f eine ganze Zahl von 1 bis 10, vorzugsweise 1 bis 4, bedeutet und wobei Z Wasserstoff, Alkyl mit 1 bis 20 C-Atomen, Carbonsäurester mit 1 bis 20 C-Atomen oder ein Rest X ist,
wobei X ein organischer Rest ist, der a) wenigstens ein, vorzugsweise wenigstens zwei, vorzugsweise wenigstens drei, vorzugsweise wenigstens vier, weiter bevorzugt wenigstens fünf, neutrale, protonierbare Stickstoffatome oder b) wenigstens zwei, vorzugsweise wenigstens drei, vorzugsweise wenigstens vier, weiter bevorzugt wenigstens fünf, positiv geladene, vorzugsweise quartäre, Stickstoffatome enthält, wenn mindestens zwei der Reste Z ein Rest X bedeuten, oder
wobei X ein organischer Rest ist, der a) wenigstens zwei, vorzugsweise wenigstens drei, vorzugsweise wenigstens vier, weiter bevorzugt wenigstens fünf, neutrale, protonierbare Stickstoffatome oder b) wenigstens zwei, vorzugsweise wenigstens drei, vorzugsweise wenigstens vier, weiter bevorzugt wenigstens fünf, positiv geladene, vorzugsweise quartäre, Stickstoffatome enthält, wenn nur 1 Rest Z ein Rest X bedeutet,
wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest der allgemeinen Formel -CH₂(CH(OZ))_{f}CH₍₃₋ₑ₎(OZ)ₑ, wobei e 1, oder 2 ist und f eine ganze Zahl von 1 bis 10, vorzugsweise 1 bis 4, bedeutet und wobei Z ein Ester einer Aminocarbonsäure ist, der sich von einer Am inocarbonsäure, vorzugsweise alpha-Aminocarbonsäure, mit 1 bis 20 C-Atomen ableitet, die vorzugsweise aus der Gruppe, die aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin, L-Homoserin, Ornithin, N5-(Aminocarbonyl)-L-ornithin, L-(-)-Carnitin, alpha-Phenylglycin, L-3,4-Dihydroxyphenylalanin, 3,6-Diaminohexansäure, N-(Aminoiminomethyl)-N-methyl-glycin, gamma-Aminobuttersäure, L-5-Hydroxytryptophan, Norleucin und 2,6-Diaminopimelinsäure, weiter bevorzugt Arginin, Glycin, Lysin, Ornithin, 3,6-Diaminohexansäure und gamma-Aminobuttersäure, noch weiter bevorzugt Glycin und Lysin, besteht, ausgewählt wird, wobei vorzugsweise jeweils wenigstes eine Aminogruppe mit Methylresten substituiert sein kann.

Bei einer weiter bevorzugten Ausführungsform der Variante I) des 10H-Benzo[g]pteridin-2,4-dion-Derivats ist der organische Rest mit a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, ein Rest der Formel (2), (3) oder (4): 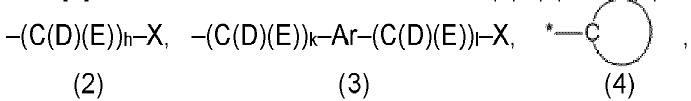
wobei h eine ganze Zahl von 6 bis 20, vorzugsweise von 6 bis 19, vorzugsweise von 8 bis 17, weiter bevorzugt von 8 bis 15, weiter bevorzugt von 8 bis 13, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) wenigstens zwei, vorzugsweise wenigstens drei, vorzugsweise wenigstens vier, weiter bevorzugt wenigstens fünf, neutrale, protonierbare Stickstoffatome, oder b) wenigstens zwei, vorzugsweise wenigstens drei, vorzugsweise wenigstens vier, weiter bevorzugt wenigstens fünf, positiv geladene, vorzugsweise quartäre, Stickstoffatome, enthält, und der Rest Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
wobei der Rest mit der Formel (4) einen Heteroaromaten, der über ein C-Atom des Heteroaromaten an den Isoalloxazinring gebunden ist und der a) wenigstens zwei, vorzugsweise wenigstens drei, neutrale, protonierbare Stickstoffatome oder b) wenigstens zwei, vorzugsweise wenigstens drei, positiv geladene, vorzugsweise quartäre, Stickstoffatome enthält, bedeutet.

Bei einer weiter bevorzugten Ausführungsform der Variante I) des 10H-Benzo[g]pteridin-2,4-dion-Derivats der Formel (1) ist der organische Rest X mit a) wenigstens zwei, vorzugsweise wenigstens drei, vorzugsweise wenigstens vier, weiter bevorzugt wenigstens fünf, neutralen, protonierbaren Stickstoffatomen, oder b) wenigstens zwei, vorzugsweise wenigstens drei, vorzugsweise wenigstens vier, weiter bevorzugt wenigstens fünf, positiv geladenen, vorzugsweise quartären, Stickstoffatomen, ein Rest der allgemeinen Formel (2): 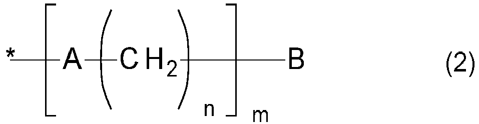 wobei A ein Sauerstoff- oder ein Schwefelatom ist und wobei n eine ganze Zahl von 1 bis 8 und m eine ganze Zahl von 0 bis 100 ist und wobei B ein Rest der Formel (3a),(3b), (4a), (4b), (5a) oder (5b): 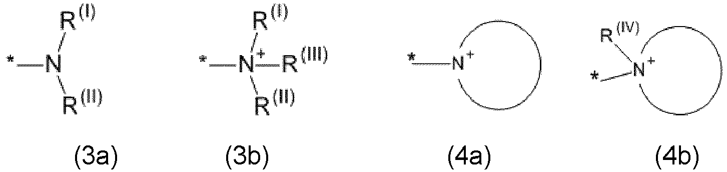 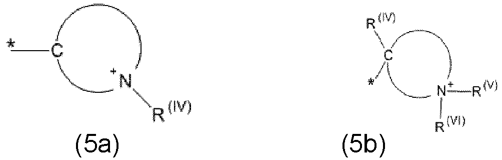 ist und wobei der Reste R^{(I)} ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist, wobei jeder der Reste R^{(II)}, R^{(III)}, R^{(IV)}, R^{(V)} und R^{(VI)} unabhängig voneinander Wasserstoff, ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist und wobei jeder der vorgenannten Arylreste, heterocyclische Reste, Alkylreste, Alkenylreste, Hydroxyalkylreste, Etherreste, Thioetherreste und Alkylaminoreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert ist,
und wobei der Rest mit der Formel (4a) und der Rest mit der Formel (5a): 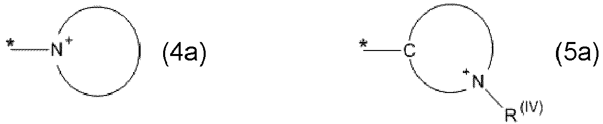 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 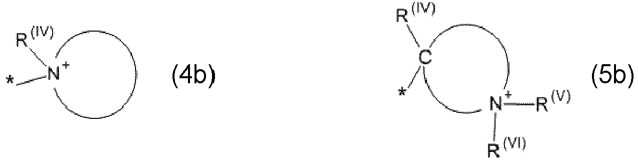 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, darstellt.

Bei einer weiter bevorzugten Ausführungsform der Variante I) des 10H-Benzo[g]pteridin-2,4-dion-Derivats der Formel (1) wird der Rest der Formel (5a) aus der Gruppe, die aus Resten der Formeln (22a), (22b) und (22c): 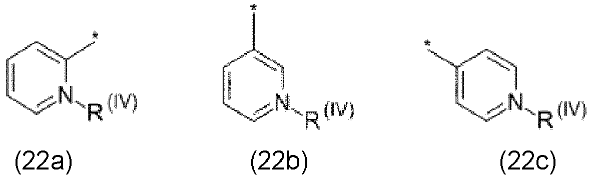 besteht, ausgewählt, wobei R^{(IV)} jeweils aus der Gruppe ausgewählt wird, die aus Arylresten mit 5 bis 20 C-Atomen, beispielsweise Phenyl oder Benzyl, heterocyclischen Resten mit 5 bis 20 C-Atomen, beispielsweise 2-Pyridyl, 3-Pyridyl, 4-Pyridyl, 1-Methylpyridin-1-ium-2-yl, 1-Methylpyridin-1-ium-3-yl oder 1-Methylpyridin-1-ium-4-yl, Alkylresten, die geradkettig oder verzweigt sein können, mit 1 bis 20 C-Atomen, beispielsweise Methyl, Ethyl, n-Propyl, i-Propyl, n-Butyl oder t-Butyl, Alkenylresten, die geradkettig oder verzweigt sein können, mit 2 bis 20 C-Atomen, Hydroxyalkylresten, die geradkettig oder verzweigt sein können, mit 1 bis 20 C-Atomen, beispielsweise Hydroxymethyl, 2-Hydroxyethyl, 3-Hydroxypropyl oder 1-Hydroxy-1-methyl-ethyl, Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, Thioetherresten, die geradkettig oder verzweigt sein können, mit 2 bis 20 C-Atomen, beispielsweise Methylsulfanylmethyl, Ethylsulfanylmethyl, 2-Ethylsulfanylethyl oder 3-Methylsulfanylpropyl, und Alkylaminoresten, die geradkettig oder verzweigt sein können, mit 1 bis 20 C-Atomen, beispielsweise Aminomethyl, 2-Aminoethyl, Dimethylaminomethyl oder (Trimethylammonio)methyl, besteht.

Bei einer bevorzugten Ausführungsform der Variante I) des 10H-Benzo[g]pteridin-2,4-dion-Derivats der Formel (1) wird der Rest der Formel (4a) aus der Gruppe, die aus Resten der Formeln (23a), (23b) und (23c): 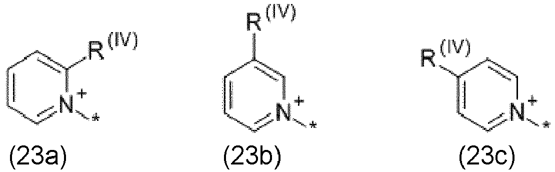 besteht, ausgewählt, wobei R^{(IV)} jeweils aus der Gruppe ausgewählt wird, die aus Wasserstoff, Arylresten mit 5 bis 20 C-Atomen, beispielsweise Phenyl oder Benzyl, heterocyclischen Resten mit 5 bis 20 C-Atomen, beispielsweise 2-Pyridyl, 3-Pyridyl, 4-Pyridyl, 1-Methylpyridin-1-ium-2-yl, 1-Methylpyridin-1-ium-3-yl oder 1-Methylpyridin-1-ium-4-yl, Alkylresten, die geradkettig oder verzweigt sein können, mit 1 bis 20 C-Atomen, beispielsweise Methyl, Ethyl, n-Propyl, i-Propyl, n-Butyl oder t-Butyl, Alkenylresten, die geradkettig oder verzweigt sein können, mit 2 bis 20 C-Atomen, Hydroxyalkylresten, die geradkettig oder verzweigt sein können, mit 1 bis 20 C-Atomen, beispielsweise Hydroxymethyl, 2-Hydroxyethyl, 3-Hydroxypropyl oder 1-Hydroxy-1-methyl-ethyl, Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, Thioetherresten, die geradkettig oder verzweigt sein können, mit 2 bis 20 C-Atomen, beispielsweise Methylsulfanylmethyl, Ethylsulfanylmethyl, 2-Ethylsulfanylethyl oder 3-Methylsulfanylpropyl, und Alkylaminoresten, die geradkettig oder verzweigt sein können, mit 1 bis 20 C-Atomen, beispielsweise Aminomethyl, 2-Aminoethyl, Dimethylaminomethyl oder (Trimethylammonio)methyl, besteht.

Bei einer bevorzugten Ausführungsform der Variante I) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Alkylrest der allgemeinen Formel - (CH₂)ₙ-CH₃ und der Rest R^{(I)} ist ein Alkylrest der allgemeinen Formel -(CH₂)ₙ-CH₃, wobei jeweils n eine ganze Zahl von 0 bis 19, vorzugsweise von 1 bis 17, ist, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, vorzugsweise 1 bis 4 C-Atomen, beispielsweise Aminomethyl, 2-Aminoethyl, Dimethylaminomethyl oder (Trimethylammonio)methyl, und/oder Guanidinorest substituiert sind.

Bei einer bevorzugten Ausführungsform der Variante I) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl oder 2,3-Dimethylbut-2-yl, und der Rest R^{(I)} ist Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl oder 2,3-Dimethylbut-2-yl, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, vorzugsweise 1 bis 4 C-Atomen, beispielsweise Aminomethyl, 2-Aminoethyl, Dimethylaminomethyl oder (Trimethylammonio)methyl, und/oder Guanidinorest substituiert sind.

Bei einer besonders bevorzugten Ausführungsform können die Reste R^{(I)}, R^{(II)}, R^{(III)}, R^{(IV)}, R^{(V)} und R^{(VI)} unabhängig voneinander Methyl, Ethyl, Prop-1-yl, But-1-yl, Pent-1-yl, Hex-1-yl, Hept-1-oder Oct-1-yl sein, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert sind.

Bei einer bevorzugten Ausführungsform der Variante I) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Rest der Formel (6) bis (9): 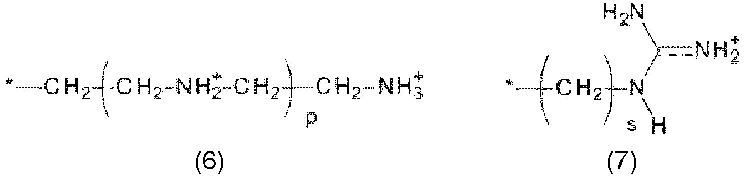 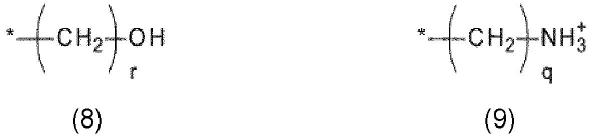 und der Rest R^{(I)} ist ein Rest der Formel (6) bis (9), wobei jeweils p eine ganze Zahl von 1 bis 10, vorzugsweise von 1 bis 7, weiter bevorzugt von 1 bis 3, bedeutet und wobei s, r, q jeweils unabhängig voneinander eine ganze Zahl von 1 bis 20, vorzugsweise von 1 bis 8, weiter bevorzugt von 1 bis 4, bedeutet, ausgewählt.

Bei einer weiter bevorzugten Ausführungsform der Variante I) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Polyaminrest der allgemeinen Formel -[(CH₂)ₐ-N(R^{(VII)})-(CH₂)_{b}-]_{c}NH_{(3-g)}(R^{(VIII)})_{g}⁺, und der Rest R^{(I)} ist ein Polyaminrest der allgemeinen Formel -[(CH₂)ₐ-N(R^{(VII)}-(CH₂)_{b}-]_{c}NH_{(3-g)}(R^{(VIII)})_{g}⁺, wobei jeweils c eine ganze Zahl von 1 bis 10, vorzugsweise von 1 bis 7, weiter bevorzugt von 1 bis 3, bedeutet, a und b jeweils unabhängig voneinander eine ganze Zahl von 1 bis 10, vorzugsweise von 1 bis 4, weiter bevorzugt von 2 bis 3, bedeuten, g 0, 1, 2 oder 3 ist und R^{(VII)} und R^{(VIII)} unabhängig voneinander Wasserstoff, Methyl oder Ethyl bedeuten.

Bei Variante II) des erfindungsgemäßen 10H-Benzo[g]pteridin-2,4-dion-Derivats der Formel (1), 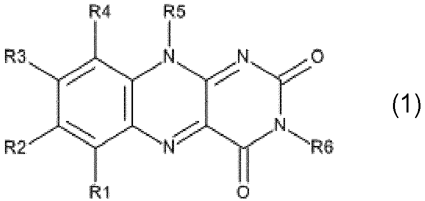
ist nur 1 Rest R1, R2, R3, R4, R5 oder R6 ein organischer Rest mit
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind),
und wobei jeder der Reste R1, R2, R3 oder R4, der kein organischer Rest mit a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
wobei jeder der Reste R5 oder R6, der kein organischer Rest mit a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet.

Bei einer bevorzugten Ausführungsform der Variante II) des 10H-Benzo[g]pteridin-2,4-dion-Derivats der Formel (1) ist der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet und wobei
A) nur 1 Rest R1, R2, R3 oder R4 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet,
   wobei jeder der Reste R1, R2, R3 oder R4, der kein organischer Rest mit a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei der Reste R6 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
   oder
B) nur der Reste R6 in organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet,
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer bevorzugten Ausführungsform der Variante II) des 10H-Benzo[g]pteridin-2,4-dion-Derivats der Formel (1) ist der organische Rest mit a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), ein Rest der Formel (2), (3) oder (4): 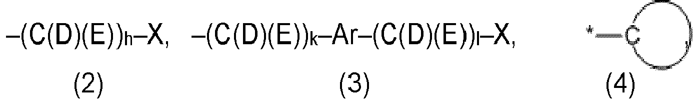 wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder

Thiol, weiter bevorzugt Wasserstoff oder Hydroxyl, bedeuten und wobei X ein organischer Rest ist, der a) wenigstens ein, vorzugsweise wenigstens zwei, vorzugsweise wenigstens drei, vorzugsweise wenigstens vier, weiter bevorzugt wenigstens fünf, neutrale(s), protonierbare(s) Stickstoffatom(e), und b) wenigstens ein, vorzugsweise wenigstens zwei, vorzugsweise wenigstens drei, vorzugsweise wenigstens vier, weiter bevorzugt wenigstens fünf, positiv geladene(s), vorzugsweise quatäre(s), Stickstoffatom(e), enthält, und der Rest Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
wobei der Rest mit der Formel (4) einen Heteroaromaten, der über ein C-Atom des Heteroaromaten an den Isoalloxazinring gebunden ist und der a) wenigstens ein, vorzugsweise wenigstens zwei, vorzugsweise wenigstens drei, neutrale(s), protonierbare(s) Stickstoffatom(e) und b) wenigstens ein, vorzugsweise wenigstens zwei, vorzugsweise wenigstens drei, positiv geladene(s), vorzugsweise quartäre(s), Stickstoffatom(e), enthält, bedeutet.

Bei einer weiter bevorzugten Ausführungsform der Variante II) des 10H-Benzo[g]pteridin-2,4-dion-Derivats ist der organische Rest mit a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), ein Rest der Formel (2) oder (3): 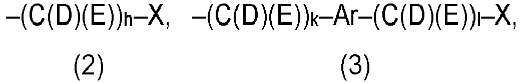 wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) wenigstens ein, vorzugsweise wenigstens zwei, vorzugsweise wenigstens drei, vorzugsweise wenigstens vier, weiter bevorzugt wenigstens fünf, neutrale(s), protonierbare(s) Stickstoffatom(e), und b) wenigstens ein, vorzugsweise wenigstens zwei, vorzugsweise wenigstens drei, vorzugsweise wenigstens vier, weiter bevorzugt wenigstens fünf, positiv geladene(s), vorzugsweise quatäre(s), Stickstoffatom(e), enthält, und der Rest Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet.

Bei einer weiteren bevorzugten Ausführungsform der Variante II) des 10H-Benzo[g]pteridin-2,4-dion-Derivats der Formel (1) ist der Rest R5 ein nichtcyclischer Polyolrest der Formel -CH₂(CH(OH))_{g}CH₂OH oder ein Ether, Ester oder Acetal davon, wobei g eine ganze Zahl von 1 bis 10, vorzugsweise 1 bis 4, bedeutet, und wobei entweder
A) nur 1 Rest R1, R2, R3 oder R4 ein organischer Rest der allgemeinen Formel (2), (3) oder (4): 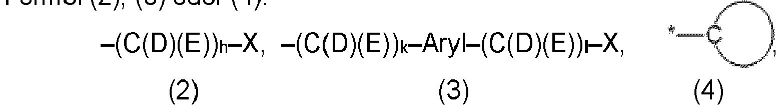 bedeutet,
   wobei h eine ganze Zahl von 1 bis 20 und k und l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest mit
      a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und
      b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), bedeutet und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) wenigstens ein, vorzugsweise wenigstens zwei, vorzugsweise wenigstens drei, neutrale(s), protonierbare(s) Stickstoffatom(e), und b) wenigstens ein, vorzugsweise wenigstens zwei, vorzugsweise wenigstens drei, positiv geladene(s), vorzugsweise quartäre(s), Stickstoffatom(e), enthält, bedeutet und
   wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei der Rest R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet
      oder
B) nur der Rest R6 ein organischer Rest der allgemeinen Formel (2), (3) oder (4): 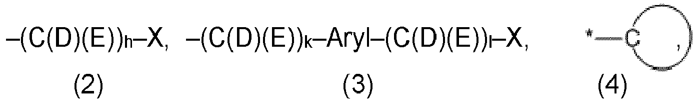 bedeutet,
   wobei h eine ganze Zahl von 1 bis 20 und k und l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, ist und wobei X ein organischer Rest mit
      a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und
      b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) wenigstens ein, vorzugsweise wenigstens zwei, vorzugsweise wenigstens drei, neutrale(s), protonierbare(s) Stickstoffatom(e), und b) wenigstens ein, vorzugsweise wenigstens zwei, vorzugsweise wenigstens drei, positiv geladene(s), vorzugsweise quartäre(s), Stickstoffatom(e), enthält, bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Bei einer weiter bevorzugten Ausführungsform der Variante II) des 10H-Benzo[g]pteridin-2,4-dion-Derivats der Formel (1) ist der Rest R5 ein nichtcyclischer Polyolrest der Formel -CH₂(CH(OH))_{g}CH₂OH oder ein Ether, Ester oder Acetal davon, wobei g eine ganze Zahl von 1 bis 10, vorzugsweise 1 bis 4, bedeutet, und wobei entweder
A) nur 1 Rest R1, R2, R3 oder R4 ein organischer Rest der allgemeinen Formel -(C(D)(E))ₕ-X oder -(C(D)(E))ₖ-Aryl-(C(D)(E))ₗ-X ist,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei X ein organischer Rest mit
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
   wobei die Reste R1, R2, R3 oder R4, die nicht ein organischer Rest der allgemeinen Formel -(C(D)(E))ₕ-X oder -(C(D)(E))ₖ-Aryl-(C(D)(E))ₗ-X sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet
   oder
B) nur der Rest R6 ein organischer Rest der allgemeinen Formel -(C(D)(E))ₕ-X oder -(C(D)(E))ₖ-Aryl-(C(D)(E))ₗ-X ist,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, wobei X ein organischer Rest mit
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), bedeutet und
   wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform der Variante II) des 10H-Benzo[g]pteridin-2,4-dion-Derivats der Formel (1) ist nur der Rest R5 ein nichtcyclischer Polyolrest der allgemeinen Formel -CH₂(CH(OZ))_{f}CH₍₃₋ₑ₎(OZ)ₑ, wobei e 0, 1 oder 2 ist und f eine ganze Zahl von 1 bis 10, vorzugsweise 1 bis 4, bedeutet und wobei Z Wasserstoff, Alkyl mit 1 bis 20 C-Atomen, Carbonsäurester mit 1 bis 20 C-Atomen oder ein Rest X ist, wobei X ein organischer Rest ist, der a) wenigstens ein, vorzugsweise wenigstens zwei, vorzugsweise wenigstens drei, vorzugsweise wenigstens vier, weiter bevorzugt wenigstens fünf, neutrale(s), protonierbare(s) Stickstoffatom(e), und b) wenigstens ein, vorzugsweise wenigstens zwei, vorzugsweise wenigstens drei, vorzugsweise wenigstens vier, weiter bevorzugt wenigstens fünf, positiv geladene(s), vorzugsweise quartäre(s), Stickstoffatom(e), enthält und
wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest der allgemeinen Formel -CH₂(CH(OZ))_{f}CH₍₃₋ₑ₎(OZ)ₑ, wobei e 1, oder 2 ist und f eine ganze Zahl von 1 bis 10, vorzugsweise 1 bis 4, bedeutet und wobei Z ein Ester einer Aminocarbonsäure ist, der sich von einer Am inocarbonsäure, vorzugsweise alpha-Aminocarbonsäure, mit 1 bis 20 C-Atomen ableitet, die vorzugsweise aus der Gruppe, die aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin, L-Homoserin, Ornithin, N5-(Aminocarbonyl)-L-ornithin, L-(-)-Carnitin, alpha-Phenylglycin, L-3,4-Dihydroxyphenylalanin, 3,6-Diaminohexansäure, N-(Aminoiminomethyl)-N-methyl-glycin, gamma-Aminobuttersäure, L-5-Hydroxytryptophan, Norleucin und 2,6-Diaminopimelinsäure, weiter bevorzugt Arginin, Glycin, Lysin, Ornithin, 3,6-Diaminohexansäure und gamma-Aminobuttersäure, noch weiter bevorzugt Glycin und Lysin, besteht, ausgewählt wird, wobei vorzugsweise jeweils wenigstes eine Aminogruppe mit Methylresten substituiert sein kann.

Bei einer weiter bevorzugten Ausführungsform der Variante II) des 10H-Benzo[g]pteridin-2,4-dion-Derivats der Formel (1) ist der organische Rest X mit
a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und
b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), ein Rest der allgemeinen Formel (2): 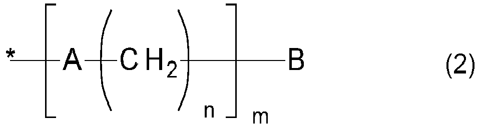 wobei A ein Sauerstoff- oder ein Schwefelatom ist und wobei n eine ganze Zahl von 1 bis 8 und m eine ganze Zahl von 0 bis 100 ist und wobei B ein Rest der Formel (3a),(3b), (4a), (4b), (5a) oder (5b): 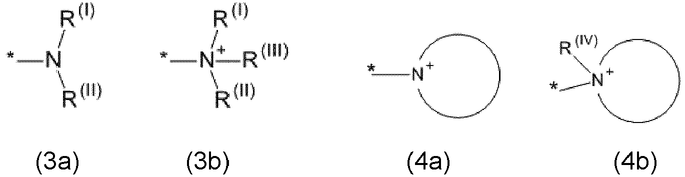 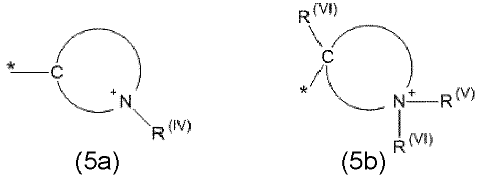 ist und wobei der Reste R^{(I)} ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist, wobei jeder der Reste R^{(II)}, R^{(III)}, R^{(IV)}, R^{(V)} und R^{(VI)} unabhängig voneinander Wasserstoff, ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist und wobei jeder der vorgenannten Arylreste, heterocyclische Reste, Alkylreste, Alkenylreste, Hydroxyalkylreste, Etherreste, Thioetherreste und Alkylaminoreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert ist,
   und wobei der Rest mit der Formel (4a) und der Rest mit der Formel (5a): 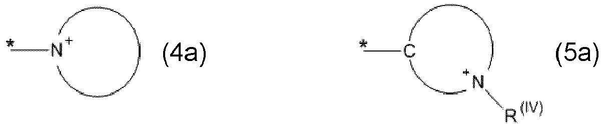 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 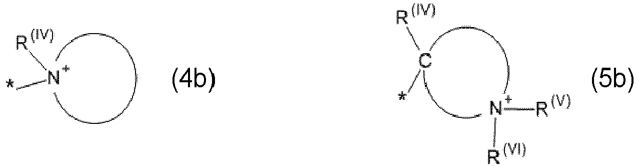 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, darstellt.

Bei einer weiter bevorzugten Ausführungsform der Variante II) des 10H-Benzo[g]pteridin-2,4-dion-Derivats der Formel (1) wird der Rest der Formel (5a) aus der Gruppe, die aus Resten der Formeln (22a), (22b) und (22c): 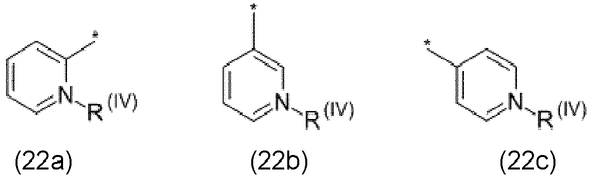 besteht, ausgewählt, wobei R^{(IV)} jeweils aus der Gruppe ausgewählt wird, die aus Arylresten mit 5 bis 20 C-Atomen, beispielsweise Phenyl oder Benzyl, heterocyclischen Resten mit 5 bis 20 C-Atomen, beispielsweise 2-Pyridyl, 3-Pyridyl, 4-Pyridyl, 1-Methylpyridin-1-ium-2-yl, 1-Methylpyridin-1-ium-3-yl oder 1-Methylpyridin-1-ium-4-yl, Alkylresten, die geradkettig oder verzweigt sein können, mit 1 bis 20 C-Atomen, beispielsweise Methyl, Ethyl, n-Propyl, i-Propyl, n-Butyl oder t-Butyl, Alkenylresten, die geradkettig oder verzweigt sein können, mit 2 bis 20 C-Atomen, Hydroxyalkylresten, die geradkettig oder verzweigt sein können, mit 1 bis 20 C-Atomen, beispielsweise Hydroxymethyl, 2-Hydroxyethyl, 3-Hydroxypropyl oder 1-Hydroxy-1-methyl-ethyl, Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, Thioetherresten, die geradkettig oder verzweigt sein können, mit 2 bis 20 C-Atomen, beispielsweise Methylsulfanylmethyl, Ethylsulfanylmethyl, 2-Ethylsulfanylethyl oder 3-Methylsulfanylpropyl, und Alkylaminoresten, die geradkettig oder verzweigt sein können, mit 1 bis 20 C-Atomen, beispielsweise Aminomethyl, 2-Aminoethyl, Dimethylaminomethyl oder (Trimethylammonio)methyl, besteht.

Bei einer bevorzugten Ausführungsform der Variante II) des 10H-Benzo[g]pteridin-2,4-dion-Derivats der Formel (1) wird der Rest der Formel (4a) aus der Gruppe, die aus Resten der Formeln (23a), (23b) und (23c): 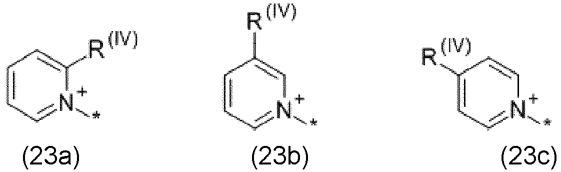 besteht, ausgewählt, wobei R^{(IV)} jeweils aus der Gruppe ausgewählt wird, die aus Wasserstoff, Arylresten mit 5 bis 20 C-Atomen, beispielsweise Phenyl oder Benzyl, heterocyclischen Resten mit 5 bis 20 C-Atomen, beispielsweise 2-Pyridyl, 3-Pyridyl, 4-Pyridyl, 1-Methylpyridin-1-ium-2-yl, 1-Methylpyridin-1-ium-3-yl oder 1-Methylpyridin-1-ium-4-yl, Alkylresten, die geradkettig oder verzweigt sein können, mit 1 bis 20 C-Atomen, beispielsweise Methyl, Ethyl, n-Propyl, i-Propyl, n-Butyl oder t-Butyl, Alkenylresten, die geradkettig oder verzweigt sein können, mit 2 bis 20 C-Atomen, Hydroxyalkylresten, die geradkettig oder verzweigt sein können, mit 1 bis 20 C-Atomen, beispielsweise Hydroxymethyl, 2-Hydroxyethyl, 3-Hydroxypropyl oder 1-Hydroxy-1-methyl-ethyl, Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, Thioetherresten, die geradkettig oder verzweigt sein können, mit 2 bis 20 C-Atomen, beispielsweise Methylsulfanylmethyl, Ethylsulfanylmethyl, 2-Ethylsulfanylethyl oder 3-Methylsulfanylpropyl, und Alkylaminoresten, die geradkettig oder verzweigt sein können, mit 1 bis 20 C-Atomen, beispielsweise Aminomethyl, 2-Aminoethyl, Dimethylaminomethyl oder (Trimethylammonio)methyl, besteht.

Bei einer bevorzugten Ausführungsform der Variante II) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Alkylrest der allgemeinen Formel - (CH₂)ₙ-CH₃ und der Rest R^{(I)} ist ein Alkylrest der allgemeinen Formel -(CH₂)ₙ-CH₃, wobei jeweils n eine ganze Zahl von 0 bis 19, vorzugsweise von 1 bis 17, ist, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, vorzugsweise 1 bis 4 C-Atomen, beispielsweise Aminomethyl, 2-Aminoethyl, Dimethylaminomethyl oder (Trimethylammonio)methyl, und/oder Guanidinorest substituiert sind.

Bei einer bevorzugten Ausführungsform der Variante II) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl oder 2,3-Dimethylbut-2-yl, und der Rest R^{(I)} ist Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl oder 2,3-Dimethylbut-2-yl, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, vorzugsweise 1 bis 4 C-Atomen, beispielsweise Aminomethyl, 2-Aminoethyl, Dimethylaminomethyl oder (Trimethylammonio)methyl, und/oder Guanidinorest substituiert sind.

Bei einer besonders bevorzugten Ausführungsform können die Reste R^{(I)}, R^{(II)}, R^{(III)}, R^{(IV)}, R^{(V)} und R^{(VI)} unabhängig voneinander Methyl, Ethyl, Prop-1-yl, But-1-yl, Pent-1-yl, Hex-1-yl, Hept-1-oder Oct-1-yl sein, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert sind.

Bei einer bevorzugten Ausführungsform der Variante II) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Rest der Formel (6) bis (9): 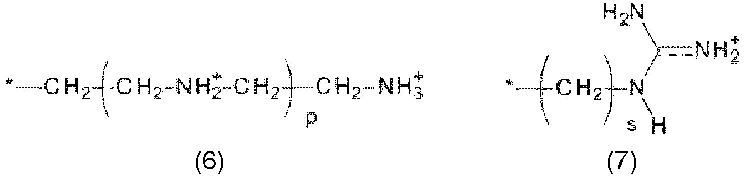 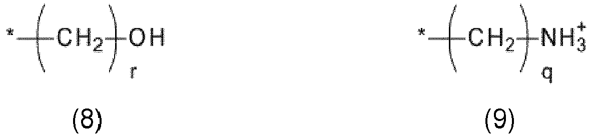 und der Rest R^{(I)} ist ein Rest der Formel (6) bis (9), wobei jeweils p eine ganze Zahl von 1 bis 10, vorzugsweise von 1 bis 7, weiter bevorzugt von 1 bis 3, bedeutet und wobei s, r, q jeweils unabhängig voneinander eine ganze Zahl von 1 bis 20, vorzugsweise von 1 bis 8, weiter bevorzugt von 1 bis 4, bedeutet, ausgewählt.

Bei einer weiter bevorzugten Ausführungsform der Variante II) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Polyaminrest der allgemeinen Formel -[(CH₂)ₐ-N(R^{(VII)})-(CH₂)_{b}-]_{c}NH_{(3-g)}(R^{(VIII)}_{g}⁺, und der Rest R^{(I)} ist ein Polyaminrest der allgemeinen Formel -[(CH₂)ₐ-N(R^{(VII)}-(CH₂)_{b}-]_{c}NH_{(3-g)}(R^{(VIII)})_{g}⁺, wobei jeweils c eine ganze Zahl von 1 bis 10, vorzugsweise von 1 bis 7, weiter bevorzugt von 1 bis 3, bedeutet, a und b jeweils unabhängig voneinander eine ganze Zahl von 1 bis 10, vorzugsweise von 1 bis 4, weiter bevorzugt von 2 bis 3, bedeuten, g 0, 1, 2 oder 3 ist und R^{(VII)} und R^{(VIII)} unabhängig voneinander Wasserstoff, Methyl oder Ethyl bedeuten.

Bei Variante III) des erfindungsgemäßen 10H-Benzo[g]pteridin-2,4-dion-Derivats der Formel (1), 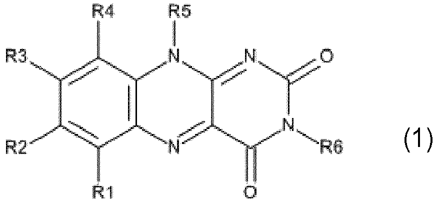
sind wenigstens 2 Reste R1, R2, R3, R4, R5 oder R6 ein organischer Rest mit:
   a) wenigstens einem neutralen, protonierbaren Stickstoffatom, das nicht direkt an den Isoalloxazinring gebunden ist, und/oder
   b) wenigstens einem positiv geladenen, vorzugsweise quartären, Stickstoffatom, das nicht direkt an den Isoalloxazinring gebunden ist, bedeutet und
wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und/oder b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
wobei jeder der Reste R5 oder R6, der kein organischer Rest mit a) wenigstens einem neutralen, protonierbaren Stickstoffatom, das nicht direkt an den Isoalloxazinring gebunden ist, und/oder b) wenigstens einem positiv geladenen, vorzugsweise quartäres, Stickstoffatom, das nicht direkt an den Isoalloxazinring gebunden ist, bedeutet, Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet
und wobei die Verbindung mit der Formel (20): ausgenommen ist.

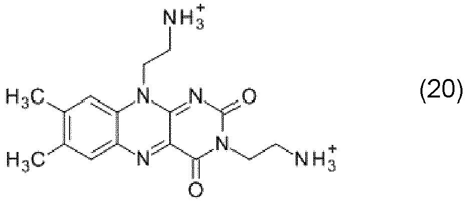

Vorzugsweise ist der Rest R3 kein heterocyclischer Rest mit 4 bis 7 C-Atomen und mindestens einem Stickstoffatom.

Bei einer bevorzugten Ausführungsform der Variante III) des 10H-Benzo[g]pteridin-2,4-dion-Derivats der Formel (1) ist der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet und wobei
wenigstens 2 Reste R1, R2, R3, R4 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und/oder
   b) wenigstens einem, vorzugsweise wenigstens zwei, vorzugsweise wenigstens drei, vorzugsweise wenigstens vier, weiter bevorzugt wenigstens fünf, permanent positiv geladenen, vorzugsweise quartären, Stickstoffatom(en), das (die) nicht direkt an den Isoalloxazinring gebunden ist (sind), bedeutet
und wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und b) wenigstens einem permanent positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
wobei der Rest R6, wenn R6 nicht ein organischer Rest mit a) wenigstens einem neutralen, protonierbaren Stickstoffatom, das nicht direkt an den Isoalloxazinring gebunden ist, und b) wenigstens einem permanent positiv geladenen, vorzugsweise quartären, Stickstoffatom, das nicht direkt an den Isoalloxazinring gebunden ist, bedeutet, Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, ist.

Weiter bevorzugt ist der Rest R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform der Variante III) des 10H-Benzo[g]pteridin-2,4-dion-Derivats der Formel (1) sind
wenigstens 2 Reste R1, R2, R3 oder R4 unabhängig voneinander ein organischer Rest der allgemeinen Formel (2), (3) oder (4), : 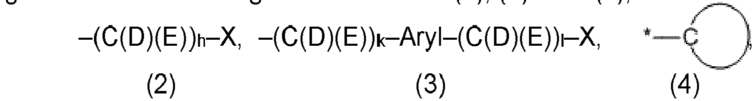
wobei h eine ganze Zahl von 1 bis 20 und k und l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest mit
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und/oder
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), bedeutet und
wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) wenigstens ein, vorzugsweise wenigstens zwei, vorzugsweise wenigstens drei, , neutrale(s), protonierbare(s) Stickstoffatom(e), und/oder b) wenigstens ein, vorzugsweise wenigstens zwei, vorzugsweise wenigstens drei, positiv geladene(s) vorzugsweise quartäre(s), Stickstoffatom(e), enthält, bedeutet und
wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
wobei jeder der Reste R5 oder R6 jeweils unabhängig voneinander gleich oder verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet.

Bei einer weiter bevorzugten Ausführungsform sind wenigstens 2 Reste R1, R2, R3 oder R4 unabhängig voneinander ein organischer Rest der allgemeinen Formel (2) oder (3).

Bei einer weiteren bevorzugten Ausführungsform der Variante III) des 10H-Benzo[g]pteridin-2,4-dion-Derivats der Formel (1) sind
beide Reste R5 und R6 unabhängig voneinander ein organischer Rest der allgemeinen Formel (2), (3) oder (4): 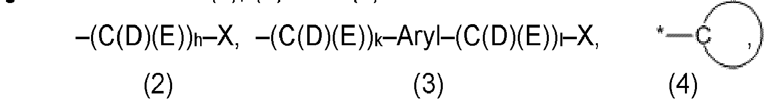
wobei h eine ganze Zahl von 1 bis 20 und k und l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, ist und wobei X ein organischer Rest mit
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und/oder
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), bedeutet und
wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) wenigstens ein, vorzugsweise wenigstens zwei, vorzugsweise wenigstens drei, neutrale(s), protonierbare(s) Stickstoffatom(e), und/oder b) wenigstens ein, vorzugsweise wenigstens zwei, vorzugsweise wenigstens drei, positiv geladene(s), vorzugsweise quartäre(s), Stickstoffatom(e), das (die) nicht direkt an den Isoalloxazinring gebunden ist (sind), enthält, bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Bei einer weiter bevorzugten Ausführungsform sind die beide Reste R5 und R6 unabhängig voneinander ein organischer Rest der allgemeinen Formel (2) oder (3).

Bei einer weiteren bevorzugten Ausführungsform der Variante III) des 10H-Benzo[g]pteridin-2,4-dion-Derivats der Formel (1) sind
wenigstens 1 Rest R1, R2, R3 oder R4 unabhängig voneinander ein organischer Rest der allgemeinen Formel (2), (3) oder (4) und wenigstens1 Rest R5 oder R6 unabhängig voneinander ein organischer Rest der allgemeinen Formel (2), (3) oder (4): 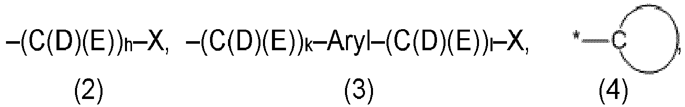
wobei h eine ganze Zahl von 1 bis 20 und k und l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest mit
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und/oder
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), bedeutet und
wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) wenigstens ein, vorzugsweise wenigstens zwei, vorzugsweise wenigstens drei, neutrale(s), protonierbare(s) Stickstoffatom(e), und/oder b) wenigstens ein, vorzugsweise wenigstens zwei, vorzugsweise wenigstens drei, positiv geladene(s), vorzugsweise quartäre(s), Stickstoffatom(e), enthält, bedeutet und
wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
wobei die Reste R5 oder R6, die nicht ein organischer Rest der allgemeinen Formel (2), (3) oder (4) sind, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet.

Bei einer weiter bevorzugten Ausführungsform sind wenigstens 1 Rest R1, R2, R3 oder R4 unabhängig voneinander ein organischer Rest der allgemeinen Formel (2) oder (3) und wenigstens1 Rest R5 oder R6 unabhängig voneinander ein organischer Rest der allgemeinen Formel (2), (3) oder (4), vorzugsweise ein organischer Rest der allgemeinen Formel (2) oder (3).

Bei einer weiteren bevorzugten Ausführungsform der Variante III) des 10H-Benzo[g]pteridin-2,4-dion-Derivats der Formel (1) ist
wenigstens 1 Rest R1, R2, R3 oder R4 sowie der Rest R6 jeweils unabhängig voneinander ein organischer Rest der allgemeinen Formel (2), (3) oder (4): 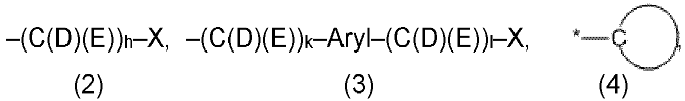 und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet.

Bei einer weiter bevorzugten Ausführungsform der Variante III) des 10H-Benzo[g]pteridin-2,4-dion-Derivats der Formel (1) sind
wenigstens 2 Reste R1, R2, R3 oder R4 unabhängig voneinander ein organischer Rest der allgemeinen Formel (2), (3) oder (4): 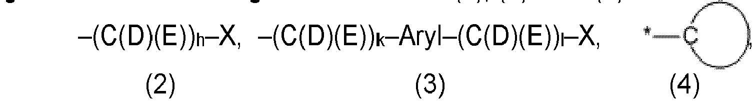
und wobei der Rest R5 ein nichtcyclischer Polyolrest der allgemeinen Formel -CH₂(CH(OH))_{g}CH₂OH oder ein Ether, Ester oder Acetal davon, wobei g eine ganze Zahl von 1 bis 10, vorzugsweise 1 bis 4, bedeutet und
wobei der Rest R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform der Variante III) des 10H-Benzo[g]pteridin-2,4-dion-Derivats der Formel (1) ist nur der Rest R5 ein nichtcyclischer Polyolrest der allgemeinen Formel -CH₂(CH(OZ))_{f}CH₍₃₋ₑ₎(OZ)ₑ, wobei e 0, 1 oder 2 ist und f eine ganze Zahl von 1 bis 10, vorzugsweise 1 bis 4, bedeutet und wobei Z Wasserstoff, Alkyl mit 1 bis 20 C-Atomen, Carbonsäurester mit 1 bis 20 C-Atomen oder ein Rest X ist, wobei X ein organischer Rest ist, der a) wenigstens ein, vorzugsweise wenigstens zwei, vorzugsweise wenigstens drei, vorzugsweise wenigstens vier, weiter bevorzugt wenigstens fünf, neutrale(s), protonierbare(s) Stickstoffatom(e), und b) wenigstens ein, vorzugsweise wenigstens zwei, vorzugsweise wenigstens drei, vorzugsweise wenigstens vier, weiter bevorzugt wenigstens fünf, positiv geladene(s), vorzugsweise quartäre(s), Stickstoffatom(e), enthält und
wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Rest R5 ein nichtcyclischer Polyolrest der allgemeinen Formel -CH₂(CH(OZ))_{f}CH₍₃₋ₑ₎(OZ)ₑ, wobei e 1, oder 2 ist und f eine ganze Zahl von 1 bis 10, vorzugsweise 1 bis 4, bedeutet und wobei Z ein Ester einer Aminocarbonsäure ist, der sich von einer Am inocarbonsäure, vorzugsweise alpha-Aminocarbonsäure, mit 1 bis 20 C-Atomen ableitet, die vorzugsweise aus der Gruppe, die aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin, L-Homoserin, Ornithin, N5-(Aminocarbonyl)-L-ornithin, L-(-)-Carnitin, alpha-Phenylglycin, L-3,4-Dihydroxyphenylalanin, 3,6-Diaminohexansäure, N-(Aminoiminomethyl)-N-methyl-glycin, gamma-Aminobuttersäure, L-5-Hydroxytryptophan, Norleucin und 2,6-Diaminopimelinsäure, weiter bevorzugt Arginin, Glycin, Lysin, Ornithin, 3,6-Diaminohexansäure und gamma-Aminobuttersäure, noch weiter bevorzugt Glycin und Lysin, besteht, ausgewählt wird, wobei vorzugsweise jeweils wenigstens eine Aminogruppe mit Methylresten substituiert sein kann.

Bei einer weiter bevorzugten Ausführungsform der Variante III) des 10H-Benzo[g]pteridin-2,4-dion-Derivats der Formel (1) ist der organische Rest X mit
a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), und/oder
b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), ein Rest der allgemeinen Formel (2): 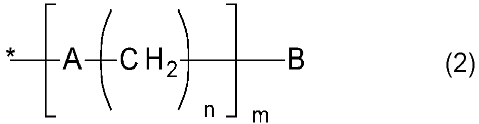 wobei A ein Sauerstoff- oder ein Schwefelatom ist und wobei n eine ganze Zahl von 1 bis 8 und m eine ganze Zahl von 0 bis 100 ist und wobei B ein Rest der Formel (3a),(3b), (4a), (4b), (5a) oder (5b): 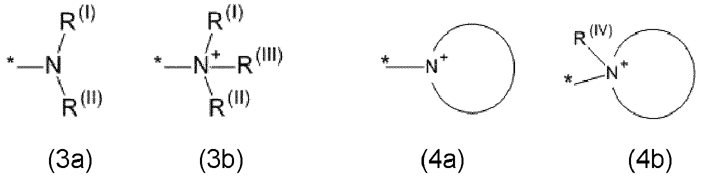 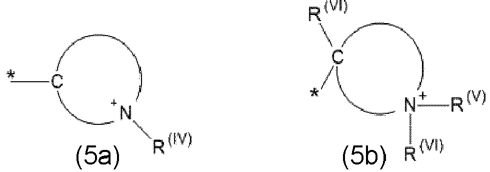 ist und wobei der Reste R^{(I)} ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist, wobei jeder der Reste R^{(II)}, R^{(III)}, R^{(IV)}, R^{(V)} und R^{(VI)} unabhängig voneinander Wasserstoff, ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist und wobei jeder der vorgenannten Arylreste, heterocyclische Reste, Alkylreste, Alkenylreste, Hydroxyalkylreste, Etherreste, Thioetherreste und Alkylaminoreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert ist,
   und wobei der Rest mit der Formel (4a) und der Rest mit der Formel (5a): 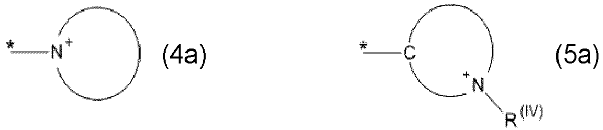 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 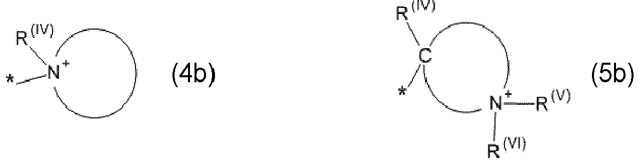 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, darstellt.

Bei einer weiter bevorzugten Ausführungsform der Variante III) des 10H-Benzo[g]pteridin-2,4-dion-Derivats der Formel (1) wird der Rest der Formel (5a) aus der Gruppe, die aus Resten der Formeln (22a), (22b) und (22c): 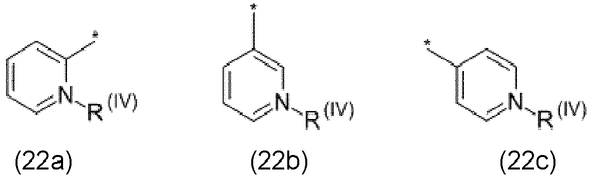 besteht, ausgewählt, wobei R^{(IV)} jeweils aus der Gruppe ausgewählt wird, die aus Arylresten mit 5 bis 20 C-Atomen, beispielsweise Phenyl oder Benzyl, heterocyclischen Resten mit 5 bis 20 C-Atomen, beispielsweise 2-Pyridyl, 3-Pyridyl, 4-Pyridyl, 1-Methylpyridin-1-ium-2-yl, 1-Methylpyridin-1-ium-3-yl oder 1-Methylpyridin-1-ium-4-yl, Alkylresten, die geradkettig oder verzweigt sein können, mit 1 bis 20 C-Atomen, beispielsweise Methyl, Ethyl, n-Propyl, i-Propyl, n-Butyl oder t-Butyl, Alkenylresten, die geradkettig oder verzweigt sein können, mit 2 bis 20 C-Atomen, Hydroxyalkylresten, die geradkettig oder verzweigt sein können, mit 1 bis 20 C-Atomen, beispielsweise Hydroxymethyl, 2-Hydroxyethyl, 3-Hydroxypropyl oder 1-Hydroxy-1-methyl-ethyl, Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, Thioetherresten, die geradkettig oder verzweigt sein können, mit 2 bis 20 C-Atomen, beispielsweise Methylsulfanylmethyl, Ethylsulfanylmethyl, 2-Ethylsulfanylethyl oder 3-Methylsulfanylpropyl, und Alkylaminoresten, die geradkettig oder verzweigt sein können, mit 1 bis 20 C-Atomen, beispielsweise Aminomethyl, 2-Aminoethyl, Dimethylaminomethyl oder (Trimethylammonio)methyl, besteht.

Bei einer bevorzugten Ausführungsform der Variante III) des 10H-Benzo[g]pteridin-2,4-dion-Derivats der Formel (1) wird der Rest der Formel (4a) aus der Gruppe, die aus Resten der Formeln (23a), (23b) und (23c): 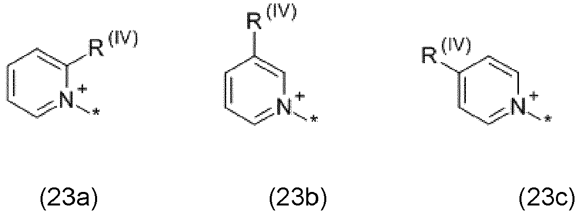 besteht, ausgewählt, wobei R^{(IV)} jeweils aus der Gruppe ausgewählt wird, die aus Wasserstoff, Arylresten mit 5 bis 20 C-Atomen, beispielsweise Phenyl oder Benzyl, heterocyclischen Resten mit 5 bis 20 C-Atomen, beispielsweise 2-Pyridyl, 3-Pyridyl, 4-Pyridyl, 1-Methylpyridin-1-ium-2-yl, 1-Methylpyridin-1-ium-3-yl oder 1-Methylpyridin-1-ium-4-yl, Alkylresten, die geradkettig oder verzweigt sein können, mit 1 bis 20 C-Atomen, beispielsweise Methyl, Ethyl, n-Propyl, i-Propyl, n-Butyl oder t-Butyl, Alkenylresten, die geradkettig oder verzweigt sein können, mit 2 bis 20 C-Atomen, Hydroxyalkylresten, die geradkettig oder verzweigt sein können, mit 1 bis 20 C-Atomen, beispielsweise Hydroxymethyl, 2-Hydroxyethyl, 3-Hydroxypropyl oder 1-Hydroxy-1-methyl-ethyl, Etherresten, die geradkettig oder verzweigt sein können, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, Thioetherresten, die geradkettig oder verzweigt sein können, mit 2 bis 20 C-Atomen, beispielsweise Methylsulfanylmethyl, Ethylsulfanylmethyl, 2-Ethylsulfanylethyl oder 3-Methylsulfanylpropyl, und Alkylaminoresten, die geradkettig oder verzweigt sein können, mit 1 bis 20 C-Atomen, beispielsweise Aminomethyl, 2-Aminoethyl, Dimethylaminomethyl oder (Trimethylammonio)methyl, besteht.

Bei einer bevorzugten Ausführungsform der Variante III) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Alkylrest der allgemeinen Formel - (CH₂)ₙ-CH₃ und der Rest R^{(I)} ist ein Alkylrest der allgemeinen Formel -(CH₂)ₙ-CH₃, wobei jeweils n eine ganze Zahl von 0 bis 19, vorzugsweise von 1 bis 17, ist, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, vorzugsweise 1 bis 4 C-Atomen, beispielsweise Aminomethyl, 2-Aminoethyl, Dimethylaminomethyl oder (Trimethylammonio)methyl, und/oder Guanidinorest substituiert sind.

Bei einer bevorzugten Ausführungsform der Variante III) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl oder 2,3-Dimethylbut-2-yl, und der Rest R^{(I)} ist Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl oder 2,3-Dimethylbut-2-yl, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, vorzugsweise 1 bis 4 C-Atomen, beispielsweise Aminomethyl, 2-Aminoethyl, Dimethylaminomethyl oder (Trimethylammonio)methyl, und/oder Guanidinorest substituiert sind.

Bei einer besonders bevorzugten Ausführungsform können die Reste R^{(I)}, R^{(II)}, R^{(III)}, R^{(IV)}, R^{(V)} und R^{(VI)} unabhängig voneinander Methyl, Ethyl, Prop-1-yl, But-1-yl, Pent-1-yl, Hex-1-yl, Hept-1-oder Oct-1-yl sein, wobei vorgenannte Alkylreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert sind.

Bei einer bevorzugten Ausführungsform der Variante III) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Rest der Formel (6) bis (9): 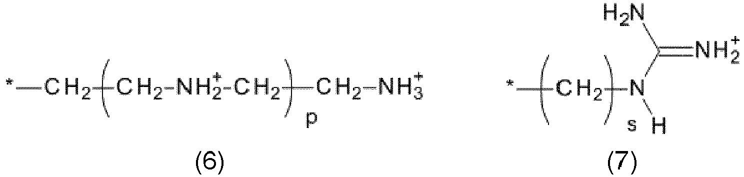 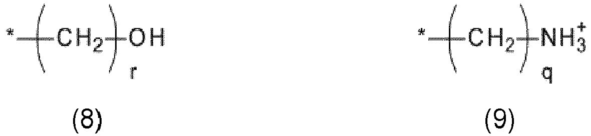 und der Rest R^{(I)} ist ein Rest der Formel (6) bis (9), wobei jeweils p eine ganze Zahl von 1 bis 10, vorzugsweise von 1 bis 7, weiter bevorzugt von 1 bis 3, bedeutet und wobei s, r, q jeweils unabhängig voneinander eine ganze Zahl von 1 bis 20, vorzugsweise von 1 bis 8, weiter bevorzugt von 1 bis 4, bedeutet, ausgewählt.

Bei einer weiter bevorzugten Ausführungsform der Variante III) des 10H-Benzo[g]pteridin-2,4-dion-Derivats der Formel (1) sind die Reste R^{(II)}, R^{(III)}, R^{(IV)}, R^{(V)} und R^{(VI)} unabhängig voneinander Wasserstoff oder ein Polyaminrest der allgemeinen Formel -[(CH₂)ₐ-N(R^{(VII)})-(CH₂)_{b}-]_{c}NH_{(3-g)}(R^{(VIII)})_{g}⁺, und der Rest R^{(I)} ist ein Polyaminrest der allgemeinen Formel -[(CH₂)ₐ-N(R^{(VII)})-(CH₂)_{b}-]_{c}NH_{(3-g)}(R^{(VIII)})_{g}⁺, wobei jeweils c eine ganze Zahl von 1 bis 10, vorzugsweise von 1 bis 7, weiter bevorzugt von 1 bis 3, bedeutet, a und b jeweils unabhängig voneinander eine ganze Zahl von 1 bis 10, vorzugsweise von 1 bis 4, weiter bevorzugt von 2 bis 3, bedeuten, g 0, 1, 2 oder 3 ist und R^{(VII)} und R^{(VIII)} unabhängig voneinander Wasserstoff, Methyl oder Ethyl bedeuten..

Bei einer weiter bevorzugten Ausführungsform der Varianten I), II) und III) der Verbindung der Formel (1) können vorgenannte Alkylreste und Alkenylreste geradkettig oder verzweigt, vorzugsweise geradkettig, sein und sowohl unsubstituiert oder mit wenigstens einem Rest, der aus der Gruppe, die aus Hydroxyl, Sulfanyl, Alkyloxy, vorzugsweise Methoxy, Ethoxy, n-Propyloxy, i-Propyloxy, n-Butyloxy und n-Pentyloxy, Alkylsulfanyl, vorzugsweise Methylsulfanyl, Ethylsulfanyl, n-Propylsulfanyl, i-Propylsulfanyl, n-Butylsulfanyl und n-Pentylsulfanyl, Alkanoyloxy, vorzugsweise Formyloxy, Acetoxy und n-Propanoyloxy, Amino, Alkylamino mit 1 bis 4 C-Atomen, vorzugsweise Methylamino, Ethylamino, n-Propylamino, i-Propylamino und n-Butylamino, Dialkylamino mit 2 bis 8 C-Atomen, vorzugsweise Dimethylamino, Ethylmethylamino, Diethylamino und Di-n-propylamino, Trialkylammonio mit 3 bis 12 C-Atomen, vorzugsweise Trimethylammonio, Triethylammonio, Methyldiethylammonio, Ethyldimethylammonio und Tri-n-propylammonio, und Guaridino, besteht, ausgewählt wird, substituiert sein.

Bei einer weiter bevorzugten Ausführungsform werden die vorgenannte Alkylreste jeweils aus Methyl, Ethyl, n-Propyl, n-Butyl, n-Pentyl, n-Hexyl, n-Heptyl oder n-Octyl ausgewählt.

Bei einer weiter bevorzugten Ausführungsform der Varianten I), II) und III) der Verbindung der Formel (1) können vorgenannte Cycloalkylreste und Cycloalkenylreste Sauerstoff- und/oder Schwefelatome, als Ringatome aufweisen und sowohl unsubstituiert oder mit wenigstens einem Rest, der aus der Gruppe, die aus Hydroxyl, Sulfanyl, Alkyloxy, vorzugsweise Methoxy, Ethoxy, n-Propyloxy, i-Propyloxy, n-Butyloxy und n-Pentyloxy, Alkylsulfanyl, vorzugsweise Methylsulfanyl, Ethylsulfanyl, n-Propylsulfanyl, i-Propylsulfanyl, n-Butylsulfanyl und n-Pentylsulfanyl, Alkanoyloxy, vorzugsweise Formyloxy, Acetoxy und n-Propanoyloxy, Amino, Alkylamino mit 1 bis 4 C-Atomen, vorzugsweise Methylamino, Ethylamino, n-Propylamino, i-Propylamino und n-Butylamino, Dialkylamino mit 2 bis 8 C-Atomen, vorzugsweise Dimethylamino, Ethylmethylamino, Diethylamino und Di-n-propylamino, Trialkylammonio mit 3 bis 12 C-Atomen, vorzugsweise Trimethylammonio, Triethylammonio, Methyldiethylammonio, Ethyldimethylammonio und Tri-n-propylammonio, und Guaridino, besteht, ausgewählt wird, substituiert sein.

Bei einer weiter bevorzugten Ausführungsform werden die vorgenannten Cycloalkylreste und Cycloalkenylreste, die Sauerstoff- und/oder Schwefelatome, als Ringatome aufweisen, jeweils aus der Gruppe, die aus Tetrahydrofuranyl, Tetrahydrothiophenyl, Tetrahydropyranyl, Tetrahydrothiopyranyl, Dioxolanyl und Dioxanyl besteht, ausgewählt.

Bei einer bevorzugten Ausführungsform der Varianten I), II) und III) der Verbindung der Formel (1) weisen die vorgenannten Arylreste jeweils höchstens 4, weiter bevorzugt höchstens 3, weiter bevorzugt höchstens 2, anellierte Ringe auf. Noch weiter bevorzugt weisen die Arylreste jeweils 1 Ring auf.

Bei einer bevorzugten Ausführungsform der Varianten I), II) und III) der Verbindung der Formel (1) werden die vorgenannten Arylreste aus der Gruppe, die aus Phenyl, Benzyl, Naphthyl, Anthracenyl, Phenanthenyl und Pyrenyl besteht, ausgewählt.

Bei einer weiteren bevorzugten Ausführungsform der Varianten I), II) und III) der Verbindung der Formel (1) weisen die vorgenannten Alkenylreste 2 bis 17 C-Atome, weiter bevorzugt 2 bis 13 C-Atome, weiter bevorzugt 2 bis 9 C-Atome, weiter bevorzugt 2 bis 5 C-Atome, auf. Bei einer weiter bevorzugten Ausführungsform werden die vorgenannten Alkenylreste aus der Gruppe, die aus Ethenyl, n-Propenyl und n-Butenyl besteht, ausgewählt.

Bei einer weiteren bevorzugten Ausführungsform der Varianten I), II) und III) der Verbindung der Formel (1) weisen die vorgenannten Aldehyde 1 bis 17 C-Atome, weiter bevorzugt 1 bis 13 C-Atome, weiter bevorzugt 1 bis 9 C-Atome, weiter bevorzugt 1 bis 5 C-Atome, auf. Bei einer weiter bevorzugten Ausführungsform werden die vorgenannten Aldehyde aus der Gruppe, die aus Methanal-1-yl (Formyl), Ethanal-1-yl (2-Oxoethyl), n-Propanal-1-yl (3-Oxopropyl) und n-Butanal-1-yl (4-Oxobutyl) besteht, ausgewählt.

Bei einer weiteren bevorzugten Ausführungsform der Varianten I), II) und III) der Verbindung der Formel (1) weisen die vorgenannten Ketone 2 bis 17 C-Atome, weiter bevorzugt 3 bis 14 C-Atome, weiter bevorzugt 3 bis 9 C-Atome, auf. Bei einer weiter bevorzugten Ausführungsform werden die vorgenannte Ketone aus der Gruppe, die aus Dimethylketyl, Methyl-Ethyl-ketyl, Ethyl-Methyl-ketyl, Diethylketyl, Methyl-Propyl-ketyl, Ethyl-Propyl-ketyl, Propyl-Methyl-ketyl, Propyl-Ethyl-ketyl und Dipropyl-ketyl, der das geradkettig oder verzweigt sein kann, besteht, ausgewählt.

Bei einer weiter bevorzugten Ausführungsform können vorgenannte Aldehydreste und/oder Ketonreste Monosaccharidreste, vorzugsweise Pentose- oder Ketosereste, sein.

Vorzugsweise haben geeignete Monosaccharidreste 3 bis 7 Kohlenstoffatome, vorzugsweise 5 bis 6 Kohlenstoffatome, und weisen eine Carbonylgruppe, vorzugsweise Aldehydgruppe oder Ketogruppe, sowie mindestens eine Hydroxylgruppe auf und können offenkettig oder cyclisch, vorzugsweise als Furanose oder Pyranose, vorliegen.

Bevorzugt leiten sich geeignete Monosaccharidreste von Monosacchariden ab, die aus der Gruppe, die aus D-Glycerinaldehyd, L-Glycerinaldehyd, D-Erythrose, L-Erythrose, D-Threose, L-Threose, D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, Dihydroxyaceton, D-Erythrulose, L-Erythrulose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt. Weiter bevorzugt werden geeignete Monosaccharide aus der Gruppe, die aus D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt werden.

Bei einer weiteren bevorzugten Ausführungsform der Varianten I), II) und III) der Verbindung der Formel (1) weisen die vorgenannten Carbonsäureester 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureester aus der Gruppe, die aus Ethylester, n-Propylester, i-Propylester, n-Butylester, sec.-Butylester, tert.-Butylester und Benzylester besteht, ausgewählt.

Bei einer weiteren bevorzugten Ausführungsform der Varianten I), II) und III) der Verbindung der Formel (1) weisen die vorgenannten Carbonsäureamide 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureamide aus der Gruppe, die aus Amid, N-Methylamid, N-Ethylamid, N-(n-Propyl)amid, N-(i-Propyl)amid, N-(n-Butyl)amid, N-(sec.-Butyl)amid, N-(tert.-Butyl)amid, N-Phenylamid, N-Benzylamid, N,N-Dimethylamid, N-Methyl-N-Ethylamid, N,N-Diethylamid, N-Methyl-N-(n-Propyl)amid, N-Methyl-N-(i-Propyl)amid, N-Methyl-N-(n-Butyl)amid, N-Methyl-N-(sec.-Butyl)amid, N-Methyl-N-(tert.-Butyl)amid, N-Ethyl-N-(n-Propyl)amid, N-Ethyl-N-(i-Propyl)amid, N-Ethyl-N-(n-Butyl)amid, N-Ethyl-N-(sec.-Butyl)amid, N-Ethyl-N-(tert.-Butyl)amid, N-(n-Propyl)-N-(n-Propyl)amid, N-(n-Propyl)-N-(i-Propyl)amid, N-(n-Propyl)-N-(n-Butyl)amid, N-(n-Propyl)-N-(sec.-Butyl)amid, N-(n-Propyl)-N-(tert.-Butyl)amid, N-(i-Propyl)-N-(n-Propyl)amid, N-(i-Propyl)-N-(i-Propyl)amid, N-(i-Propyl)-N-(n-Butyl)amid, N-(i-Propyl)-N-(sec.-Butyl)amid, N-(i-Propyl)-N-(tert.-Butyl)amid, N-(n-Butyl)-N-(n-Propyl)amid, N-(n-Butyl)-N-(i-Propyl)amid, N-(n-Butyl)-N-(n-Butyl)amid, N-(n-Butyl)-N-(sec.-Butyl)amid, N-(n-Butyl)-N-(tert.-Butyl)amid, N-(sec.-Butyl)-N-(n-Propyl)amid, N-(sec.-Butyl)-N-(i-Propyl)amid, N-(sec.-Butyl)-N-(n-Butyl)amid, N-(sec.-Butyl)-N-(sec.-Butyl)amid, N-(sec.-Butyl)-N-(tert.-Butyl)amid, N-(tert.-Butyl)-N-(n-Propyl)amid, N-(tert.-Butyl)-N-(i-Propyl)amid, N-(tert.-Butyl)-N-(n-Butyl)amid, N-(tert.-Butyl)-N-(sec.-Butyl)amid, N-( tert.-Butyl)-N-(tert.-Butyl)amid, N,N-Diphenylamid, N,N-Dibenzylamid, N-Phenyl-N-Benzylamid, N-Methyl-N-Phenylamid, N-Methyl-N-Benzylamid, N-Ethyl-N-Phenylamid, N-Ethyl-N-Benzylamid, N-Phenyl-N-(n-Propyl)amid, N-Phenyl-N-(i-Propyl)amid, N-Phenyl-N-(n-Butyl)amid, N-Phenyl-N-(sec.-Butyl)amid, N-Phenyl-N-(tert.-Butyl)amid, N-Benzyl-N-(n-Propyl)amid, N-Benzyl-N-(i-Propyl)amid, N-Benzyl-N-(n-Butyl)amid, N-Benzyl-N-(sec.-Butyl)amid und N-Benzyl-N-(tert.-Butyl)amid besteht, ausgewählt.

Bei einer weiteren bevorzugten Ausführungsform der Varianten I), II) und III) der Verbindung der Formel (1) wird der vorgenannten Heteroarylrest, der kein Stickstoffatom enthält, mit 4 bis 20 C-Atomen aus der Gruppe, die aus Thiophenyl, Furanyl, Benzothiofuranyl und Benzofuranyl besteht, ausgewählt.

Bei einer weiteren bevorzugten Ausführungsform der Varianten I), II) und III) der Verbindung der Formel (1) weisen die vorgenannten Etherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Etherreste beispielsweise aus der Gruppe, die aus Methoxymethyl, Methoxyethyl, Methoxy-n-propyl, Ethoxymethyl, n-Propoxymethyl, 2-Ethoxyethoxymethyl, 2-(2-Ethoxyethoxy)ethyl, i-Propoxymethyl, tert.-Butyloxymethyl, Dioxa-3,6-heptyl und Benzyloxymethyl besteht, ausgewählt. Bei einer weiter bevorzugten Ausführungsform können die vorgenannten Etherreste einfache Etherreste, Oligoetherreste, Polyetherreste oder Mischungen davon sein.

Bei einer weiteren bevorzugten Ausführungsform der Varianten I), II) und III) der Verbindung der Formel (1) weisen die vorgenannten Thioetherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Thioetherreste beispielsweise aus der Gruppe, Methylsulfanylmethyl, Methylsulfanylethyl, 3-Methylsulfanyl-n-propyl, Ethylsulfanylmethyl, n-Propylsulfanylmethyl, 2-Ethylsulfanylethylsulfanylmethyl, 2-(2-ethylsulfanylethylsulfanyl)ethyl, 2-Methylsulfanylpropyl, tert.-Butylsulfanymethyl und Benzylsulfanymethyl besteht, ausgewählt.

Bei einer weiteren bevorzugten Ausführungsform der Varianten I), II) und III) weist die Verbindung der Formel (1) ein Molekulargewicht von weniger als 1300 g/mol, vorzugsweise weniger als 990 g/mol, weiter bevorzugt weniger als 810 g/mol, weiter bevorzugt weniger als 690 g/mol, noch weiter bevorzugt weniger als 610 g/mol, noch weiter bevorzugt weniger als 600 g/mol, noch weiter bevorzugt weniger als 570 g/mol, auf.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch Mesomere und/oder Tautomere der Verbindung mit der Formel (1), sowohl die reinen Verbindungen als auch die Isomerengemische in jedem Verhältnis.

Bei einer weiteren bevorzugten Ausführungsform der Variante III) der Verbindung der Formel (1) wird der organische Rest mit:
a) wenigstens zwei neutralen, protonierbaren Stickstoffatomen, die nicht direkt an den Isoalloxazinring gebunden sind, oder
b) wenigstens zwei positiv geladenen, vorzugsweise quartären, Stickstoffatomen, die nicht direkt an den Isoalloxazinring gebunden sind, jeweils aus der Gruppe, die aus den Resten der Formel (30a) bis (37b) besteht, 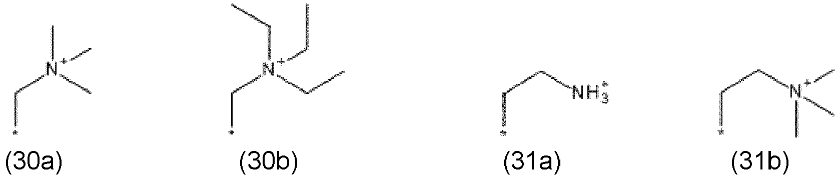 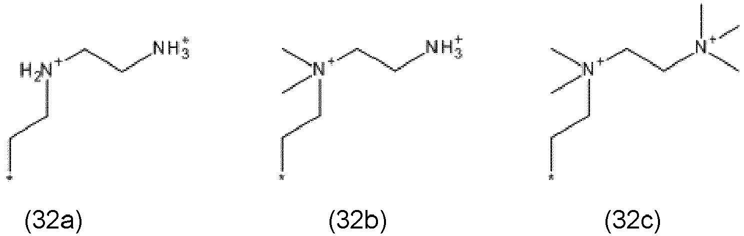 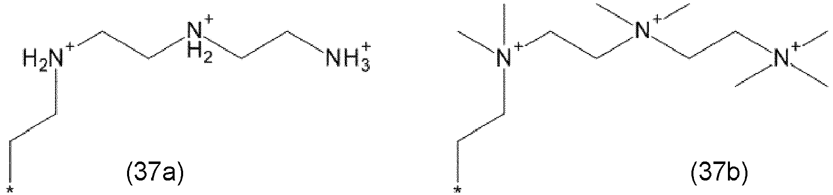 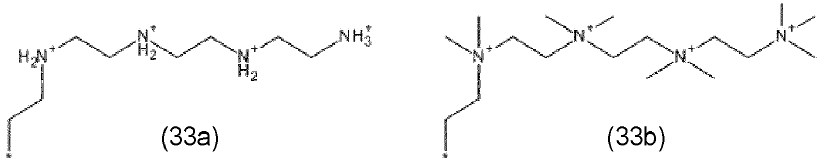 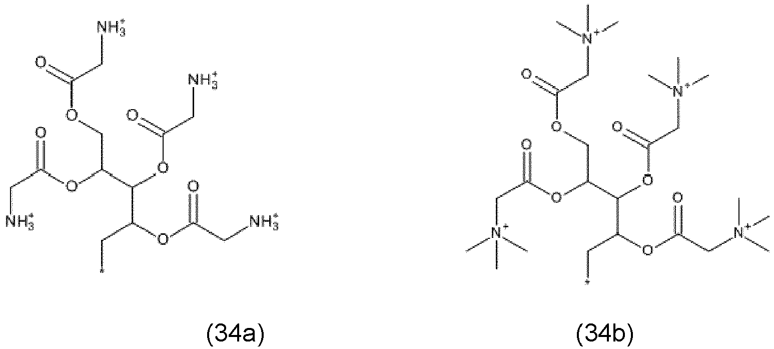 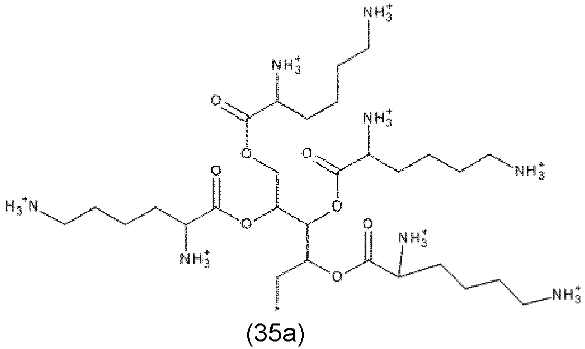 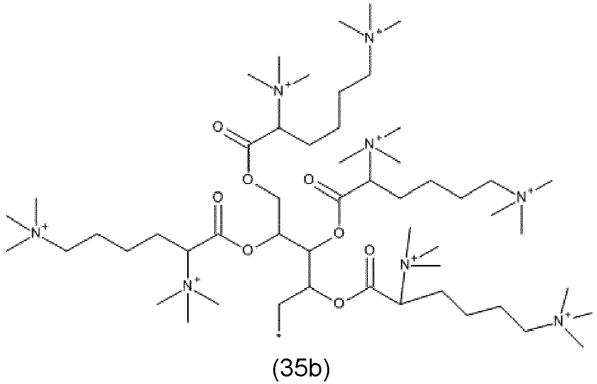 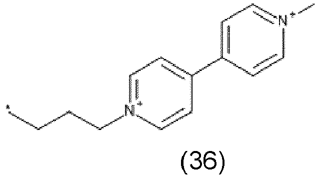
ausgewählt.

Bei einer weiteren bevorzugten Ausführungsform der Variante II) der Verbindung der Formel (1) ist der organische Rest mit:
a) wenigstens zwei neutralen, protonierbaren Stickstoffatomen, die nicht direkt an den Isoalloxazinring gebunden sind, oder
b) wenigstens zwei positiv geladenen, vorzugsweise quartären, Stickstoffatomen, die nicht direkt an den Isoalloxazinring gebunden sind, ein Rest der Formel (32b):

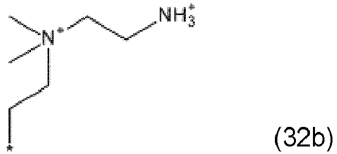

Bei einer weiteren bevorzugten Ausführungsform der Variante I) der Verbindung der Formel (1) wird der organische Rest mit:
a) wenigstens zwei neutralen, protonierbaren Stickstoffatomen, die nicht direkt an den Isoalloxazinring gebunden sind, oder
b) wenigstens zwei positiv geladenen, vorzugsweise quartären, Stickstoffatomen, die nicht direkt an den Isoalloxazinring gebunden sind, jeweils aus der Gruppe, die aus den Resten der Formel (32a), (32c),(33a), (33b) bis (37b) besteht, 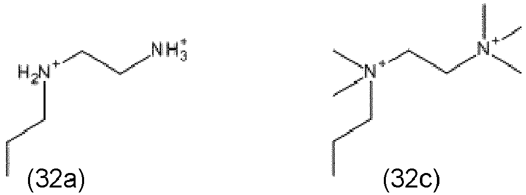 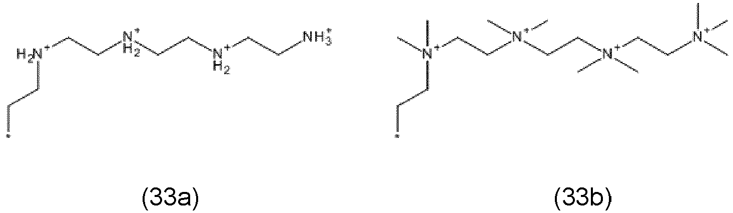 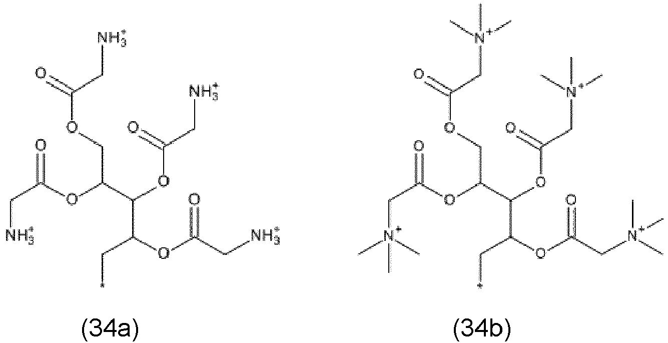 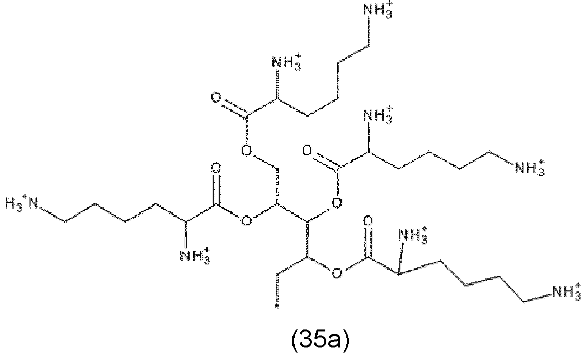 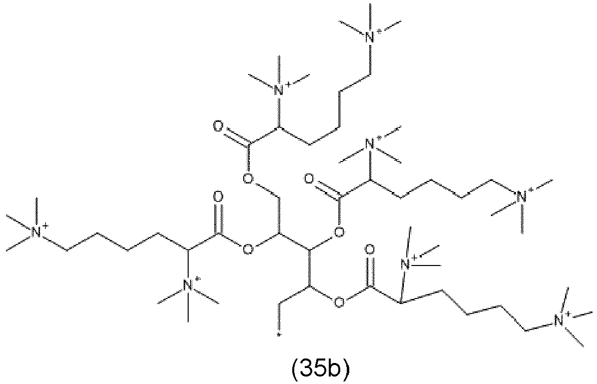 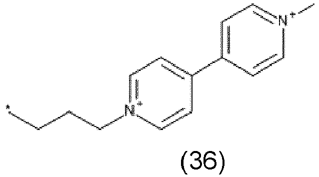 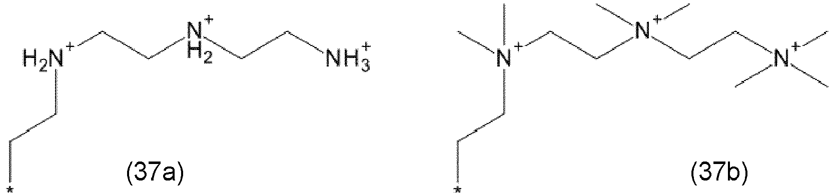
ausgewählt.

Bei einer weiteren bevorzugten Ausführungsform der Variante III) wird das erfindungsgemäße 10H-Benzo[g]pteridin-2,4-dion-Derivat der Formel (1) aus der Gruppe, die aus Verbindungen mit den Formeln (40) bis (44) besteht, ausgewählt:

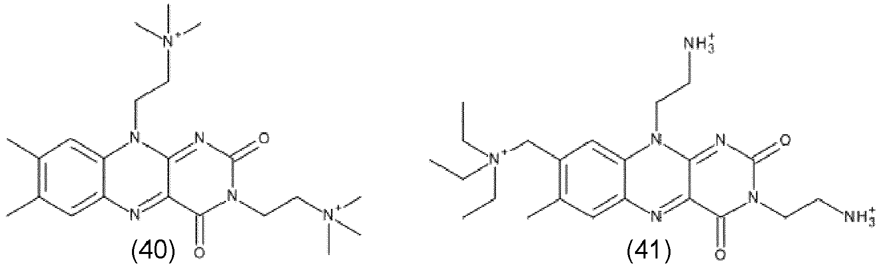

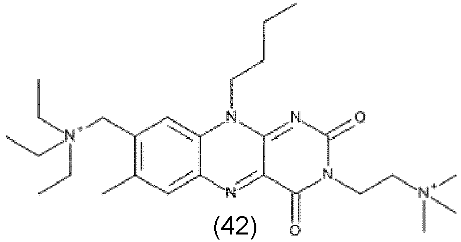

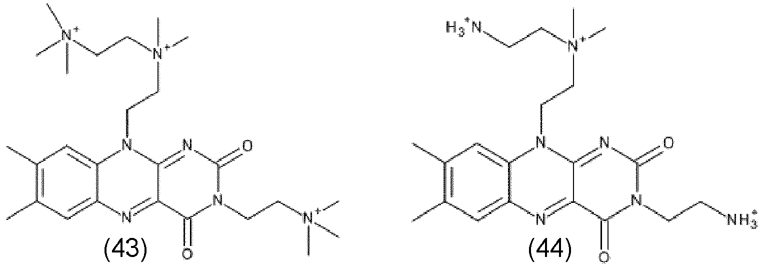

Bei einer weiteren bevorzugten Ausführungsform der Variante I) wird das erfindungsgemäße 10H-Benzo[g]pteridin-2,4-dion-Derivat der Formel (1) aus der Gruppe, die aus Verbindungen mit den Formeln (45) bis (49) und (115) bis (117) besteht, ausgewählt:

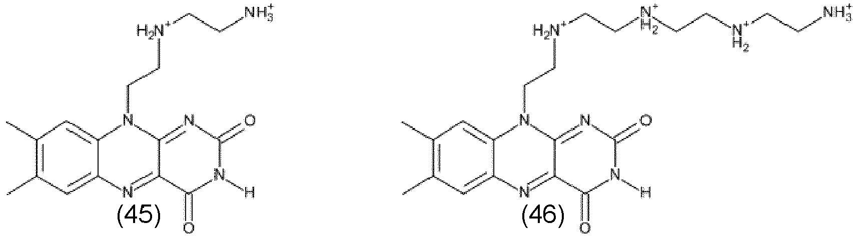

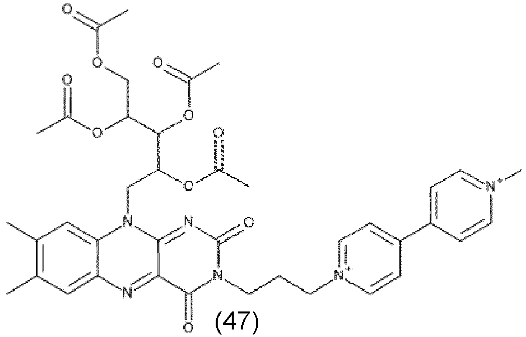

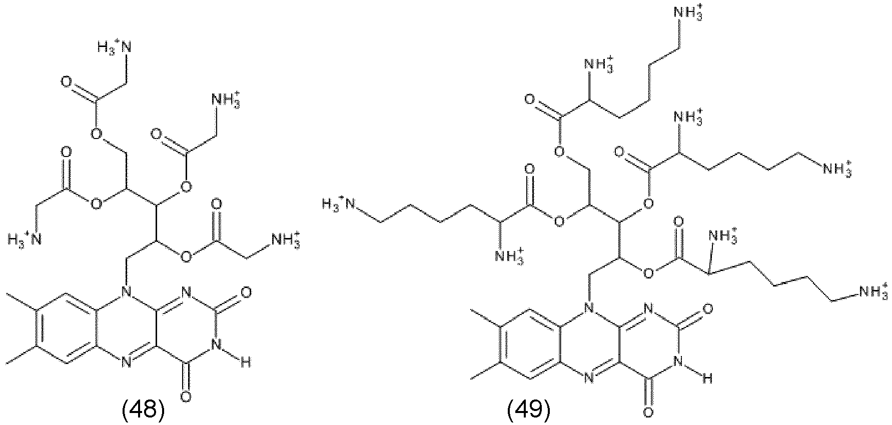

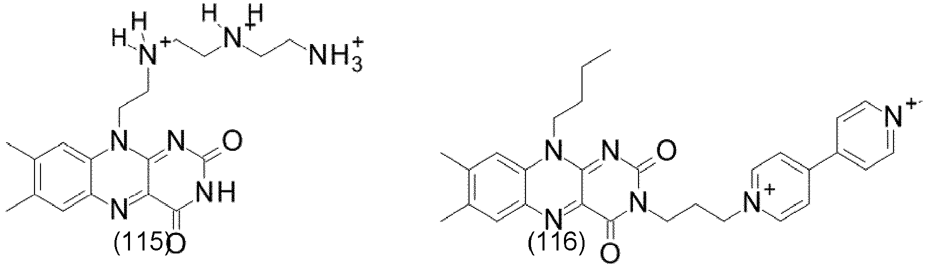

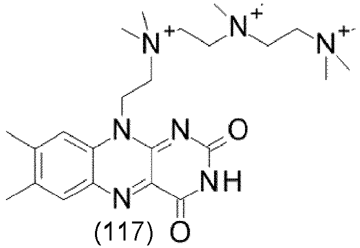

Eine Variante des Verfahrens zur Herstellung einer Verbindung nach Anspruch 1 umfasst folgende Schritte:
(A) Reduzieren eines substituierten Nitroanilins der Formel (9) zu einem substituierten o-Phenylendiamin der Formel (10), 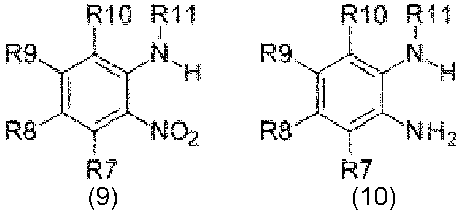
   wobei jeder der Reste R7 bis R10 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet und
   wobei der Rest R11 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
(B) Kondensieren des in Schritt (A) erhaltenen substituierten o-Phenylendiamin der Formel (10) mit Alloxan oder dessen Hydrat unter Erhalt einer Verbindung mit der Formel (11): 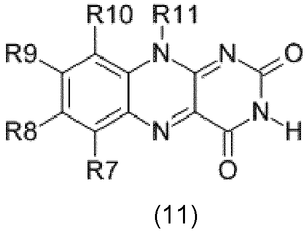
(C) Optional Umsetzen der in Schritt (B) erhaltenen Verbindung der Formel (11) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (12): 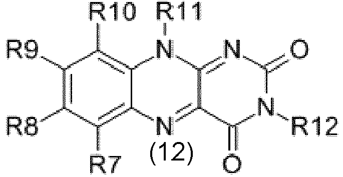 wobei der Rest R12 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das kein Stickstoffatom enthält, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
(D) Optional Umsetzen der in Schritt (B) erhaltenen Verbindung der Formel (11) oder der in Schritt (C) erhaltenen Verbindung der Formel (12) mit Tosylchlorid, Mesylchlorid oder Iod, optional in Gegenwart eines Katalysators, und nachfolgend mit einer organischen Verbindung, die wenigstens ein neutrales, protonierbares Stickstoffatom und/oder b) wenigstens ein positiv geladenes Stickstoffatom enthält, wenn mindestes 1 Rest R7, R8, R9, R10, R11 oder R12 -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, und
mit der Maßgabe, dass
I) nur 1 Rest R1, R2, R3, R4, R5 oder R6 ein organischer Rest mit:
   a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
   b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet
      oder
II) nur 1 Rest R1, R2, R3, R4, R5 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet
   oder
III) wenigstens 2 Reste R1, R2, R3, R4, R5 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf,neutralen, protonierbaren Stickstoffatom(en), das (die) nicht direkt an den Isoalloxazinring gebunden ist (sind), und/oder
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeuten.

Eine weitere Variante des Verfahrens zur Herstellung einer Verbindung nach Anspruch 1 umfasst folgende Schritte:
(A) Kondensieren eines Amins mit der Formel R11-NH₂ mit einem Chloruracil-Derivate der Formel (13), optional in Gegenwart eines Katalysators, vorzugsweise Lewis-Säure oder Broenstedt-Säure, unter Erhalt einer Verbindung mit der Formel (14): 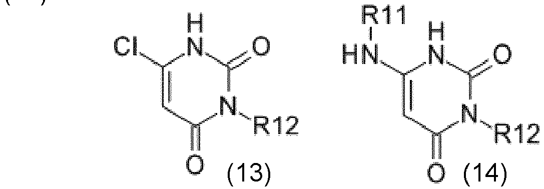 wobei jeder der Reste R11 oder R12 jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet,
(B) Umsetzen der in Schritt (A) erhaltenen Verbindung der Formel (14) mit einer Nitrosoverbindung der Formel (15) unter Erhalt einer Verbindung der Formel (12): 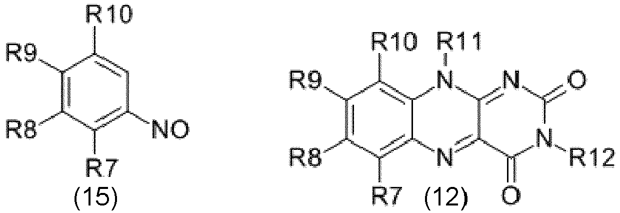 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
(C) Optional Umsetzen der in Schritt (B) erhaltenen Verbindung der Formel (12) mit Tosylchlorid, Mesylchlorid oder Iod, optional in Gegenwart eines Katalysators, und nachfolgend mit einer organischen Verbindung, die wenigstens ein tertiäres Stickstoffatom enthält, umgesetzt wird, wenn mindestes 1 Rest R7, R8, R9, R10, R11 oder R12 -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, und
mit der Maßgabe, dass
I) nur 1 Rest R1, R2, R3, R4, R5 oder R6 ein organischer Rest mit:
   a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
   b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet oder
II) nur 1 Rest R1, R2, R3, R4, R5 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet
   oder
III) wenigstens 2 Reste R1, R2, R3, R4, R5 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und/oder
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeuten.

Eine weitere Variante des erfindungsgemäßen Verfahrens zur Herstellung eines 10H-Benzo[g]pteridin-2,4-dion-Derivats der Formel (1), umfasst folgende Schritte:
(A) Herstellen eines substituierten Anilins mit der Formel (122a) oder (122b) durch (a) Peptidkupplung/Reduktion an ein Anilin mit der Formel (121) oder (b) reduktive Aminierung eines Anilins mit der Formel (121) mit Aldehyden oder (c) Pd-katalysierte Kupplung von einem Halogenid der Formel (124) an ein Amin der Formel R11-NH₂ oder (d) Pd-katalysierte Kupplung von einem Amin der Formel (120) an ein Halogenid der Formel R11-NH₂
   a) 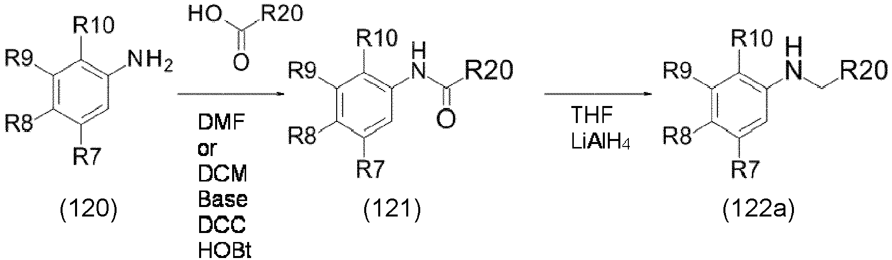
   b) 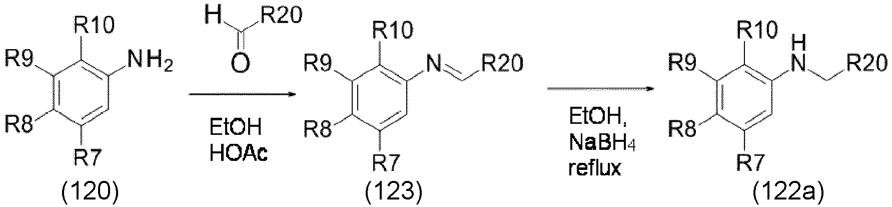
   c) 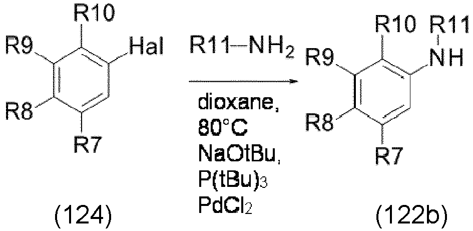
   d) 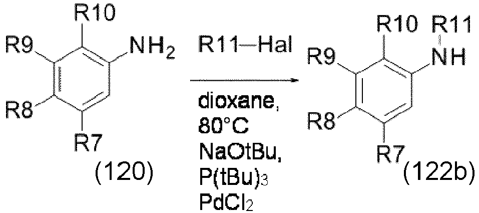
      wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))h-OH, -(C(D)(E))k-Aryl-(C(D)(E))l-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
      wobei der Rest R11 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
      wobei der Rest R20 Wasserstoff, Alkyl mit 1 bis 19 C-Atomen, Alkenyl mit 2 bis 19 C-Atomen, Ether mit 1 bis 19 C-Atomen, Thioether mit 1 bis 19 C-Atomen, Cycloalkyl mit 3 bis 19 C-Atomen, Cycloalkenyl mit 3 bis 19 C-Atomen, Aryl mit 5 bis 19 C-Atomen, Heteroaryl mit 4 bis 19 C-Atomen, das keine N-Atome enthält, -(C(D)(E))ₕ₋₁-OH, -(C(D)(E))ₖ₋₁-Aryl-(C(D)(E))ₗ₋₁-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
      wobei der Rest Hal Fluor, Chlor, Brom oder Iod bedeutet,
(B) Umsetzen des in Schritt (A) erhaltenen substituierten Anilins mit der Formel (122a) mit Violursäure unter Erhalt einer Verbindung der Formel (11z): 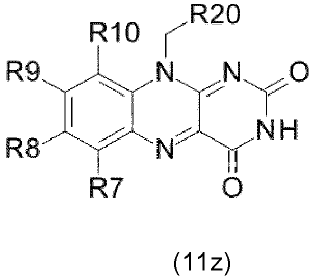 oder Umsetzen des in Schritt (A) erhaltenen substituierten Anilins mit der Formel (122b) mit Violursäure unter Erhalt einer Verbindung der Formel (11): 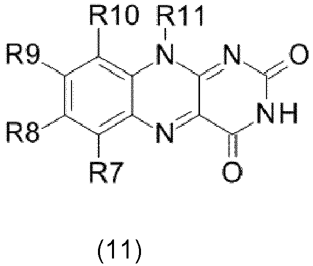
(C) Optional Umsetzen der in Schritt (B) erhaltenen Verbindung der Formel (11z) mit Tosylchlorid, Mesylchlorid oder Iod, optional in Gegenwart eines Katalysators, und nachfolgend mit einer organischen Verbindung, die wenigstens ein tertiäres Stickstoffatom enthält, umgesetzt wird, wenn mindestes 1 Rest R7, R8, R9, R10, R11 oder R20 -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (12z): 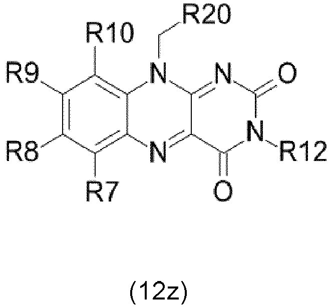 wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeuten,
   oder
   Umsetzen der in Schritt (B) erhaltenen Verbindung der Formel (11) mit Tosylchlorid, Mesylchlorid oder Iod, optional in Gegenwart eines Katalysators, und nachfolgend mit einer organischen Verbindung, die wenigstens ein tertiäres Stickstoffatom enthält, umgesetzt wird, wenn mindestes 1 Rest R7, R8, R9, R10, R11 oder R20 -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (12): 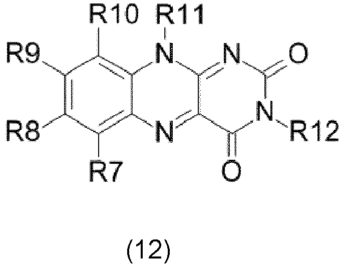 wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, und
mit der Maßgabe, dass
I) nur 1 Rest R1, R2, R3, R4, R5 oder R6 ein organischer Rest mit:
   a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
   b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet oder
II) nur 1 Rest R1, R2, R3, R4, R5 oder R6 ein organischer Rest mit:
   a) wenigstens einem, vorzugsweise wenigstens zwei, vorzugsweise
   b) wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
      b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet
      oder
III) wenigstens 2 Reste R1, R2, R3, R4, R5 oder R6 ein organischer Rest mit:
   a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und/oder
   b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeuten.

Bei einer bevorzugten Ausführungsform der beiden Verfahrensvarianten ist keiner der Reste R7 bis R12 bzw. R7 bis R11 sowie R20 ein organischer Rest der allgemeinen Formel -(C(D)(E))ₕ-X oder -(C(D)(E))ₖ-Aryl-(C(D)(E))ₗ-X, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R(IX) Wasserstoff, Methyl, Ethyl, n-Propyl, n-Butyl, Phenyl oder Benzyl bedeutet,oder Thiol, weiter bevorzugt Wasserstoff oder Hydroxyl, bedeuten, und 1 Rest R7 bis R10 ist Methyl wobei das Verfahren dann folgende Schritte umfassen kann,
(A) Radikalische Halogenierung der Verbindung (11) oder (12) in Gegenwart eines Radikalstarters, vorzugsweise eines Peroxides oder einer AzoVerbindung, unter Erhalt einer Verbindung mit der Formel (11a-11d) oder (12a-12d): 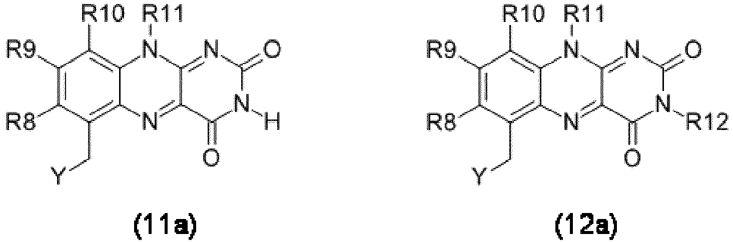 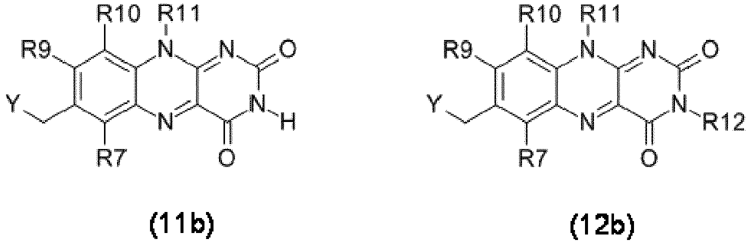 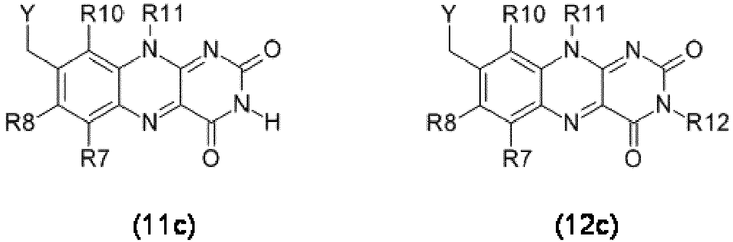 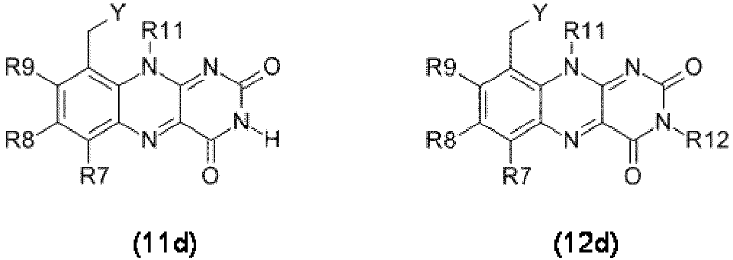 wobei der Rest Y Cl, Br oder I bedeutet, und
B) Umsetzen der in Schritt (A) erhaltenen Verbindung mit der Formel (11a-11d) oder (12a-12d) mit einer organischen Verbindung, die wenigstens ein tertiäres Stickstoffatom enthält, unter Erhalt des erfindungsgemäßen 10H-Benzo[g]pteridin-2,4-dion-Derivats der Formel (1).

Bei der Verwendung unterschiedlicher Amino-Schutzgruppe PG in einer Synthese, ergibt sich die Möglichkeit der orthogonalen Schutzgruppenstrategie, wobei unterschiedliche Amino-Funktionen eines Moleküls gezielt nacheinander freigesetzt können und zur Reaktion gebracht werden.

Geeignete Methoden zur Entfernung der Amino-Schutzgruppe PG sind aus dem Stand der Technik bekannt. Beispielsweise kann Benzyloxycarbonyl (Cbz) durch katalytische Hydrierung unter hydrogenolytischer Spaltung der Benzyl-Heteroatom-Bindung mit anschließender Decarboxylierung der so entstehenden instabilen Carbaminsäure oder Behandlung mit Säuren wieder entfernt werden. Di-tert-butyloxycarbonyl (Boc) kann beispielsweise durch saure Hydrolyse entfernt werden. Allyloxycarbonyl (Alloc) kann beispielsweise durch Einwirkung von Tetrakis(triphenylphosphan)palladium(0) und einem Nukleophil abgespalten werden.

Bei einer weiteren bevorzugten Ausführungsform finden die Schritte (B) und/oder (C) in Gegenwart eines oder mehrerer Lösungsmittel statt. Der Schritt (B) kann beispielsweise in Gegenwart von Dichlormethan (DCM), Dimethylformamid (DMF) oder Acetonitril (MeCN) durchgeführt werden. Der Schritt (C) kann beispielsweise in Gegenwart von Wasser/Dichlormethan oder Toluol/Tetrabutylammoiumiodid (TBAI) durchgeführt werden. Vorzugsweise wird Schritt (C) in Gegenwart einer Base, beispielsweise, LiOH, NaOH oder einer organischen Stickstoffbase, beispielsweise Triethylamin, durchgeführt.

Einzellige oder mehrzellige Mikroorganismen können Auslöser von infektiösen Erkrankungen sein. Durch Applikation wenigstens eines Erreger-spezifischen Gegenmittels, beispielsweise Antibiotikum, Antimikotikum oder Virustatikum, kann die Anzahl der Erreger reduziert und/oder der Erreger inaktiviert werden. Die Applikation eines Erreger-spezifischen Gegenmittels kann systemisch und/oder topisch erfolgen.

Bei der systemischen Applikation wird das Erreger-spezifische Gegenmittel in das Blut- und/oder Lymphsystem des zu behandelnden Körpers übertragen und hierüber im gesamten Körper verteilt. Bei einer system ischen Aufnahme des Erreger-spezifischen Gegenmittels kann es zu einem Abbau des Gegenmittels und/oder zu Nebenwirkungen, beispielsweise durch eine biochemische Umwandlung (Metabolisierung) des Gegenmittels, kommen.

Bei der topischen Applikation des Erreger-spezifischen Gegenmittels erfolgt die Anwendung des Gegenmittels dort, wo es therapeutisch wirken soll, beispielsweise auf einer infizierten Hautpartie, während die gesunde Haut nicht belastet wird. Somit können systemische Nebenwirkungen weitgehend vermieden werden.

Oberflächliche Haut- oder Weichteilinfektionen müssen nicht notwendigerweise mit, einer system ischen Applikation eines Erreger-spezifischen Gegenmittels behandelt werden, da das Gegenmittel direkt auf die infizierte Hautpartien aufgetragen werden kann.

Die bisher bekannten Erreger-spezifischen Gegenmittel weisen sowohl bei systemischer als auch topischer Applikation teilweise starke Neben- und Wechselwirkungen auf. Darüber hinaus kann es auch bei topischer Applikation durch eine unzuverlässige Medikamenteneinnahme (Compliance) des Patienten, insbesondere bei Verwendung von Antibiotika, zur Resistenzbildung kommen.

Eine Alternative stellt hier die photodynamische Inaktivierung von Mikroorganismen dar, bei der Resistenzen gegenüber der photodynamischen Inaktivierung unbekannt sind. Unabhängig von der Art der zu bekämpfenden Mikroorganismen und der damit verbundenen infektiösen Erkrankungen wird die Anzahl der Erreger reduziert und/oder die Erreger werden abtötet. Beispielsweise können Mischungen aus verschieden Mikroorganismen, beispielsweise Pilze und Bakterien oder unterschiedliche Bakterienstämme, bekämpft werden.

Eine erfindungsgemäß als Photosensibilisator, vorzugsweise zur photodynamischen Inaktivierung von Mikroorganismen, verwendete Verbindung weist die Formel (1): auf, 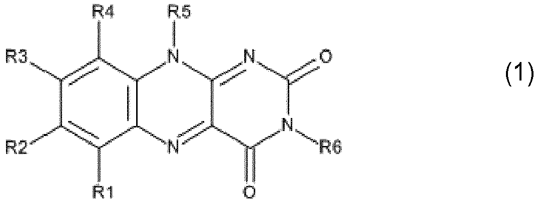
wobei I) nur 1 Rest R1, R2, R3, R4, R5 oder R6 ein organischer Rest mit:
   a) wenigstens zwei neutralen, protonierbaren Stickstoffatomen, die nicht direkt an den Isoalloxazinring gebunden sind, oder
   b) wenigstens zwei positiv geladenen Stickstoffatomen, die nicht direkt an den Isoalloxazinring gebunden sind, bedeutet
   und wobei jeder der Reste R1, R2, R3 oder R4, der kein organischer Rest mit a) wenigstens zwei neutralen, protonierbaren Stickstoffatomen, die nicht direkt an den Isoalloxazinring gebunden sind, oder b) wenigstens zwei positiv geladenen Stickstoffatomen, die nicht direkt an den Isoalloxazinring gebunden sind, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens zwei neutralen, protonierbaren Stickstoffatomen, die nicht direkt an den Isoalloxazinring gebunden sind, oder b) wenigstens zwei positiv geladenen Stickstoffatomen, die nicht direkt an den Isoalloxazinring gebunden sind, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
wobei II) nur 1 Rest R1, R2, R3, R4, R5 oder R6 ein organischer Rest mit:
   a) wenigstens einem neutralen, protonierbaren Stickstoffatom, das nicht direkt an den Isoalloxazinring gebunden ist, und
   b) wenigstens einem positiv geladenen Stickstoffatom, das nicht direkt an den Isoalloxazinring gebunden ist, bedeutet
   und wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens einem neutralen, protonierbaren Stickstoffatom, das nicht direkt an den Isoalloxazinring gebunden ist, und b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
wobei III) wenigstens 2 Reste R1, R2, R3, R4, R5 oder R6 ein organischer Rest mit:
   a) wenigstens einem neutralen, protonierbaren Stickstoffatom, das nicht direkt an den Isoalloxazinring gebunden ist, und/oder
   b) wenigstens einem positiv geladenen Stickstoffatom, das nicht direkt an den Isoalloxazinring gebunden ist, bedeutet und
   wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und/oder b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
   wobei jeder der Reste R5 oder R6, der kein organischer Rest mit a) wenigstens einem neutralen, protonierbaren Stickstoffatom, das nicht direkt an den Isoalloxazinring gebunden ist, und/oder b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet. Bei einer weiter bevorzugten Ausführungsform weist ein erfindungsgemäß verwendetes 10H-Benzo[g]pteridin-2,4-dion-Derivat der Formel (1) kein neutrales, protonierbares Stickstoffatom, das direkt an den Isoalloxazinring gebunden ist, beispielsweise als Amino-Rest, Methylamino-Rest oder Dimethylamino-Rest, und kein positiv geladenes Stickstoffatom, das direkt an den Isoalloxazinring gebunden ist, beispielsweise als Pyridin-1-ium-1-yl-Rest oder Trimethylammonio-Rest, auf.

Bei einer bevorzugten Ausführungsform der vorliegenden Erfindung wird wenigstens ein 10H-Benzo[g]pteridin-2,4-dion-Derivat gemäß einem der Ansprüche 1 bis 5 oder ein pharmakologisch verträgliches Salz und/oder Ester und/oder Komplex davon als Photosensibilisator, vorzugsweise bei der photodynamischen Inaktivierung von Mikroorganismen verwendet.

Bei einer weiter bevorzugten Ausführungsform der vorliegenden Erfindung wird wenigstens ein 10H-Benzo[g]pteridin-2,4-dion-Derivat der Formel (40) bis (49) und (115) bis (117) oder ein pharmakologisch verträgliches Salz und/oder Ester und/oder Komplex davon als Photosensibilisator, vorzugsweise bei der photodynamischen Inaktivierung von Mikroorganismen verwendet:

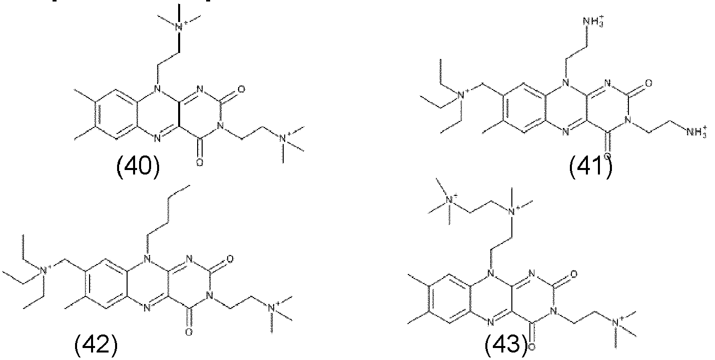

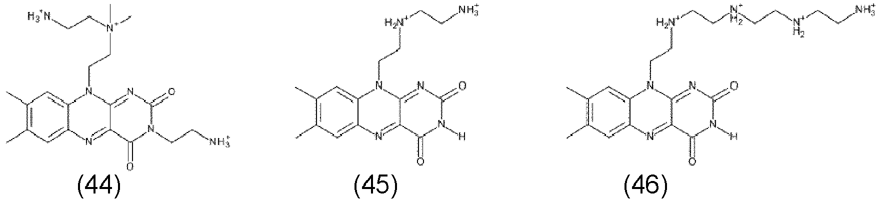

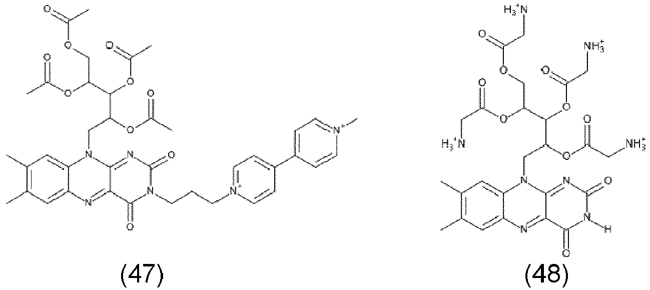

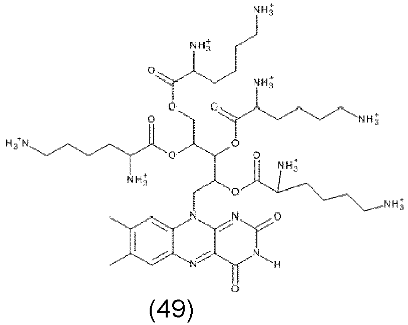

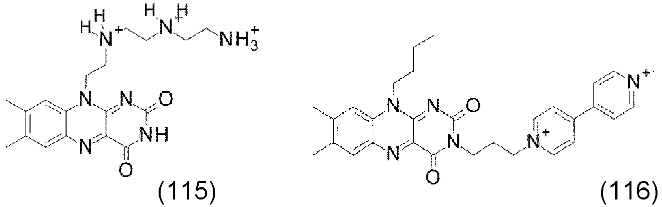

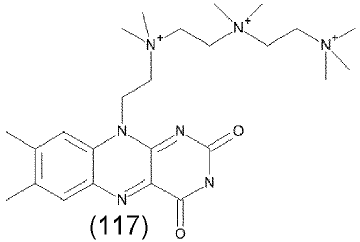

Bei einer weiter bevorzugten Ausführungsform der vorliegenden Erfindung wird wenigstens ein 10H-Benzo[g]pteridin-2,4-dion-Derivat der Formel (90) bis (102) oder ein pharmakologisch verträgliches Salz und/oder Ester und/oder Komplex davon als Photosensibilisator, vorzugsweise bei der photodynamischen Inaktivierung von Mikroorganismen verwendet:

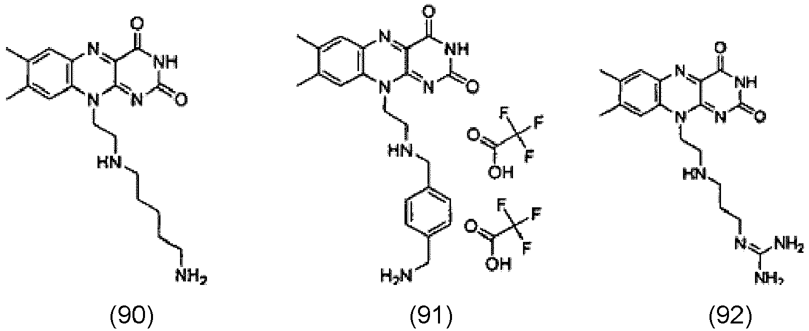

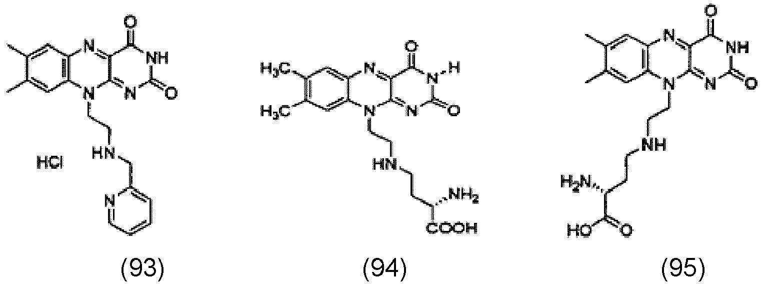

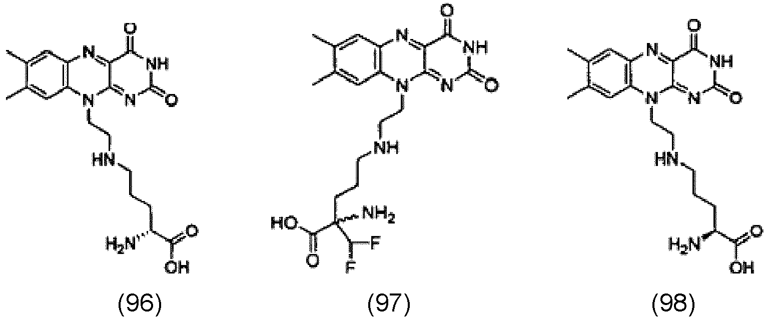

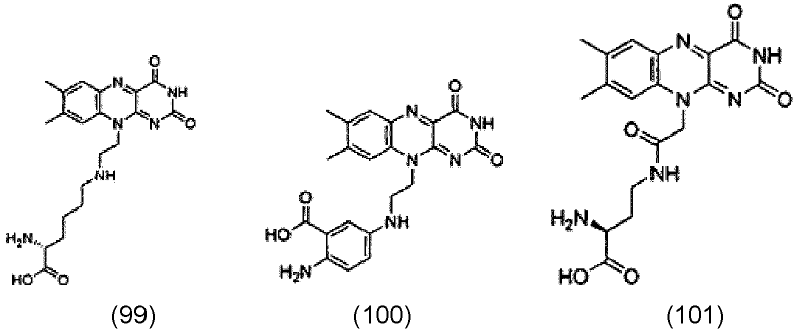

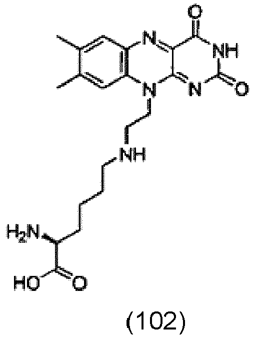

Die Herstellung der 10H-Benzo[g]pteridin-2,4-dion-Derivate der Formel (90) bis (102) wird in der WO 2010/019208 A1 beschrieben, auf die hiermit Bezug genommen wird.

Bei einer weiter bevorzugten Ausführungsform der vorliegenden Erfindung wird wenigstens ein 10H-Benzo[g]pteridin-2,4-dion-Derivat der Formel (103) bis (114) oder ein pharmakologisch verträgliches Salz und/oder Ester und/oder Komplex davon als Photosensibilisator, vorzugsweise bei der photodynamischen Inaktivierung von Mikroorganismen verwendet:

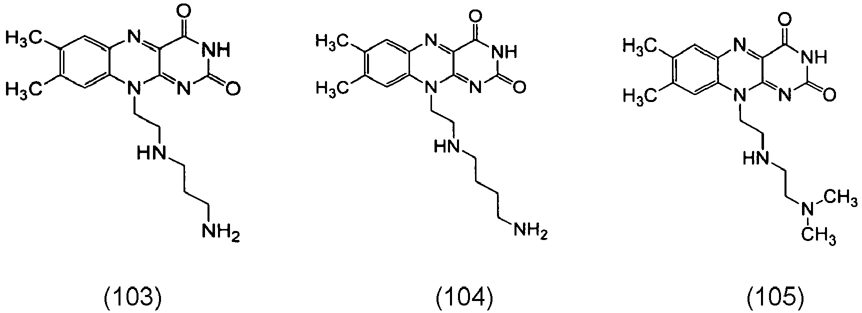

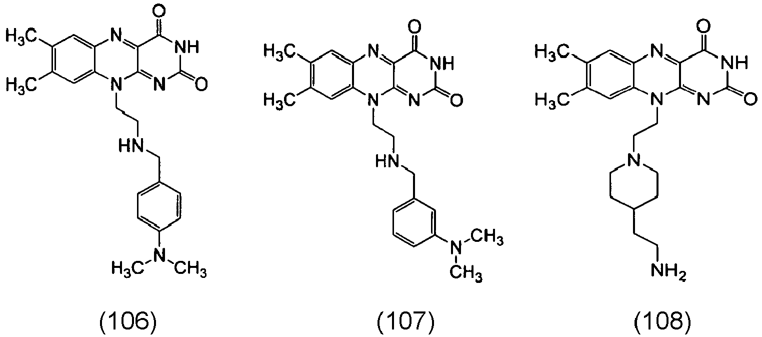

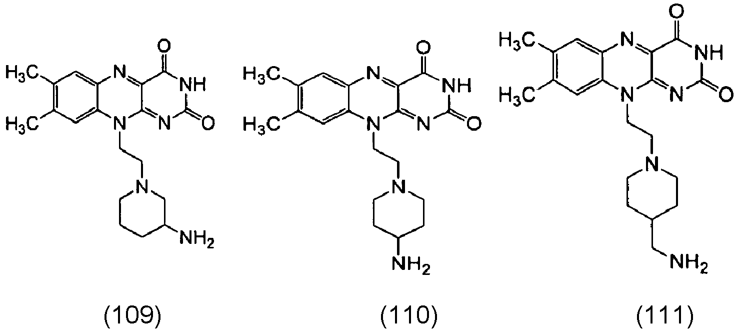

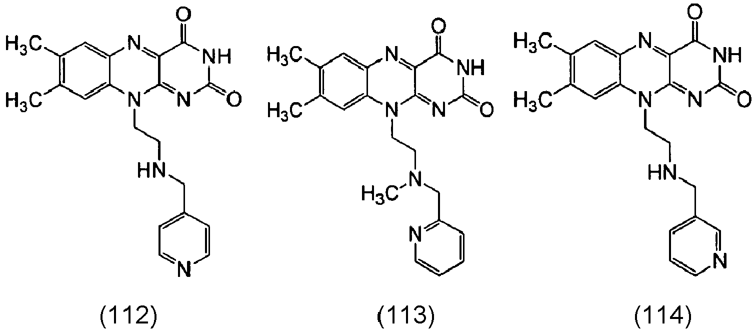

Die Herstellung der 10H-Benzo[g]pteridin-2,4-dion-Derivate der Formel (103) bis (114) wird in der WO 2011/008247 A1 beschrieben, auf die hiermit Bezug genommen wird.

Bei einer bevorzugten Ausführungsform der vorliegenden Erfindung wird wenigstens ein 10H-Benzo[g]pteridin-2,4-dion-Derivat der Formel (1) oder ein pharmakologisch verträgliches Salz und/oder Ester und/oder Komplex davon als Photosensibilisator bei der photodynamischen Inaktivierung von Mikroorganismen, vorzugsweise bei der photodynamischen Therapie, verwendet.

Das erfindungsgemäß verwendete 10H-Benzo[g]pteridin-2,4-dion-Derivat weist nach Bestrahlung mit elektromagnetischer Strahlung geeigneter Wellenlänge und Energiedichte eine hohe Ausbeute an Singulett-Sauerstoff auf.

Vorzugsweise liegt die elektromagnetische Strahlung im sichtbaren Spektralbereich, ultravioletten und/oder infraroten Bereich. Weiter bevorzugt weist die elektromagnetische Strahlung eine Wellenlänge aus einem Bereich von 280 bis 1000 nm, weiter bevorzugt von 380 bis 1000 nm, auf.

Weiter bevorzugt weist die elektromagnetische Strahlung eine Energiedichte aus einem Bereich von 1 µW/cm² bis 1 MW/cm², weiter bevorzugt von 1 mW/cm² bis 1 kW/cm², auf.

Die Bestrahlungszeit kann in Abhängigkeit von der Art der Mikroorganismen und/oder der Schwere der Infektion variiert werden. Vorzugsweise liegt die Bestrahlungszeit in einem Bereich von 1 µs bis 1 h, weiter bevorzugt von 1 ms bis 1000 s.

Vorzugsweise wird die elektromagnetische Strahlung durch eine Strahlungsquelle erzeugt, die aus der Gruppe, die aus Sonne und künstlichen Strahlungsquellen, beispielsweise UV-Lampe, IR-Lampe, Leuchtstofflampen, Leuchtdioden, Laser oder chemisches Licht, besteht, ausgewählt wird.

Darüber hinaus haben die Erfinder überraschenderweise festgestellt, dass das erfindungsgemäß verwendete 10H-Benzo[g]pteridin-2,4-dion-Derivat oder pharmakologisch verträgliche Salze und/oder Ester und/oder Komplexe davon vorzugsweise eine hohe Affinität zu Mikroorganismen aufweist.

Auf Grund der Affinität kann das erfindungsgemäß verwendete 10H-Benzo[g]pteridin-2,4-dion-Derivats der Formel (1) an Mikroorganismen effektiv binden und lokal ausreichend Singulett-Sauerstoff erzeugen, um die Mikroorganismen zu inaktivieren, vorzugsweise abzutöten.

Bei dieser bevorzugten Verwendung als Photosensibilisator wird wenigstens ein erfindungsgemäß verwendetes 10H-Benzo[g]pteridin-2,4-dion-Derivat von Mikroorganismen gebunden. Nach Bestrahlung mit elektromagnetischer Strahlung geeigneter Wellenlänge und Energiedichte werden die Mikroorganismen durch die entstandenen reaktiven Sauerstoffspezies (ROS), vorzugsweise Sauerstoffradikale und/oder Singulett-Sauerstoff, inaktiviert, vorzugsweise abgetötet.

Vorzugsweise erlaubt die Bindung wenigstens eines erfindungsgemäß verwendeten 10H-Benzo[g]pteridin-2,4-dion-Derivates oder eines pharmakologisch verträglichen Salzes und/oder Esters und/oder Komplexes davon an Mikroorganismen ebenfalls eine Anfärbung oder Lokalisierung von Mikroorganismen. Dadurch kann vorzugsweise auch der Verlauf der Inaktivierung von Mikroorganismen oder der Dekolonisation verfolgt werden.

Erfindungsgemäß wird unter dem Begriff "Dekolonisation" das Entfernen, vorzugsweise vollständige Entfernen, von Mikroorganismen verstanden.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon bei der Inaktivierung von einzelligen oder mehrzelligen Mikroorganismen, die vorzugsweise aus der Gruppe, die aus Viren, Archäeen, Bakterien, Bakteriensporen, Pilzen, beispielsweise Myzelpilze und Hefen, Pilzsporen, Protozoen, Algen und blutübertragbaren Parasiten besteht, ausgewählt werden, verwendet.

Vorzugsweise können Körperoberflächen, beispielsweise Haut oder Schleimhaut, von Menschen und Tieren, vorzugsweise Säugetieren, behandelt werden. Bei dieser bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon, vorzugsweise in einer pharmazeutischen Zubereitung, bei der Desinfektion und/oder Dekolonisierung von Haut- oder Weichteiloberflächen verwendet, wobei vorzugsweise die Hautintegrität erhalten bleibt.

Bei einer weiteren bevorzugten Ausführungsform ist wenigstens ein erfindungsgemäß verwendetes 10H-Benzo[g]pteridin-2,4-dion-Derivat oder ein pharmakologisch verträgliches Salz und/oder Ester und/oder Komplex davon in einer pharmazeutischen Zubereitung zur topischen, vorzugsweise nasalen, oralen, analen, vaginalen oder dermalen, Applikation vorhanden.

Unter topischer Applikation wird auch die Anwendung am oder im Ohr, vorzugsweise Außenohr, verstanden. Das Außenohr umfasst den Ohrknorpel, die Ohrmuschel, das Ohrläppchen und den äußeren Gehörgang oder auch Ohrkanal und die Außenseite des Trommelfells.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Herstellung einer pharmazeutischen Zubereitung bei der Prophylaxe und/oder Behandlung einer infektiösen, vorzugsweise viralen, bakteriellen und/oder mykotischen, Hautkrankheit, die vorzugsweise ausgewählt wird aus der Gruppe, die aus Staphylococcal scalded skin syndrome, Impetigo, Hautabszess, Furunkel, Karbunkel, Phlegmone, Cellulitis, Akute Lymphadenitis, Pilonidalzyste, Pyodermie, Dermatitis purulenta, Dermatitis septica, Dermatitis suppurativa, Erythrasma, Erysipelas, Akne vulgaris oder Pilzinfektion besteht, verwendet.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Herstellung einer pharmazeutischen Zubereitung bei der Wundheilung, beispielsweise bei Heilungsstörungen nach chirurgischen Interventionen, verwendet.

Vorzugsweise wird wenigstens ein erfindungsgemäß verwendetes 10H-Benzo[g]pteridin-2,4-dion-Derivat oder ein pharmakologisch verträgliches Salz und/oder Ester und/oder Komplex davon bzw. eine pharmazeutische Zubereitung enthaltend wenigstens ein erfindungsgemäßes 10H-Benzo[g]pteridin-2,4-dion-Derivat oder ein pharmakologisch verträgliches Salz und/oder Ester und/oder Komplex davon bei der Desinfektion und/oder Reduktion der Keimzahl in infizierten Wunden verwendet.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Herstellung einer pharmazeutischen Zubereitung bei der Prophylaxe und/oder Behandlung von infektiösen, vorzugsweise viralen, bakteriellen und/oder mykotischen, Erkrankungen des Ohrs, der oberen Luftwege, der Mundhöhle, des Rachens, des Kehlkopfes, der unteren Luftwege und/oder der Speiseröhre verwendet.

Das Vorherrschen pathogener Mikroorganismen ist beispielsweise die Hauptursache für Infektionen in der Mundhöhle. Dabei tritt das Problem auf, dass die Mikroorganismen in äußerst komplex aufgebauten Biofilmen synergetisch organisiert sind. Diese Biofilme, beispielsweise Plaque oder Zahnbelag, bestehen aus mehreren, komplex aufgebauten Schichten und enthalten Eiweiße, Kohlenhydrate, Phosphate und Mikroorganismen. Zahnbelag entsteht besonders dort, wo Zahnflächen nicht durch natürliche oder künstliche Reinigung belagfrei gehalten werden können. Dieser Umstand macht es schwierig, einen Zugang zu den im Biofilm eingebundenen Mikroorganismen zu finden.

Konventionelle Therapien, wie beispielsweise Antibiotika und Spüllösungen oder mechanische Zahnreinigung, können nur begrenzt eingesetzt werden, da sie entweder die Bakterien nicht direkt beeinflussen, beispielsweise bei der Zahnreinigung, nur schwer dosiert und appliziert werden können, beispielsweise bei Antibiotika und Spüllösungen, oder eine generelle Anwendung aufgrund von negativen Begleiterscheinungen nicht zu rechtfertigen ist.

Bei einer bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon als Photosensibilisator bei der photodynamischen Inaktivierung von Mikroorganismen in der Mundhöhle verwendet.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Herstellung einer pharmazeutischen Zubereitung bei der Behandlung und/oder Prophylaxe einer infektiösen, vorzugsweise viralen, bakteriellen und/oder mykotischen, Erkrankung des Zahngewebes, vorzugsweise Plaque, Karies oder Pulpitis, und/oder infektiösen, vorzugsweise viralen, bakteriellen und/oder mykotischen, Erkrankung des Zahnhalteapparates, vorzugsweise Gingivitis, Paradontitis, Endodontitis oder Periimplantitis, verwendet.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon bzw. eine pharmazeutische Zubereitung enthaltend wenigstens ein erfindungsgemäß verwendetes 10H-Benzo[g]pteridin-2,4-dion-Derivat oder ein pharmakologisch verträgliches Salz und/oder Ester und/oder Komplex davon bei der Reinigung von Zähnen, Zahnersatz und/oder Zahnspangen verwendet.

Bei einer weiteren bevorzugten Ausführungsform wird das erfindungsgemäß verwendete 10H-Benzo[g]pteridin-2,4-dion-Derivat oder ein pharmakologisch verträgliches Salz und/oder Ester und/oder Komplex davon bzw. eine pharmazeutische Zubereitung enthaltend wenigstens ein erfindungsgemäßes 10H-Benzo[g]pteridin-2,4-dion-Derivat oder ein pharmakologisch verträgliches Salz und/oder Ester und/oder Komplex davon bei der nasalen Dekolonisierung von Mikroorganismen verwendet.

Beispielsweise besitzen Methicillin-resistente Staphylococcus aureus (MRSA)-Stämme eine monatelange Persistenz bei nasaler Kolonisierung sowie eine hohe Umweltresistenz. Daher reduziert eine nasale Dekolonisierung, d.h. Entfernung der Mikroorganismen, in der Regel auch die Kolonisation an anderen Körperstellen.

Des Weiteren betrifft die vorliegende Erfindung eine pharmazeutische Zusammensetzung enthaltend wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon, vorzugsweise wenigstens ein erfindungsgemäßes 10H-Benzo[g]pteridin-2,4-dion-Derivat der Formel (1) oder ein pharmakologisch verträgliches Salz und/oder Ester und/oder Komplex davon,.

Vorzugsweise wird die pharmazeutische Zusammensetzung durch Mischen von mindestens einer Verbindung der Formel (1) oder ein pharmakologisch verträgliches Salz und/oder Ester und/oder Komplex davon mit einem oder mehreren physiologisch annehmbaren Hilfsstoff(en) hergestellt und in eine geeignete Darreichungsform gebracht.

Eine geeignete Darreichungsformen der erfindungsgemäßen pharmazeutischen Zusammensetzung wird vorzugsweise aus der Gruppe, die aus Salbe, Creme, Gel, Lotion, Schüttelmixtur, Lösung, beispielsweise in Tropfen- oder Sprayform, Puder, Mikrokapsel und Paste besteht, ausgewählt.

Die erfindungsgemäße pharmazeutische Zusammensetzung kann topisch, vorzugsweise nasal, oral, anal, vaginal oder dermal, angewendet werden.

Als physiologisch annehmbare Hilfsstoffe kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Bei einer weiteren bevorzugten Ausführungsform enthält die pharmazeutische Zusammensetzung eine effektive Menge wenigstens eines erfindungsgemäß verwendeten 10H-Benzo[g]pteridin-2,4-dion-Derivates oder eines pharmakologisch verträglichen Salzes und/oder Esters und/oder Komplexes davon, wobei die effektive Menge von 0,01 µg bis 1000 µg pro Gramm der Zusammensetzung, vorzugsweise von 0,1 µg bis 500 µg pro Gramm der Zusammensetzung, umfasst.

Bei einer bevorzugten Ausführungsform der Erfindung umfasst die pharmazeutische Zusammensetzung wenigstens ein erfindungsgemäß verwendetes 10H-Benzo[g]pteridin-2,4-dion-Derivat oder ein pharmakologisch verträgliches Salz und/oder Ester und/oder Komplex davon und wenigstens einen weiteren pharmazeutisch aktiven Bestandteil.

Vorzugsweise wird der wenigstens eine weitere pharmazeutisch aktive Bestandteil aus der Gruppe, die aus Antibiotika, Antimikotika, Virustatika, Antihistaminika, Sympathomimetika, Antihämorrhagika, Emmolientia und Hautschutzmittel, Analgetika, Desinfektionsmittel, Immunsera und Immunglobuline, Antiparasitäre Substanzen, Insektizide, Repellenzien und Corticosteroide besteht, ausgewählt.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon bzw. eine pharmazeutische Zubereitung enthaltend wenigstens ein erfindungsgemäß verwendetes 10H-Benzo[g]pteridin-2,4-dion-Derivat oder ein pharmakologisch verträgliches Salz und/oder Ester und/oder Komplex davon durch den Anwender selbst aufgebracht und, optional, nachfolgend mit einer geeigneten Strahlenquelle, die elektromagnetische Strahlung geeigneter Wellenlänge und Energiedichte erzeugt, bestrahlt.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon bzw. eine Zubereitung enthaltend wenigstens ein erfindungsgemäß verwendetes 10H-Benzo[g]pteridin-2,4-dion-Derivat oder ein pharmakologisch verträgliches Salz und/oder Ester und/oder Komplex davon bei der Inaktivierung von Mikroorganismen in medizinischen Blutprodukten verwendet.

Bei einer bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Inaktivierung von Mikroorganismen auf Oberflächen aller Art verwendet. Weiter bevorzugt wird das erfindungsgemäß verwendete 10H-Benzo[g]pteridin-2,4-dion-Derivat oder ein pharmakologisch verträgliches Salz und/oder Ester und/oder Komplex davon bei der Oberflächenreinigung und/oder -beschichtung, vorzugsweise von Medizinprodukten, Lebensmittelverpackungen oder Hygieneartikeln, verwendet.

Weiter bevorzugt wird wenigstens ein erfindungsgemäß verwendetes 10H-Benzo[g]pteridin-2,4-dion-Derivat oder ein pharmakologisch verträgliches Salz und/oder Ester und/oder Komplex davon auf Oberflächen aufgebracht und/oder eingebracht und, optional, nachfolgend mit einer geeigneten Strahlenquelle, die elektromagnetische Strahlung geeigneter Wellenlänge und Energiedichte erzeugt, bestrahlt. Vorzugsweise bewirkt das wenigstens eine erfindungsgemäß verwendete 10H-Benzo[g]pteridin-2,4-dion-Derivat oder ein pharmakologisch verträgliches Salz und/oder Ester und/oder Komplex davon während der Bestrahlung eine "Selbstdesinfektion" der Oberfläche.

Die Bestrahlung kann dabei direkt nach der Behandlung der Oberfläche mit wenigstens einem erfindungsgemäß verwendeten 10H-Benzo[g]pteridin-2,4-dion-Derivat oder ein pharmakologisch verträgliches Salz und/oder Ester und/oder Komplex davon, vorzugsweise nach dem Aufbringen des wenigstens einen erfindungsgemäß verwendeten 10H-Benzo[g]pteridin-2,4-dion-Derivates oder eines pharmakologisch verträglichen Salzes und/oder Esters und/oder Komplexes davon auf die Oberfläche und/oder Einbringen des wenigstens einen erfindungsgemäß verwendeten 10H-Benzo[g]pteridin-2,4-dion-Derivates oder eines pharmakologisch verträglichen Salzes und/oder Esters und/oder Komplexes davon in die Oberfläche, erfolgen und/oder zu einem späteren Zeitpunkt.

Weiter bevorzugt werden Gegenstände behandelt, die eine thermisch begrenzte Haltbarkeit aufweisen, beispielsweise Gegenstände aus thermoplastische Kunststoffen, oder von Desinfektionsmitteln angegriffen werden.

Gegenstände, die eine thermisch begrenzte Haltbarkeit aufweisen, können beispielsweise nur unzureichend sterilisiert werden, da sie bei höheren Temperaturen ihre Form verlieren oder spröde werden.

Darüber hinaus kann es bei einer unsachgemäßen und/oder übermäßigen Anwendung von Desinfektionsmitteln zu Resistenzbildung durch Selektion robuster Mikroorganismen kommen, wenn beispielsweise die Wirkstoffkonzentration und Einwirkzeit und damit die keimreduzierende Wirkung zu gering ist.

Bei einer weiter bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Inaktivierung von Mikroorganismen auf Oberflächen von Medizinprodukten, vorzugsweise invasiven medizinischen Hilfsmitteln wie etwa Kathetern, Hohlsonden, Schläuchen oder Nadeln, verwendet.

Vorzugsweise werden die Medizinprodukte aus Wundauflagen, Verbände, Kathetern, Hohlsonden, Schläuchen oder Nadeln ausgewählt.

Weiter bevorzugt werden unter Medizinprodukten auch zahnärztliche Abdrücke oder Zahnersatz, beispielsweise Prothesen, Kronen oder Implantate, verstanden.

Vorzugsweise wird durch eine Behandlung der Oberfläche von Medizinprodukten mit wenigstens einem erfindungsgemäß verwendeten 10H-Benzo[g]pteridin-2,4-dion- oder ein pharmakologisch verträgliches Salz und/oder Ester und/oder Komplex davon und/oder Beschichtung und/oder Immobilisierung wenigstens eines erfindungsgemäß verwendeten 10H-Benzo[g]pteridin-2,4-dion-Derivates oder eines pharmakologisch verträglichen Salzes und/oder Esters und/oder Komplexes davon auf der Oberfläche von Medizinprodukten und nachfolgender Bestrahlung mit elektromagnetische Strahlung geeigneter Wellenlänge und Energiedichte die Besiedelung von Mikroorganismen auf der behandelten Oberflächen reduziert, vorzugsweise verhindert.

Die Bestrahlung kann dabei direkt nach der Behandlung der Oberfläche mit wenigstens einem erfindungsgemäß verwendeten 10H-Benzo[g]pteridin-2,4-dion-Derivat oder eines pharmakologisch verträglichen Salzes und/oder Esters und/oder Komplexes davon, vorzugsweise nach dem Aufbringen des wenigstens einen erfindungsgemäß verwendete n 10H-Benzo[g]pteridin-2,4-dion-Derivates oder eines pharmakologisch verträglichen Salzes und/oder Esters und/oder Komplexes davon auf die Oberfläche und/oder Einbringen des wenigstens einen erfindungsgemäß verwendeten 10H-Benzo[g]pteridin-2,4-dion-Derivates oder eines pharmakologisch verträglichen Salzes und/oder Esters und/oder Komplexes davon in die Oberfläche, erfolgen und/oder zu einem späteren Zeitpunkt, vor oder während der Verwendung des behandelten Medizinproduktes.

Bei einer weiter bevorzugten Verwendung des wenigstens einen erfindungsgemäß verwendeten 10H-Benzo[g]pteridin-2,4-dion-Derivates oder eines pharmakologisch verträgliches Salzes und/oder Esters und/oder Komplexes davon in Wundauflagen und/oder Verbänden, beispielsweise Baumwollgaze, kann während oder nach dem Aufbringen einer Wundauflage und/oder Verbandes, die bzw. der wenigstens ein erfindungsgemäß verwendetes 10H-Benzo[g]pteridin-2,4-dion-Derivat oder ein pharmakologisch verträgliches Salz und/oder Ester und/oder Komplex davon enthält, eine Bestrahlung mit elektromagnetische Strahlung geeigneter Wellenlänge und Energiedichte erfolgen wodurch es nachfolgend zu einer Reduzierung, vorzugsweise Inaktivierung, von Mikroorganismen im Wundbereich oder behandelten Hautpartien kommen.

Bei einer weiteren bevorzugten Ausführungsform umfasst die Wundauflage und/oder Verband neben wenigstens einem erfindungsgemäß verwendeten 10H-Benzo[g]pteridin-2,4-dion-Derivat oder eines pharmakologisch verträgliches Salzes und/oder Esters und/oder Komplexes davon weitere Bestandteile, vorzugsweise Absorbtionsmittel, beispielsweise Calciumalginat oder Polyurethanschaum, oder weitere pharmazeutisch wirksame Substanzen.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Inaktivierung von Mikroorganismen auf Oberflächen von Lebensmittelverpackungen verwendet.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon zur Inaktivierung von Mikroorganismen in einer Flüssigkeit oder flüssigen, vorzugsweise wässrigen, Zubereitung, beispielsweise Dispersionsfarbe, verwendet.

Vorzugsweise handelt es sich bei der Flüssigkeit um Wasser.

Dabei kann wenigstens ein erfindungsgemäß verwendetees 10H-Benzo[g]pteridin-2,4-dion-Derivat oder ein pharmakologisch verträgliches Salz und/oder Ester und/oder Komplex davon zur Aufbereitung von Wasser für die Getränke- und Lebensmittelindustrie, die Pharma-, Chemie- und Kosmetikindustrie, die Elektroindustrie verwendet werden. Ferner kann wenigstens ein erfindungsgemäß verwendetes 10H-Benzo[g]pteridin-2,4-dion-Derivat oder ein pharmakologisch verträgliches Salz und/oder Ester und/oder Komplex davon bei der Trinkwasser- und Regenwasseraufbereitung, der Behandlung von Abwasser oder bei der Aufbereitung von Wasser für den Einsatz in der Klimatechnik eingesetzt werden.

Bei dieser bevorzugten Verwendung wenigstens eines erfindungsgemäß verwendeten 10H-Benzo[g]pteridin-2,4-dion-Derivates oder eines pharmakologisch verträglichen Salzes und/oder Esters und/oder Komplexes davon kann die Flüssigkeit oder flüssige Zubereitung nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäß verwendete 10H-Benzo[g]pteridin-2,4-dion-Derivat oder ein pharmakologisch verträgliches Salz und/oder Ester und/oder Komplex davon während der Bestrahlung eine "Selbst-desinfektion" der Flüssigkeit oder der flüssigen Zubereitung.

Bei einer weiteren bevorzugten Verwendung des wenigstens einen erfindungsgemäß verwendeten 10H-Benzo[g]pteridin-2,4-dion-Derivates oder eines pharmakologisch verträglichen Salzes und/oder Estere und/oder Komplexes davon kann das 10H-Benzo[g]pteridin-2,4-dion-Derivat an einen festen Träger gebunden vorliegen und so als Teil einer festen Matrix verwendet werden. Besonders bevorzugt wird wenigstens ein an einen festen Träger gebundenes erfindungsgemäß verwendetes 10H-Benzo[g]pteridin-2,4-dion-Derivat oder ein pharmakologisch verträgliches Salz und/oder Ester und/oder Komplex davon in die zu behandelnde Flüssigkeit, vorzugsweise Wasser oder Blut, eingebracht.

Besonders bevorzugt ist als Träger ein Polymer, welches wenigstens ein erfindungsgemäß verwendetes 10H-Benzo[g]pteridin-2,4-dion-Derivat oder ein pharmakologisch verträgliches Salz und/oder Ester und/oder Komplex davon daran in kovalent gebundener Weise trägt. Diese Zusammensetzung, umfassend den Träger und wenigstens ein erfindungsgemäß verwendetes 10H-Benzo[g]pteridin-2,4-dion-Derivat oder ein pharmakologisch verträgliches Salz und/oder Ester und/oder Komplex davon, entwickelt eine antimikrobielle Aktivität sobald sie elektromagnetischer Strahlung geeigneter Wellenlänge und Energiedichte ausgesetzt wird.

Des Weiteren betrifft die vorliegende Erfindung einen beschichteten Gegenstand, der wenigstens ein erfindungsgemäß verwendetes 10H-Benzo[g]pteridin-2,4-dion-Derivat oder ein pharmakologisch verträgliches Salz und/oder Ester und/oder Komplex davon enthält und/oder damit beschichtet ist.

Vorzugsweise weist die Oberfläche des beschichteten Gegenstand wenigstens ein erfindungsgemäß verwendetes 10H-Benzo[g]pteridin-2,4-dion-Derivat oder ein pharmakologisch verträgliches Salz und/oder Ester und/oder Komplex davon auf.

Der beschichtete Gegenstand kann nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäß verwendete 10H-Benzo[g]pteridin-2,4-dion-Derivat oder ein pharmakologisch verträgliches Salz und/oder Ester und/oder Komplex davon während der Bestrahlung eine "Selbst-desinfektion" der Oberfläche des beschichteten Gegenstandes.

Die Bestrahlung kann dabei direkt nach der Behandlung des beschichteten Gegenstandes mit wenigstens einem erfindungsgemäß verwendeten 10H-Benzo[g]pteridin-2,4-dion-Derivat oder eines pharmakologisch verträglichen Salzes und/oder Esters und/oder Komplexes davon, vorzugsweise nach dem Aufbringen des wenigstens einen erfindungsgemäß verwendetenen 10H-Benzo[g]pteridin-2,4-dion-Derivates auf die Oberfläche des beschichteten Gegenstandes und/oder Einbringen des wenigstens einen erfindungsgemäß verwendeten 10H-Benzo[g]pteridin-2,4-dion-Derivates in die Oberfläche des beschichteten Gegenstandes, erfolgen und/oder zu einem späteren Zeitpunkt, vorzugsweise vor oder während der Verwendung des beschichteten Gegenstandes.

Geeignete Gegenstände werden vorzugsweise aus der Gruppe, die aus Medizinprodukten, Lebensmittelverpackungen oder Hygieneartikeln, besteht, ausgewählt.

Bei einer weiteren bevorzugten Ausführungsform des beschichteten Gegenstandes handelt es sich um mit wenigstens einem erfindungsgemäß verwendeten 10H-Benzo[g]pteridin-2,4-dion-Derivat oder eines pharmakologisch verträglichen Salzes und/oder Esters und/oder Komplexes davon beschichtete Partikel, beispielsweise anorganische oder organische Partikel.

Weiter bevorzugt umfassen die Partikel wenigstens ein erfindungsgemäß verwendetes 10H-Benzo[g]pteridin-2,4-dion-Derivat oder ein pharmakologisch verträgliches Salz und/oder Ester und/oder Komplex davon, das an die Partikel kovalent gebunden vorliegt.

Die Erfindung wird nachfolgend durch Figuren und Beispiele erläutert, ohne hierauf beschränkt zu sein.

### Beispiel 1) Herstellung verschiedener 10H-Benzo[g]pteridin-2,4-dion-Derivate. Überblick über die Synthesen:

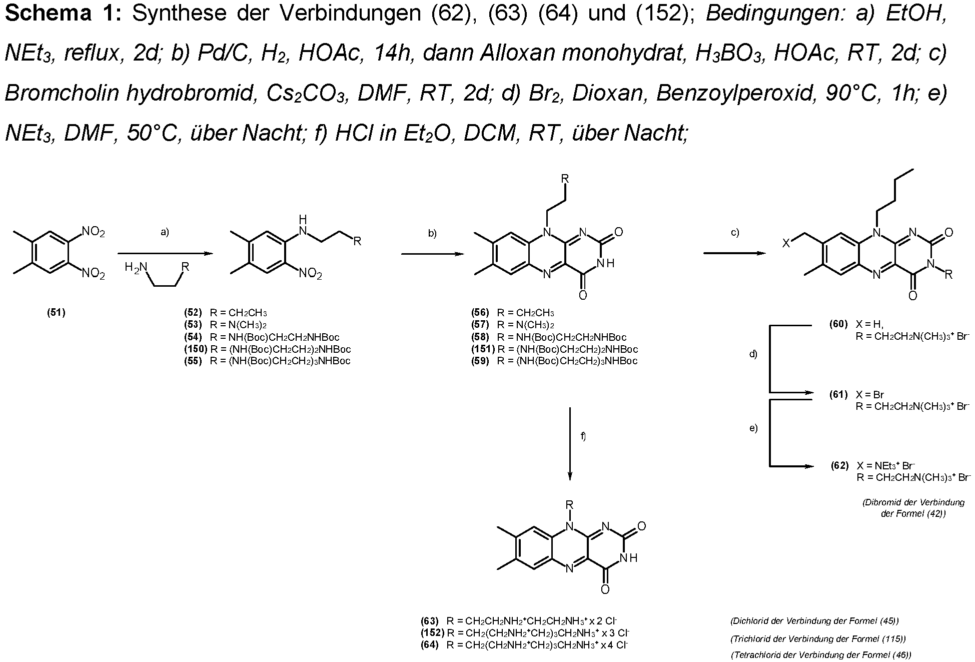

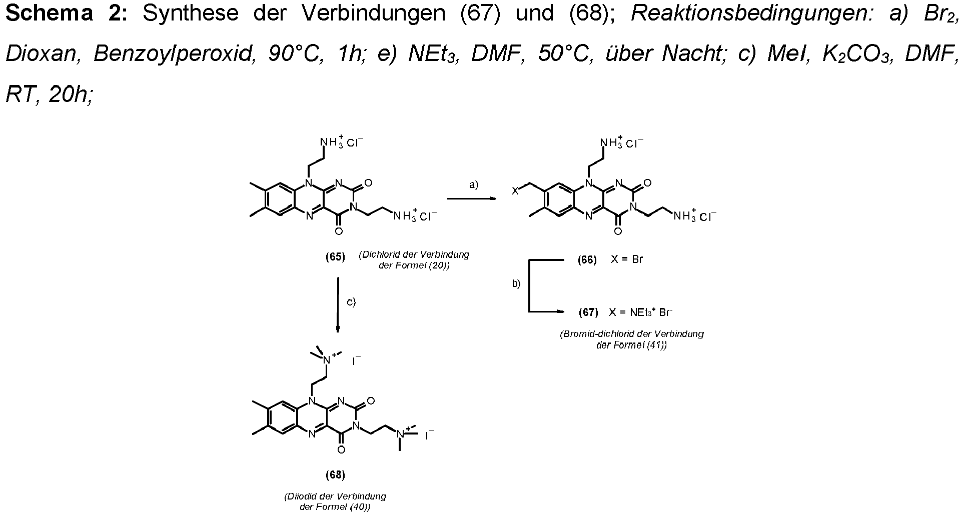

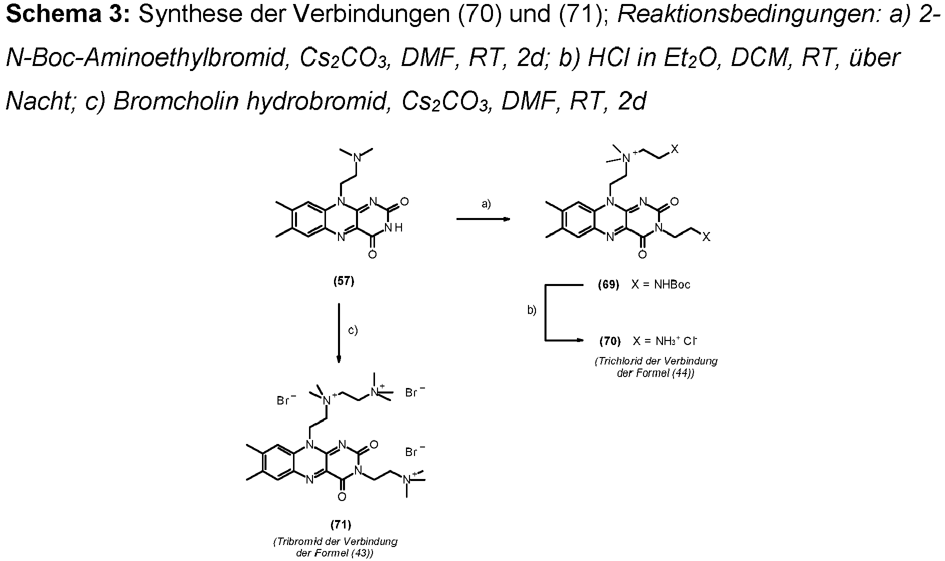

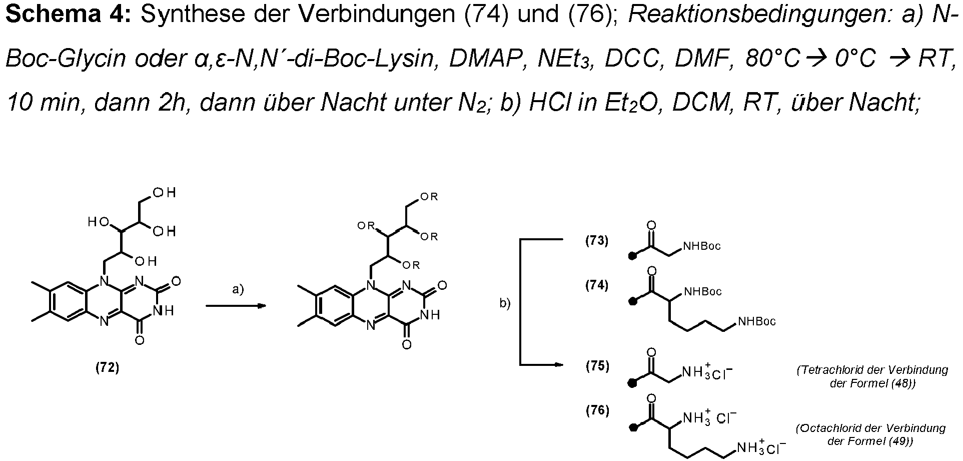

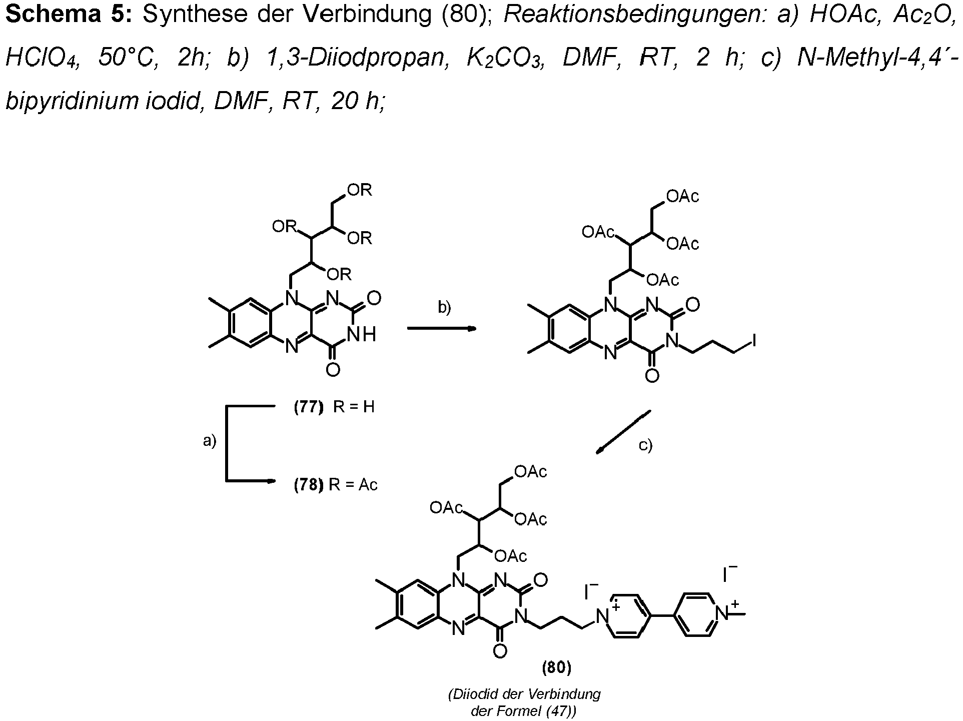

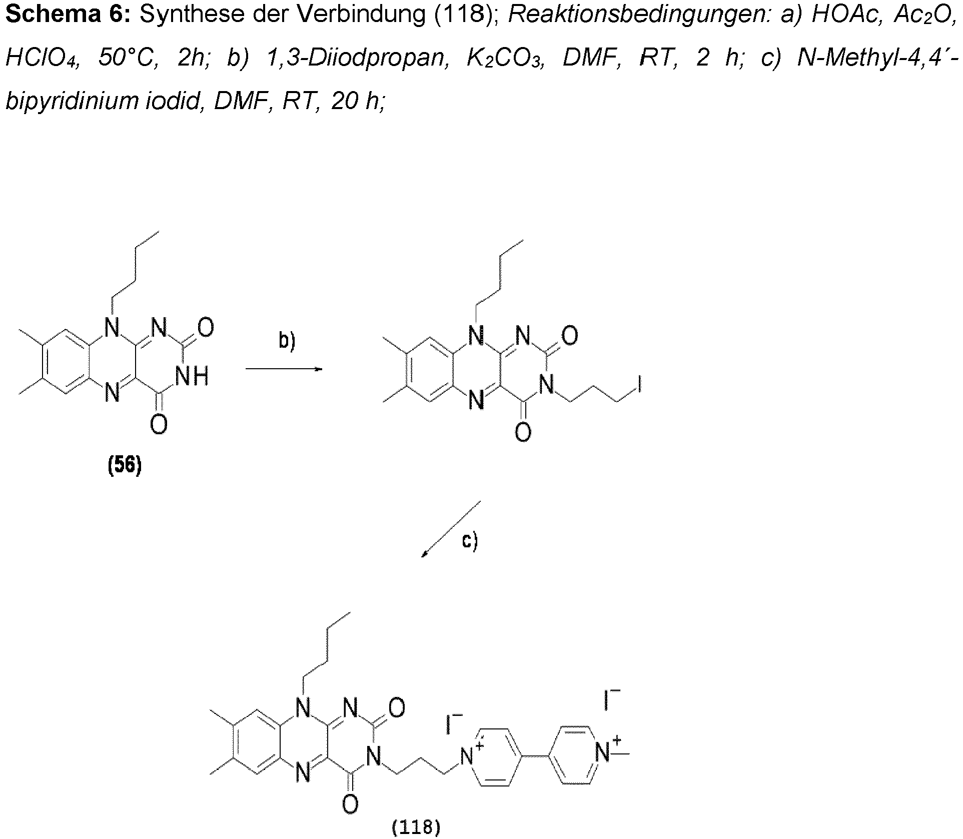

Alle verwendeten Chemikalien wurden von gängigen Anbietern käuflich erworben (TCI, ABCR, Acros, Merck und Fluka) und ohne weitere Reinigung eingesetzt. Lösemittel wurden vor Gebrauch destilliert und bei Bedarf auf übliche Art und Weise getrocknet. Trockenes DMF wurde bei Fluka (Taufkirchen, DE) käuflich erworben.

Dünnschichtchromatographien wurden auf Dünnschicht-Aluminiumfolien, beschichtet mit Kieselgel 60 F254, der Firma Merck (Darmstadt, DE) durchgeführt. Präparative Dünnschichtchromatographien wurden auf kommerziell erhältlichen, mit Kieselgel 60 beschichteten Glasplatten (20cm x 20 cm, Carl Roth GmbH & Co. KG, Karlsruhe, DE)) durchgeführt. Die Verbindungen wurden durch UV-Licht (λ = 254 nm, 333 nm) und teilweise mit bloßem Auge detektiert oder mit Ninhydrin angefärbt. Chromatographien wurden mit Kieselgel (0.060 - 0.200) der Firma Acros (Waltham, US) durchgeführt.

NMR-Spektren wurden auf einem Bruker-Spektrometer Avance 300 (300 MHz [¹H-NMR], 75 MHz [¹³C-NMR]) (Bruker Corporation, Billerica, US) gemessen.

Alle chemischen Verschiebungen sind in δ [ppm] relativ zum externen Standard (Tetramethylsilan, TMS) angegeben. Die Kopplungskonstanten sind jeweils in Hz angegeben; Charakterisierung der Signale: s = Singulett, d = Dublett, t = Triplett, m = Multiplett, dd = Dublett vom Dublett, br = breit. Die Integration bestimmt die relative Anzahl an Atomen. Die eindeutige Bestimmung der Signale in den Kohlenstoffspektren erfolgte mittels der DEPT-Methode (Pulswinkel: 135°). Fehlergrenzen: 0.01 ppm für ¹H-NMR, 0.1 ppm für ¹³C-NMR und 0.1 Hz für Kopplungskonstanten. Das verwendete Lösemittel ist jeweils für jedes Spektrum aufgeführt.

Die IR-Spektren wurden an einem Biorad Spektrometer Excalibur FTS 3000 (Bio-Rad Laboratories GmbH, München, DE) aufgenommen.

ES-MS wurden mit einem ThermoQuest Finnigan TSQ 7000 Spektrometer gemessen, sämtliche HR-MS auf einem ThermoQuest Finnigan MAT 95 (jeweils Thermo Fisher Scientific Inc, Waltham, US) Spektrometer ermittelt, Argon diente als Ionisierungsgas für FAB.

Schmelzpunkte wurden mit Hilfe des Schmelzpunktmessgerätes Büchi SMP-20 (Büchi Labortechnik GmbH, Essen, DE) unter Verwendung einer Glaskapillare bestimmt.

Sämtliche UV/Vis-Spektren wurden mit einem Varian Cary 50 Bio UV/VIS Spektrometer, Fluoreszenzspektren mit einem Varian Cary Eclipse Spektrometer (jeweils Agilent Technologies, Santa Clara, US) aufgenommen.

Die Lösungsmittel für Absorptions- und Emissionsmessungen wurden in spezieller spektroskopischer Reinheit von Acros oder Baker bzw. Uvasol von Merck gekauft. Milliporewasser (18 MΩ, Milli Q_{Plus}) wurde für alle Messungen verwendet.

2-N-tert-Butyloxycarbonyl-aminoethylbromid^{[i]} 1,2-Dinitro-4,5-dimethyl-benzen (**51**)^{[ii]}, Butyl-(4,5-dimethyl-2-nitro-phenyl)-amin (**52**) und 10-Butyl-7,8-dimethyl-10H-benzo[g]pteridin-2,4-dion (**56**)^{[iii]}, N-[2-({[(tert-Butyl)oxy]carbonyl}amino)ethyl]-4,5-dimethyl-2-nitro-anilin, Riboflavin tetraacetate^{[iv]}, N¹, N²-bis(tert-butoxycarbonyl)-diethylenetriamin^{[v]} und N¹, N², N³, N⁴-tetra(tert-butoxycarbonyl)-tetraethylenepentamine^{[vi]}, N-Methyl-4,4'-bipyridinium iodid^{[vii]}, Essigsäure-2,3,4-triacetoxy-1-[3-(3-iodo-propyl)-7,8-dimethyl-2,4-dioxo-3,4-dihydro-2H-benzo[g]pteridin-10-ylmethyl]-butyl ester (**79**)^{[viii]}, sowie 3,10-Bis[2'-(tert-butyloxycarbonylamino)eth-1'-yl]-7,8-dimethylbenzo[g]-pteridine-2,4-dion und 3,10-Bis(2'-aminoeth-1'-yl)-7,8-dimethylbenzo[g]pteridine-2,4-dion hydrochlorid (**65**)^{[ix]} wurden nach literaturbekannten Vorschriften hergestellt:
[i] N. Sakai, D. Gerard, S. Matile, J. Am. Chem. Soc. 2001, 123, 2517 - 2524.
[ii] a) A. Monge, J. A. Palop, A. López de Ceráin, V. Senador, F. J. Martinez-Crespo, Y. Sainz, S. Narro, E. Garcia, C. de Miguel, M. González, E. Hamilton, A. J. Barker, E. D. Clarke, D. T. Greenhow, J. Med. Chem. 1995, 38, 1786-1792; b) T. Sugaya, K. Nobuyuki, A. Sakaguchi, S. Tomioka, Synthesis 1995, 1257-1262; c) R. R. Holmes, R. P. Bayer, J. Am. Chem. Soc. 1960, 82, 3454-3456.
[iii] O.Wiest, Ch.B. Harrison, N.J. Saettel, R. Cibulka, M. Sax, B. König, J. Org. Chem. 2004, 69, 8183 - 8185
[iv] McCormick, D. B. J. Heterocycl. Chem. 1970, 7, 447-450.
[v] C. Moura, R.F. Vitor, L. Maria, A. Paulo, I.C. Santos, I. Santos, Dalton Trans., 2006, 5630 - 5640
[vi] S. Srinivasachari, K.M. Fichter, Th.M. Reineke, J. Am. Chem. Soc. 2008, 130, 4618 - 4627
[vii] Y. Xiao, L.Chu, Y. Sanakis, P. Liu, J. Am. Chem. Soc. 2009, 131, 9931-9933
[viii] A. Barthel, L. Trieschmann, D. Ströhl, R. Kluge, G. Böhm, Rene Csuk, Arch. Pharm. Chem. Life Sci. 2009, 342, 445 - 452
[ix] J. Svoboda, H. Schmaderer, B. König, Chem. Eur. J. 2008, 14, 1854 - 1865

Bromocholin hydrobromid wurde mit einem Reinheitsgrad >98% von TCI Deutschland GmbH (Eschborn, DE) bezogen und ohne weitere Reinigung verwendet.

### Allgemeine Vorschrift I): Umsetzung von 1,2-Dinitro-4,5-dimethyl-benzen zu substituierten 4,5-Dimethyl-2-Nitroanilinen

1,2-Dinitro-4,5-dimethyl-benzen (3,92g, 20 mmol) und das jeweils in Tabelle 1 angegebene Amin (100 mmol) wurden in trockenem Ethanol (100 mL) und frisch destilliertem Triethylamin (50 mL) 2 Tage bei 90°C Ölbadtemperatur unter Stickstoff refluxiert. Nach Abkühlen auf Raumtemperatur wurde das Lösungsmittelgemisch bei vermindertem Druck abgezogen und der Rückstand getrocknet.

**Tabelle 1: verwendete Amine**

| Beispiel | Zielverbindung | verwendetes Amin |
|---|---|---|
| - | **(52)** | H₂NCH₂CH₂CH₂CH₃ |
| Ia) | **(53)** | H₂NCH₂CH₂N(CH₃)₂ |
| Ib) | **(54)** | H₂NCH₂CH₂NH(Boc)CH₂CH₂NHBoc |
| Ic) | **(55)** | H₂NCH₂CH₂(NH(Boc)CH₂CH₂)₃NHBoc |
| Id) | **(150)** | H₂NCH₂CH₂(NH(Boc)CH₂CH₂)₂NHBoc |

### Ia) N'-(4,5-Dimethyl-2-nitro-phenyl)-N,N-dimethyl-ethane-1,2-diamine (53)

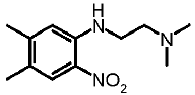

Das Rohprodukt wurde aus Ethanol umkristallisiert. Man erhält 2,99 g orange Kristallnadeln (63 % der Theorie, 126 mmol).
**¹H-NMR** (300 MHz, CDCl₃): δ [ppm] = 2,17 (s, 3 H), 2,26 (s, 3 H), 2,32 (s, 6 H), 2,71 (t, 2 H, J = 544 Hz), 3,32 (m, 2 H), 6,61 (s, 1 H), 7,91 (s, 1 H), 8,19 (bs, 1 H); - **MS** (CI-MS, NH₃): m/z (%) = 238.1 (100, (MH⁺)); - **MG** = 237,30 g/mol - **MF** = C₁₂H₁₉N₃O₂

### Ib) (2-{tert-Butoxycarbonyl-[2-(4,5-dimethyl-2-nitro-phenylamino)-ethyl]-amino}-ethyl)-carbamic acid tert-butvl ester (54)

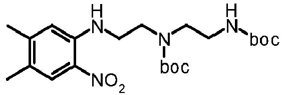

Das Rohprodukt wurde durch Säulenchromatographie an Kieselgel mit Ethylacetat / Petrolether 1:4 gereinigt wodurch ein oranger Feststoff erhalten wurde. (6,97 g, 77 % der Theorie, 15,4 mmol).
**¹H-NMR** (300 MHz, CDCl₃): δ [ppm] = 1,43 (s, 9 H), 1,45 (s, 9 H), 2,18 (s, 3 H), 2,27 (s, 3 H), 3,23 (m, 2 H), 3,32 (m, 2 H), 3,48 (m, 2 H), 3,53 (app. d, 2 H), 4,78 (bs, 1 H), 6,69 (m, 1 H), 7,90 (s, 1 H), 8,03 (m, 1 H); - **MS** (ESI-MS, H₂O/MeOH + 0.1 % TFA): m/z (%) = 453.1 (100, (MH⁺)); - **MG** = 452,56 g/mol - **MF** = C₂₂H₃₆N₄O₆

### Ic) {2-[tert-Butoxycarbonyl-(2-tert-butoxycarbonylamino-ethyl)-amino]-ethyl}-(2-{tert-butoxycarbonyl-[2-(4,5-dimethyl-2-nitro-phenylamino)-ethyl]-amino}-ethyl)-carbamic acid tert-butvl ester (55)

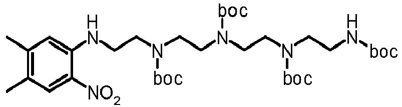

Das Rohprodukt wurde durch Säulenchromatographie an Kieselgel mit Ethylacetat / Petrolether 1:6 gereinigt und ein gelboranger Feststoff (9,01 g, 61 % der Theorie, 12,2 mmol) wurde erhalten.
**¹H-NMR** (300 MHz, CDCl₃): δ [ppm] = 1,40 (s, 9 H), 1,41 (s, 9 H), 1,43 (s, 9 H), 1,45 (s, 9 H), 2,17 (s, 3H), 2,26 (s, 3H), 3,21 - 3,39 (m, 10 H), 3,41 - 3,59 (m, 6 H), 4,72 (bs, 1 H), 4,80 (bs, 1 H), 4,96 - 5,03 (m, 2 H), 6,70 (m, 1 H), 7,89 (s, 1 H), 8,05 (m, 1 H); - **MS** (ESI-MS, H₂O/MeOH + 0.1 % TFA): m/z (%) = 739.1 (100, (MH⁺)); - **MG** = 738,93 g/mol - **MF** = C₃₂H₆₂N₆O₁₀

### Id) {2-[tert-Butoxycarbonyl-amino]-ethyl}-(2-{tert-butoxycarbonyl-[2-(4,5-dimethyl-2-nitro-phenylamino)-ethyl]-amino}-ethyl)-carbaminsäure tert-butyl ester (150)

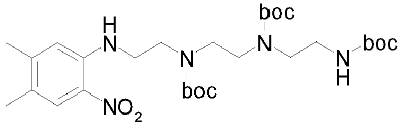

Das Rohprodukt wurde durch Säulenchromatographie an Kieselgel mit Ethylacetat / Petrolether 1:6 gereinigt und ein gelboranger Feststoff (6,58 g, 55 % der Theorie, 11,0 mmol) wurde erhalten.
**¹H-NMR** (300 MHz, CDCl₃): δ [ppm] = 1,41 (s, 9 H), 1,42 (s, 9 H), 1,43 (s, 9 H), 2,18 (s, 3H), 2,25 (s, 3H), 3,20 - 3,37 (m, 8 H), 3,42 - 3,57 (m, 4 H), 4,71 (bs, 1 H), 6,72 (m, 1 H), 7,88 (s, 1 H), 8,03 (m, 1 H); - **MS** (ESI-MS, H₂O/MeOH + 0.1 % TFA): m/z (%) = 596.1 (100, (MH⁺)); - **MG** = 595,61 g/mol - **MF** = C₂₉H₄₉N₅O₈

### Allgemeine Vorschrift II): Umsetzung von substituierten 2-Nitroanilinen zu den entsprechenden monosubstituierten 10H-benzo[g]pteridin-2,4-dion-Derivaten

Das jeweils oben unter Ia) bis Id) erhaltene 2-Nitroanilin (**53**) bis (**55**) und (**150**) (10 mmol) wurde in Essigsäure (100 mL) gelöst. Palladium auf Aktivkohle (100 mg, 10% Pd) wurde zugegeben und der Ansatz wird 14 h bei Raumtemperatur im Autoklaven bei 20 bar Wasserstoffdruck gerührt. Die farblose Lösung wurde in einen Schlenckkolben mit Stickstoffatmosphäre gefiltert, Alloxan monohydrat (4,00 g, 25 mmol) und Borsäure (15,50 g, 250 mmol) wurden zugegeben. Der Kolben wurde mit Alufolie umwickelt und der Ansatz 2 Tage bei Raumtemperatur unter Stickstoff im Dunklen gerührt. Die orangegelbe Suspension wurde mit Wasser (200 mL) verdünnt und viermal mit Dichlormethan (je 150 mL) extrahiert. Die vereinigten organischen Phasen wurden mit Wasser (100 mL) gewaschen, über Magnesiumsulfat getrocknet und einrotiert.

### IIa) 10-(2-Dimethylamino-ethyl)-7,8-dimethyl-10H-benzo[g]pteridine-2,4-dion (57)

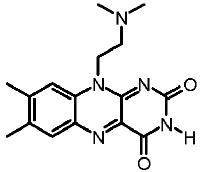

Der Rückstand wurde durch Säulenchromatographie an Kieselgel mit Ethylacetat / Methanol 20:1 → 5:2 gereinigt. Oranger Feststoff (2,22 g, 7,07 mmol, 71 % der Theorie).
**¹H-NMR** (300 MHz, DMSO-d6): δ [ppm] = 2.34 (s, 3 H), 2.43 (s, 3 H), 2,91 (s, 6 H), 3,39 (m, 2 H), 4,61 (m, 2 H), 7.52 (s, 1 H), 7.78 (s, 1 H), 11.38 (s, 1 H); - **MS** (ESI-MS, H₂O/MeOH + 0.1 % TFA): m/z (%) = 314.0 (100, (MH⁺)); - **MG** = 313,36 g/mol - **MF** = C₁₆H₁₉N₅O₂

### IIb) (2-{tert-Butoxycarbonyl-[2-(7,8-dimethyl-2,4-dioxo-3,4-dihydro-2H-benzo[g]pteridin-10-yl)-ethyl]-amino}-ethyl)-carbamic acid tert-butyl ester (58)

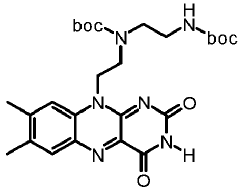

Der Rückstand wurde durch Säulenchromatographie an Kieselgel mit Dichlormethan / Methanol 95:5 gereinigt. Das Produkt ist ein oranger Feststoff (3,51 g, 6,64 mmol, 66 % der Theorie).
**¹H-NMR** (300 MHz, CDCl₃): δ [ppm] = 1,42 (s, 9 H), 1,43 (s, 9 H), 2,38 (s, 3 H), 2.51 (s, 3 H), 3,19 (m, 2 H), 3,30 (m, 2 H), 3,48 - 3,76 (m, 4 H), 5,02 (bs, 1 H), 7.78 (s, 1 H), 7.92 (s, 1 H), 9.81 (s, 1 H); - **MS** (ESI-MS, H₂O/MeOH + 0.1 % TFA): m/z (%) = 529.0 (100, (MH⁺)); - **MG** = 528,61 g/mol - **MF** = C₂₆H₃₆N₆O₆

### IIc) {2-[tert-Butoxycarbonyl-(2-tert-butoxycarbonylamino-ethyl)-amino]-ethyl}-(2-{tert-butoxycarbonyl-[2-(7,8-dimethyl-2,4-dioxo-3,4-dihydro-2H-benzo[g]pteridin-10-yl)-ethyl]-amino}-ethyl)-carbamic acid tert-butyl ester (59)

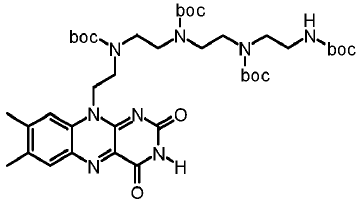

Der Rückstand wurde durch Säulenchromatographie an Kieselgel mit Dichlormethan / Methanol 95:5 gereinigt. Oranger Feststoff (4,30 g, 5,28 mmol, 53 % der Theorie).
**¹H-NMR** (300 MHz, CDCl₃): δ [ppm] = 1,36 - 1,53 (m, 36 H), 2,39 (s, 3 H), 2,50 (s, 3 H), 3,10 - 3,50 (m, 14 H), 3,55 - 3,75 (m, 2 H), 5,07 (bs, 1 H), 7.80 (s, 1 H), 7.96 (s, 1 H), 9.73 (s, 1 H); - **MS** (ESI-MS, H₂O/MeOH + 0.1 % TFA): m/z (%) = 815.1 (100, (MH⁺)); - **MG** = 814,99 g/mol - **MF** = C₄₀H₆₂N₈O₁₀

### IId) {2-(tert-Butoxycarbonyl-aminoethyl}-(2-{tert-butoxycarbonyl-[2-(7,8-dimethyl-2,4-dioxo-3,4-dihydro-2H-benzo[g]pteridin-10-yl)-ethyl]-amino}-ethyl)-carbamic acid tert-butyl ester (151)

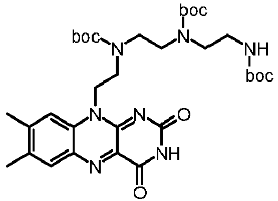

Der Rückstand wurde durch Säulenchromatographie an Kieselgel mit Dichlormethan / Methanol 95:5 gereinigt. Gelboranger Feststoff (3,28 g, 4,88 mmol, 49 % der Theorie).
**¹H-NMR** (300 MHz, CDCl₃): δ [ppm] = 1,33 - 1,51 (m, 27 H), 2,38 (s, 3 H), 2,52 (s, 3 H), 3,12 - 3,48 (m, 10 H), 3,53 - 3,76 (m, 2 H), 5,04 (bs, 1 H), 7.82 (s, 1 H), 7.95 (s, 1 H), 9.71 (s, 1 H); - **MS** (ESI-MS, H₂O/MeOH + 0.1 % TFA): m/z (%) = 815.1 (100, (MH⁺)); - **MG** = 671,8 g/mol - **MF =** C₃₃H₄₉N₇O₈

### Allgemeine Vorschrift III): Entschützung Boc-geschützter Flavinderivate

Das jeweils angegebene Flavin (2.0 mmol) wurde in Dichlormethan (100 mL) gelöst, HCl in Diethylether (10 mL) wurde zugetropft und die Reaktionsmischung über Nacht im Dunklen unter Feuchtigkeitsausschluss gerührt. Der Niederschlag wurde abgesaugt, mit Diethylether gewaschen und getrocknet.

### IIIa) (2-Amino-ethyl)-[2-(7,8-dimethyl-2,4-dioxo-3,4-dihydro-2H-benzo[g]pteridin-10-yl)-ethyl]-amin dihydrochlorid (63)

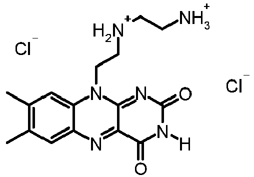

Das unter IIb) erhaltene Flavin (**58**) wurde nach der allgemeinen Vorschrift III) entschützt und ein hellbrauner Feststoff (0,79 g, 1,97 mmol, 98 % der Theorie) wurde erhalten.
**IR** (neat); v [cm⁻¹] = 3440 (m), 1714 (s), 1616 (s), 1584 (s), 1544 (s), 1459 (m), 1346 (m), 1252 (s), 1194 (m), 1024 (w), 931 (w), 876 (w), 809 (w), 772 (w); - **MS** (ESI-MS, H₂O/MeOH + 0.1 % TFA): m/z (%) = 330.1 (47, (MH⁺)), 165.0 (100, (M+2H⁺)²⁺); - **MG** = 330.39 + 2x 35.45 g/mol - **MF** = C₁₆H₂₂N₆O₂Cl₂

### Illb) 10-(2-{2-[2-(2-Amino-ethylamino)-ethylamino]-ethylamino}-ethyl)-7,8-dimethyl-10H-benzo[g]pteridine-2,4-dione tetrahydrochlorid (14)

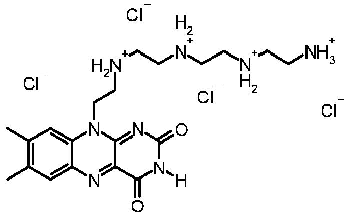

Das unter IIc) erhaltene Flavin (**59**) wurde nach der allgemeinen Vorschrift III) entschützt und ein hellbrauner Feststoff (0,97 g, 1,71 mmol, 86 % der Theorie) wurde erhalten.
**IR** (neat); v [cm⁻¹] = 3440 (m), 1713 (s), 1618 (s), 1583 (s), 1541 (s), 1458 (m), 1344 (m), 1256 (s), 1192 (m), 1023 (w), 932 (w), 878 (w), 808 (w), 771 (w); - **MS** (ESI-MS, H₂O/MeOH + 0.1 % TFA): m/z (%) = 418.1 (63, (MH⁺)), 209.0 (100, (M+2H⁺)²⁺); - **MG** = 418.55 + 4x 35.45 g/mol - **MF** = C₂₀H₃₄N₈O₂Cl₄

### IIId) 10-(2-{2-[2-ethylamino]-ethylamino}-ethyl)-7,8-dimethyl-10H-benzo[g]pteridine-2,4-dione trihydrochlorid (152)

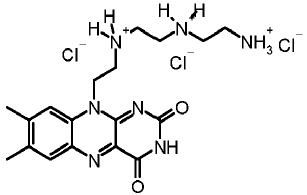

Das unter IId)) erhaltene Flavin (**151**) wurde nach der allgemeinen Vorschrift III) entschützt und ein orangebrauner Feststoff (0,81 g, 1,40 mmol, 70 % der Theorie) wurde erhalten.
**IR** (neat); v [cm⁻¹] = 3442 (m), 1711 (s), 1616 (s), 1584 (s), 1542 (s), 1458 (m), 1346 (m), 1258 (s), 1193 (m), 1024 (w), 932 (w), 877 (w), 806 (w), 772 (w); - **MS** (ESI-MS, H₂O/MeOH + 0.1 % TFA): m/z (%) = 474.1 (49, (MH⁺)), 237.0 (100, (M+2H⁺)²⁺); - **MG** = 474.47 + 3x 35.45 g/mol - **MF** = C₁₈H₂₈N₇O₂Cl₃

### IV) [2-(10-Butyl-7,8-dimethyl-2,4-dioxo-4,10-dihydro-2H-benzo[g]pteridin-3-yl)-ethyl]-trimethyl-ammonium bromid (60)

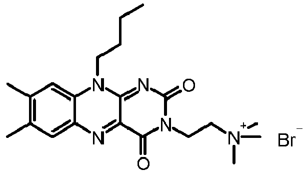

Flavin (**56**) (310 mg, 1.0 mmol) wurde in trockenem DMF (20 mL) gelöst, Caesium carbonat (1,63 g, 5 mmol) und Bromocholin hydrobromid (0.5 g, 2.0 mmol) wurden zugegeben und der Ansatz für 1d bei Raumtemperatur im Dunklen gerührt. Es wurde erneut Bromocholin hydrobromid (0.5 g, 2.0 mmol) zugegeben und der Ansatz einen weiteren Tag bei Raumtemperatur im Dunklen gerührt. Das DMF wurde abgezogen und der Rückstand in Chloroform/Methanol 6:1 (50 mL) suspendiert. Die Suspension wurde gefiltert und das Filtrat wurde bis zur Trockene eingeengt. Das Rohprodukt wurde durch präparative Dünnschichtchromatographie an Kieselgel (CHCl₃/MeOH - 4:1) und durch Umkristallisation aus Wasser gereinigt.
Ausbeute: 161 mg eines orangen Feststoffes (0,347 mmol, 35 % der Theorie)
R_{f} = 0.1 (CHCl₃:MeOH - 4:1)
**¹H-NMR** (300 MHz, DMSO-d6): δ [ppm] = 0,97 (t, 3 H, J = 7.3 Hz), 1,48 (m, 2 H), 1,70 (m, 2 H), 2,41 (s, 3 H), 2,51 (s, 3 H), 3,14 - 3,35 (m, 8 H), 3,95 (t, 2 H, J = 6 Hz), 4,56 (t, 2 H, J = 7,8 Hz), 7,77 (s, 1 H), 7,89 (s, 1 H); - **MS** (ESI-MS, H₂O/MeOH + 0.1 % TFA): m/z (%) = 384.1 (100, (M⁺)); - **MG** = 384.51 + 79.90 g/mol - **MF** = C₂₁H₃₀N₅O₂Br

### V) [10-Butyl-3-(2-trimethylammonium-ethyl)-7-methyl-2,4-dioxo-2,3,4,10-tetrahydro-benzo[g]pteridin-8-ylmethyl]-triethyl-ammonium dibromid (62)

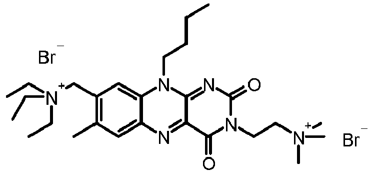

[2-(10-Butyl-7,8-dimethyl-2,4-dioxo-4,10-dihydro-2H-benzo[g]pteridin-3-yl)-ethyl]-trimethyl-ammonium bromid (**60**) (140 mg, 0.3 mmol) wurden in 15 ml Dioxan suspendiert. Die Mischung wurde auf Refluxtemperatur gebracht, Dibenzoylperoxid (10 mg in etwas Dioxan) wurde zugegeben, und 0.80 g Dioxan dibromid (3.0 mmol) in 5 mL Dioxan wurde zugetropft. Es wurde 30 min refluxiert, auf Raumtemperatur abgekühlt und das Lösungsmittel bei vermindertem Druck abgezogen.

Das rohe 8a-Bromo-Flavin (**61**) wurde in DMF (10 mL) gelöst, die Lösung wurde entgast und Triethylamin (0,60 g, 0,76 mL, 6.0 mmol) wurde über 5 min. zugetropft. Der Ansatz wurde bei 50°C über Nacht unter N₂ im Dunklen gerührt. Nach Abkühlen auf Raumtemperatur wurde die Reaktionsmischung in 200 mL eiskalten Diethylether eingetropft, das Rohprodukt wurde abgefiltert, mit Diethylether gewaschen und bei vermindertem Druck getrocknet. Der Rückstand wurde durch Chromatographie an RP-18-Gel mit MeCN/Wasser 9:1 → 4:1 gereinigt. Zur weiteren Reinigung wurde aus Wasser umkristallisiert.

Es wurde ein brauner Feststoff (31 mg, 0,048 mmol, 16 % der Theorie) erhalten.
**MS** (ESI-MS, H₂O/MeOH + 0.1 % TFA): m/z (%) = 484.1 (37, (M⁺)), 242.0 (100, (M²⁺)); - **MG** = 484.69 + 2x 79.90 g/mol - **MF** = C₂₇H₄₄N₆O₂Br₂

### VI) 3,10-Bis-(2-trimethylammonium-ethyl)-7,8-dimethyl-10H-benzo[g]pteridine-2,4-dione dibromid (68)

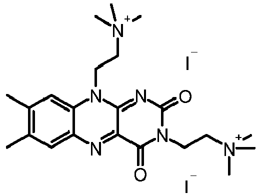

3,10-Bis(2'-aminoeth-1'-yl)-7,8-dimethylbenzo[g]pteridine-2,4-dion hydrochlorid (**65**) (400 mg, 1.0 mmol) wurde in trockenem DMF (20 mL) gelöst, Caesium carbonat (1,32 g, 4.0 mmol) und Methyliodid (1.42 g, 10.0 mmol) wurden zugegeben und der Ansatz für 20 h bei Raumtemperatur im Dunklen gerührt. Die Suspension wurde mit Chloroform (100 mL) verdünnt und mit Wasser (30 mL) gewaschen. Die organische Phase wurde über Magnesiumsulfat getrocknet und die Lösungsmittel wurden bei vermindertem Druck abgezogen. Das Rohprodukt wurde durch präparative Dünnschichtchromatographie an Kieselgel (CHCl₃/MeOH -4:1) gereinigt.
Ausbeute: 404 mg eines orangen Feststoffes (0,61 mmol, 61 % der Theorie)
**¹H-NMR** (300 MHz, DMSO-d6): δ [ppm] = 2,42 (s, 3 H), 2,54 (s, 3 H), 3.10 - 3,42 (m, 16 H), 4,15 (d, 2 H, J = 5,7 Hz), 4.93 (d, 2 H, J = 6,4 Hz), 7.96 (s, 1 H), 8.32 (s, 1 H); - **MS** (ESI-MS, H₂O/MeOH + 0.1 % TFA): m/z (%) = 414.1 (48, (M⁺)), 207.0 (100, (M²⁺)); - **MG** = 414.56 + 2x 126.90 g/mol - **MF** = C₂₂H₃₄N₆O₂I₂

### VII) 3-(2-Trimethylammonium-ethyl)-10-{2-[(2-trimethylammonium-ethyl)-dimethyl-ammonium]-ethyl)-7,8-dimethyl-10H-benzo[g]pteridine-2,4-dione tribromid (71)

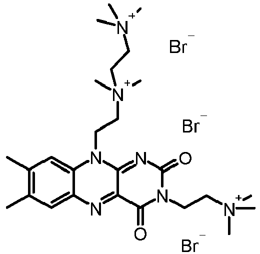

Das unter IIa) erhaltene Flavin (**57**) (300 mg, 1.0 mmol) wurde in trockenem DMF (20 mL) gelöst, Caesium carbonat (1,96 g, 6 mmol) und Bromocholin hydrobromid (1.0 g, 4.0 mmol) wurden zugegeben und der Ansatz für 1d bei Raumtemperatur im Dunklen gerührt. Es wurde erneut Bromocholin hydrobromid (0.5 g, 2.0 mmol) zugegeben und der Ansatz einen weiteren Tag bei Raumtemperatur im Dunklen gerührt. Das DMF wurde abgezogen und der Rückstand in Chloroform/Methanol 3:1 (50 mL) suspendiert. Die Suspension wurde gefiltert und das Filtrat wurde bis zur Trockene eingeengt. Der Rückstand wurde durch Chromatographie an RP-18-Gel mit MeCN/Wasser 9:1 → 4:1 gereinigt. Zur weiteren Reinigung wurde aus Wasser umkristallisiert.
Ausbeute: 213 mg eines orangen Feststoffes (0,293 mmol, 29 % der Theorie)
**MS** (ESI-MS, H₂O/MeOH + 0.1 % TFA): m/z (%) = 486.1 (11, (M⁺)), 243.0 (100, (M²⁺)), 162.0 (7, (M³⁺)); - **MG** = 486.69 + 3x 79.90 g/mol - **MF** = C₂₆H₄₄N₇O₂Br₃

### VIII) (2-tert-Butoxycarbonylamino-ethyl)-{2-[3-(2-tert-butoxycarbonylamino-ethyl)-7,8-dimethyl-2,4-dioxo-3,4-dihydro-2H-benzo[g]pteridin-10-yl]-ethyl}-dimethyl-ammonium bromid (69)

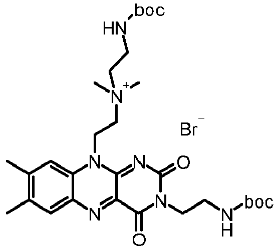

Das unter IIa) erhaltene Flavin (**57**) (300 mg, 1.0 mmol) wurde in trockenem DMF (20 mL) gelöst, Kaliumcarbonat (1.4 g, 10 mmol) und Kaliumiodid (1.1 g, 6 mmol) wurden zugegeben. Nach 20 minütigem Rühren bei Raumtemperatur wurde 2-(tert-butyloxycarbonylamino)ethyl bromid (1.15 g, 5 mmol) in DMF (5 mL) zugetropft und der Ansatz für 1d bei Raumtemperatur im Dunklen gerührt. Es wurde erneut 2-(tert-butyloxycarbonylamino)ethyl bromid (0.58 g, 2.5 mmol) zugegeben und der Ansatz einen weiteren Tag bei Raumtemperatur im Dunklen gerührt. Das DMF wurde abgezogen und der Rückstand in Dichlormethan (200 mL) gelöst. Es wurde nacheinander mit wässriger Natriumhydrogencarbonatlösung (5%, 50 mL) und Wasser (60 mL) gewaschen, über MgSO₄ getrocknet und einrotiert. Der Rückstand wurde durch präparative Dünnschichtchromatographie an Kieselgel mit Chloroform / Methanol 6:1 gereinigt.
Ausbeute: 301 mg eines orangen Feststoffes (0,442 mmol, 44 % der Theorie)
**MS** (ESI-MS, H₂O/MeOH + 0.1 % TFA): m/z (%) = 600.1 (100, (M⁺)); - **MG** = 600.74 + 79.90 g/mol - **MF** = C₃₀H₄₆N₇O₆Br

### IX) (2-Amino-ethyl)-{2-[3-(2-amino-ethyl)-7,8-dimethyl-2,4-dioxo-3,4-dihydro-2H-benzo[g]pteridin-10-yl]-ethyl}-dimethyl-ammonium chlorid dihydrochlorid (70)

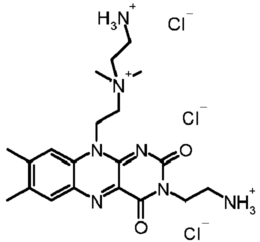

Das unter VIII) erhaltene Flavin (**69**) wurde nach der allgemeinen Vorschrift III entschützt und 0,93 g eines hellbraunen Feststoffes (1,83 mmol, 92 % der Theorie) wurden erhalten.
**MS** (ESI-MS, H₂O/MeOH + 0.1 % TFA): m/z (%) = 402.1 (16, (MH⁺)), 201.0 (100, ((M+2H⁺)²⁺)), 134.0 (4, ((M+3H⁺)³⁺)); - **MG** = 402.52 + 3x 35.45 g/mol - **MF** = C₂₀H₃₂N₇O₂Cl₃

### X) [3,10-Bis-(2-amino-ethyl)-7-methyl-2,4-dioxo-2,3,4,10-tetrahydro-benzo[g]pteridin-8-ylmethyl]-triethyl-ammonium bromid dihydrochlorid (67)

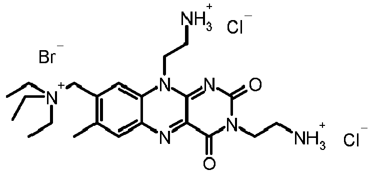

3,10-Bis(2'-aminoeth-1'-yl)-7,8-dimethylbenzo[g]pteridine-2,4-dion hydrochlorid (**65**) (400 mg, 1.0 mmol) wurden in 25 ml Dioxan suspendiert. Die Mischung wurde auf Refluxtemperatur gebracht, Dibenzoylperoxid (20 mg in etwas Dioxan) wurde zugegeben, und 0.80 g Dioxan dibromid (3.0 mmol) in 5 mL Dioxan wurde zugetropft. Es wurde 30 min refluxiert, auf Raumtemperatur abgekühlt und das Lösungsmittel bei vermindertem Druck abgezogen.

Das rohe 8a-Bromo-Flavin (**66**) wurde in DMF (10 mL) gelöst, die Lösung wurde entgast und Triethylamin (1,01 g, 1,26 mL, 10.0 mmol) wurde über 5 min. zugetropft. Der Ansatz wurde bei 50°C über Nacht unter N₂ im Dunklen gerührt. Nach Abkühlen auf Raumtemperatur wurde die Reaktionsmischung in 200 mL eiskalten Diethylether eingetropft, das Rohprodukt wurde abgefiltert, mit Diethylether gewaschen und bei vermindertem Druck getrocknet. Der Rückstand wurde durch Chromatographie an RP-18-Gel mit MeCN/Wasser 9:1 → 4:1 gereinigt. Zur weiteren Reinigung wurde aus Wasser umkristallisiert.

Es wurde ein brauner Feststoff (53 mg, 0,091 mmol, 9 % der Theorie) erhalten.
**MS** (ESI-MS, H₂O/MeOH + 0.1 % TFA): m/z (%) = 430.1 (12, (MH⁺)), 215.0 (100, ((M+2H⁺)²⁺)), 143.2 (2, ((M+3H⁺)³⁺)); - **MG** = 430.58 + 79.90 +2x 35.45 g/mol - **MF** = C₂₂H₃₆N₇O₂BrCl₂

### XI) 1-{3-[7,8-Dimethyl-2,4-dioxo-10-(2,3,4,5-tetraacetoxy-pentyl)-4,10-dihydro-2H-benzo[g]pteridin-3-yl]-propyl}-methyl-[4,4']bipyridinyl-1-ium diiodid (80)

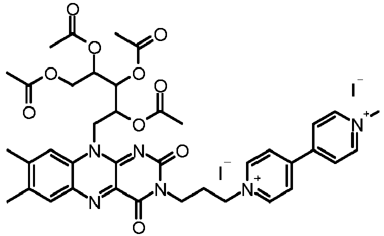

Essigsäure-2,3,4-triacetoxy-1-[3-(3-iodo-propyl)-7,8-dimethyl-2,4-dioxo-3,4-dihydro-2H-benzo[g]pteridin-10-ylmethyl]-butyl ester (**79**) (0.75 g, 1.05 mmol) wurde mit N-Methyl-4,4'-bipyridinium iodid (356 mg, 1,2 mmol) und NaHCO₃ (0.1 g, 1.2 mmol) in DMF (10 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).

Man erhält 312 mg eines hellbraunen Feststoffes (0,34 mmol, 33 % der Theorie) erhalten wurden.
**¹H-NMR** (300 MHz, MeOD): δ [ppm] = 1,71 (s, 3 H), 2,02 (s, 3 H), 2,21 (s, 3 H), 2,26 (s, 3 H,), 2,41 (s, 3 H), 2,49 (m, 2 H), 2,51 (s, 3 H), 4,26 (s, 3 H), 4,18 - 4,30 (m, 3 H), 4,50 (m, 1 H), 4,89 (m, 2 H), 5,18 (m, 2 H), 5,46 (m, 1 H), 5,53 (m, 1 H), 5,66 (m, 1 H), 7,86 (s, 1 H), 7,91 (s, 1 H), 8,21 (d, J = 5,6 Hz, 2 H), 8,98 (d, J = 5,6 Hz, 2 H), 9,24 (d, J = 5,6 Hz, 2 H), 9,59 (d, J = 5,6 Hz, 2 H); - **MS** (ESI-MS, H₂O/MeOH + 0.1 % TFA): m/z (%) = 756.1 (56, (M⁺)), 378.0 (100, (M²⁺)); - **MG** = 756.82 + 2x 126.90 - **MF** = C₃₉H₄₄N₆O₁₀I₂

### Allgemeine Vorschrift XII): Veresterung von Riboflavin mit Aminosäuren

Riboflavin (72) (1,88 g, 5 mmol) wurde unter Erwärmen auf 80°C in trockenem DMF (50 mL) gelöst. Nach Abkühlen auf Raumtemperatur wurde die jeweils angegebene geschützte Aminosäure (100 mmol), gefolgt von DMAP (1,22 g, 10 mmol), Triethylamin (1,01 g, 1,26 mL, 10 mmol) und DCC (2,06 g, 10 mmol) zugegeben. Der Ansatz wurde unter N₂ über Nacht bei 50°C im Dunklen gerührt. Das Lösungsmittel wurde unter vermindertem Druck abgezogen, der Rückstand in Chloroform (200 mL) gelöst. Es wurde mit wässriger Natriumhydrogencarbonatlösung (5%, 100 mL), Wasser (100 mL) und ges. wässriger Kochsalzlösung (100 mL) gewaschen, über MgSO₄ getrocknet und einrotiert. Das Rohprodukt wurde durch Säulenchromatographie an Kieselgel mit Dichlormethan / Methanol 20:1 gereinigt.

### Xlla) tert-Butoxycarbonylamino-acetic acid 2,3,4-tris-(2-tert-butoxycarbonylamino-acetoxy)-5-(7,8-dimethyl-2,4-dioxo-3,4-dihydro-2H-benzo[g]pteridin-10-yl)-pentyl ester (73)

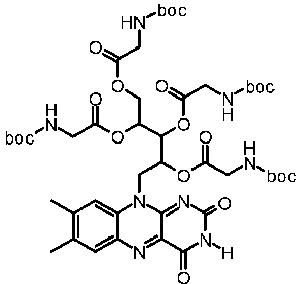

N-Boc-Glycin (3,48 g, 20 mmol) wurde mit Riboflavin (**72**) gemäß der allgemeine Vorschrift XII) zur Reaktion gebracht und es wurden 3,92 g eines hellbraunen Feststoff (3,90 mmol, 78 % der Theorie) erhalten.
**¹H-NMR** (300 MHz, CDCl₃): δ [ppm] = 1,39 (s, 9 H), 1,40 (s, 9 H), 1,41 (s, 9 H), 1,43 (s, 9 H), 2.41 (s, 3 H), 2.52 (s, 3 H), 3.48 - 3,56 (m, 3 H), 3,81 - 3,90 (m, 3 H), 3,93 - 4,32 (m, 5 H), 4,45 (m, 1 H), 4.60 - 5.30 (m, 2 H), 4,90 (m, 1 H), 5.45 - 5.68 (m, 2 H), 5.73 (m, 1 H), 6,11 (bs, 1 H), 7.63 (s, 1 H), 7.88 (s, 1 H), 8,96 (bs, 1 H); - **MS** (ESI-MS, H₂O/MeOH + 0.1 % TFA): m/z (%) = 1005.5 (100, (MH⁺)); - **MG** = 1005,05 g/mol - **MF** = C₄₅H₆₄N₈O₁₈

### XIIb) 2,6-Bis-tert-butoxycarbonylamino-hexanoic acid 2,3,4-tris-(2,6-Bis-tert-butoxycarbonylamino-hexanoxy)-5-(7,8-dimethyl-2,4-dioxo-3,4-dihydro-2H-benzo[g]pteridin-10-yl)-pentyl ester (74)

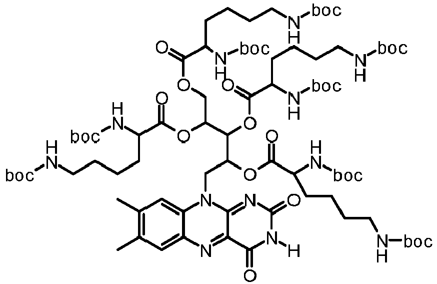

Die Umsetzung von α,ε-di-Boc-L-Lysin (6,84 g, 20 mmol) mit Riboflavin (72) gemäß der allgemeine Vorschrift XII) ergab 5,17g eines hellbraunen Feststoff (3,06 mmol, 61 % der Theorie).
**¹H-NMR** (300 MHz, CDCl₃): δ [ppm] = 1,36 - 1,52 (m, 72 H), 1,21 - 1,42 (m, 16 H), 1,44 - 1,86 (m, 16 H), 2.40 (s, 3 H), 2.53 (s, 3 H), 2.98 - 3,06 (m, 4 H), 3,41 (m, 1 H), 3,61 (m, 3 H), 3,93 - 4,32 (m, 3 H), 4,43 (m, 1 H), 4.60 - 5.30 (m, 2 H), 4,86 (m, 1 H), 5.11 (m, 2 H), 5.58 (m, 1 H), 7.61 (s, 1 H), 7.90 (s, 1 H), 8,94 (bs, 1 H); - MS (ESI-MS, H₂O/MeOH + 0.1 % TFA): m/z (%) = 1707.3 (61, (M+NH₄⁺)), 1690.2 (100, (MH⁺)), 845.8 (88, (M+2H⁺)²⁺); - **MG** = 1690,02 g/mol - **MF** = C₈₁H₁₃₂N₁₂O₂₆

### XIII) Amino-acetic acid 2,3,4-tris-(2-amino-acetoxy)-5-(7,8-dimethyl-2,4-dioxo-3,4-dihydro-2H-benzo[g]pteridin-10-yl)-pentyl ester tetrahydrochlorid (75)

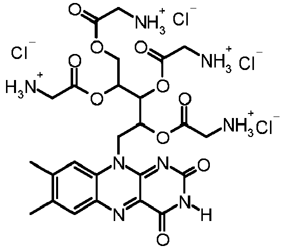

Das unter XIIa) erhaltene Flavin **(73)** wurde nach der allgemeinen Vorschrift III) entschützt und es wurden 1,44 g eines hellbraunen Feststoffes (1,69 mmol, 85 % der Theorie) erhalten.
**MS** (ESI-MS, H₂O/MeOH + 0.1 % TFA): m/z (%) = 605.5 (36, (MH⁺)), 303.3 (100, (M+2H⁺)²⁺), 202.4 (41, (M+3H⁺)³⁺); - **MG** = 608.61 + 4x 35.45 g/mol - **MF** = C₂₅H₃₆N₈O₁₀Cl₄

### XIV) 2,6-Bis-amino-hexanoic acid 2,3,4-tris-(2,6-bis-amino-hexanoxy)-5-(7,8-dimethyl-2,4-dioxo-3,4-dihydro-2H-benzo[g]pteridin-10-yl)-pentyl ester octahydrochlorid (76)

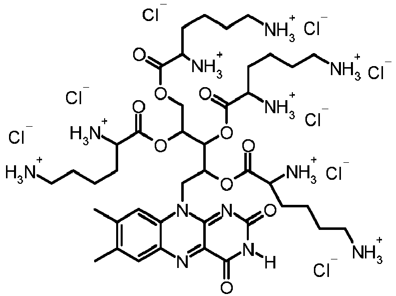

Das unter XIIb) erhaltene Flavin **(74)** wurde nach der allgemeinen Vorschrift III) entschützt und es wurden 1,49 g eines braunen Feststoffes (1,26 mmol, 63 % der Theorie) erhalten.
**MS** (ESI-MS, H₂O/MeOH + 0.1 % TFA): m/z (%) = 898.2 (15, (MH⁺)), 449.5 (100, (M+2H⁺)²⁺), 300.1 (9, (M+3H⁺)³⁺); - **MG** = 897.14 + 8x 35.45 g/mol - **MF** = C₄₁H₇₆N₁₂O₁₀Cl₈

### XV) 1-{3-[7,8-Dimethyl-2,4-dioxo-10-(butyl)-4,10-dihydro-2H-benzo[g]pteridin-3-yl]-propyl}-methyl-[4,4']bipyridinyl-1-ium diiodid (118)

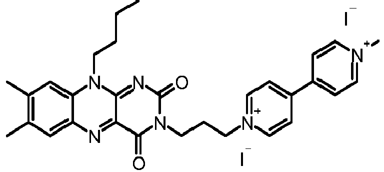

3-(10-Butyl-7,8-dimethyl-2,4-dioxo-4,10-dihydro-2H-benzo[g]pteridin-3-yl)-propyliodid **(xx)** (0.51 g, 1.05 mmol) wurde mit N-Methyl-4,4'-bipyridinium iodid (356 mg, 1,2 mmol) und NaHCO₃ (0.1 g, 1.2 mmol) in DMF (10 mL) in Stickstoffatmosphäre im Dunklen bei 50°C für 24 h gerührt. Das Lösungsmittel wurde bei vermindertem Druck abgezogen und der Rückstand durch präparative Dünnschichtchromatographie gereinigt (CHCl₃/MeOH 6:1).
Man erhält 351 mg eines orangen Feststoffes (0,34 mmol, 45 % der Theorie).
**¹H-NMR** (300 MHz, MeOD): δ [ppm] = 0,92 (t, 3 H), 1,46 (m, 2 H), 1,72 (m, 2H), 2,32 (m, 2 H), 2,47 (s, 3 H), 2,56 (s, 3 H,), 3,96 (m, 2 H), 4,16 - 4,32 (m, 3 H), 4,51 (m, 1 H), 4,86 (m, 2 H), 5,16 (m, 2 H), 5,48 (m, 1 H), 5,52 (m, 1 H), 5,64 (m, 1 H), 7,84 (s, 1 H), 7,92 (s, 1 H), 8,22 (d, J = 5,6 Hz, 2 H), 8,97 (d, J = 5,6 Hz, 2 H), 9,23 (d, J = 5,6 Hz, 2 H), 9,59 (d, J = 5,6 Hz, 2 H); - **MS** (ESI-MS, H₂O/MeOH + 0.1 % TFA): m/z (%) = 510.1 (19, (M⁺)), 255.0 (100, (M²⁺)); - **MG** = 510.6 + 2x 126.90 - **MF** = C₃₀H₃₄N₆O₂I₂

### Beispiel 2) Phototoxizitätsexperimente

### a) Herstellung der Testplatten und Bakterienstämme

Eine Probe des Bakterienstammes *Staphylococcus. aureus* (ATCC-Nummer: 25923) *oder Escherichia. coli* (ATCC-Nummer: 25922) wurde aus einer Cryo-Gefrierkultur entnommen, auf Müller-Hinton-Agar-Platten vereinzelt, und unter aeroben Bedingungen bei 37°C in einer Übernachtkultur kultiviert. Daran anschließend wurden 5 ml Müller-Hinton-Flüssigmedium mit einem Abstrich der Bakterienkultur (Einzelkolonie) beimpft und über Nacht bei 37°C bebrütet. Die so gewonnene Bakteriensuspension wurde 10 min bei 2500 Upm zentrifugiert und das erhaltene Bakterienpellet in 5 ml sterilem PBS resuspendiert. Die optische Dichte der Bakteriensupensionen für die Phototoxizitätstests betrug OD₆₀₀ₙₘ= 0.6, was einer Bakterienzahl von ∼1-8x10⁸⁻¹² Bakterien pro ml entspricht. Die biochemische Analyse und Resistenzbestimmung der Bakterien wurde mit dem VITEK2-System gemäß den Richtlinien M100-S14 des NCCLS (2004) durchgeführt.

Zur Empfindlichkeitsprüfung medizinisch bedeutender Krankheitserreger gegenüber Antibiotika und Sulfonamiden wurden gemäß der NCCLS Richtlinien Müller-Hinton-Medien verwendet (Deutsche Gesellschaft für Hygiene und Mikrobiologie (DGHM), Institut für Hygiene und Mikrobiologie, Universität Bonn, Deutschland):
a) Müller-Hinton-Bouillon (Oxoid, Wesel, Deutschland)
   2,0 g/l Rindfleisch, getrocknete Infusion aus 300 g, 17,5 g/l Caseinhydrolysat, 1,5 g/l Stärke, pH: 7,4 + 0,2
b) Müller Hinton-Agar (Oxoid, Wesel, Deutschland)
   2,0 g/l Rindfleisch, getrocknete Infusion aus 300 g, 17,5 g/l Caseinhydrolysat,
   1,5 g/l Stärke, pH: 7,4 + 0,2
   13 g/l Agaragar

### b) Durchführung des Phototoxizitätstests:

200 µl einer Bakteriensuspension (Bakteriendichte: 10⁸-10¹² /ml) wurden mit je 200 µl verschiedener Konzentrationen der zu testenden Photosensibilisatoren bei Raumtemperatur für 10 min oder 30 min inkubiert. Danach wurden die Bakterien zweimal mit Aqua dest. gewaschen, in 200 µl Aqua dest. resuspendiert, das gesamte Volumen auf eine 96-well Mikrotiterplatte übertragen und anschließend bestrahlt. Die verwendeten Photosensibilisatoren wurden in Aqua dest. gelöst und verschiedene Verdünnungsreihen hergestellt (0µM, 1µM, 10µM, 100µM).

Zur Sensibilisierung wurde die Lampe Omnicure Series 2000 (Photonics Solutions Inc., Edinburgh, UK) benutzt, die Licht aus einem Bereich von 390 nm bis 500nm emittiert und Emissionsmaxima Eₘₐₓ bei 405 nm und 436 nm aufweist. Die applizierte Leistung betrug jeweils 50mW/cm².

Als Kontrollen wurden bestrahlte und nicht bestrahlte Proben verwendet. Ebenso wurden nur mit Photosensibilisator inkubierte Bakteriensuspensionen mitgeführt (Dunkelkontrolle).

Die Bestimmung der koloniebildenden Einheiten (KBE bzw. CFU) pro ml wurde gemäß der von Miles und Misra publizierten Methode durchgeführt (Miles, AA; Misra, SS, Irwin, JO (1938 Nov). "The estimation of the bactericidal power of the blood.". The Journal of hygiene 38 (6): 732-49). Dazu wurden serielle Verdünnungen von 10⁻² bis 10⁻⁹ der entsprechenden Bakteriensuspension hergestellt. Je 3x 20 µl der entsprechenden Bakterienverdünnungen wurden dann auf Müller-Hinton Platten aufgetropft und bei 37° C für 24 h inkubiert. Danach wurde die Anzahl der überlebenden koloniebildenden Einheiten (KBE bzw. CFU) bestimmt. Alle Versuche wurden jeweils dreimal wiederholt.

### c) Ergebnis der Phototoxizitätsexperimente:

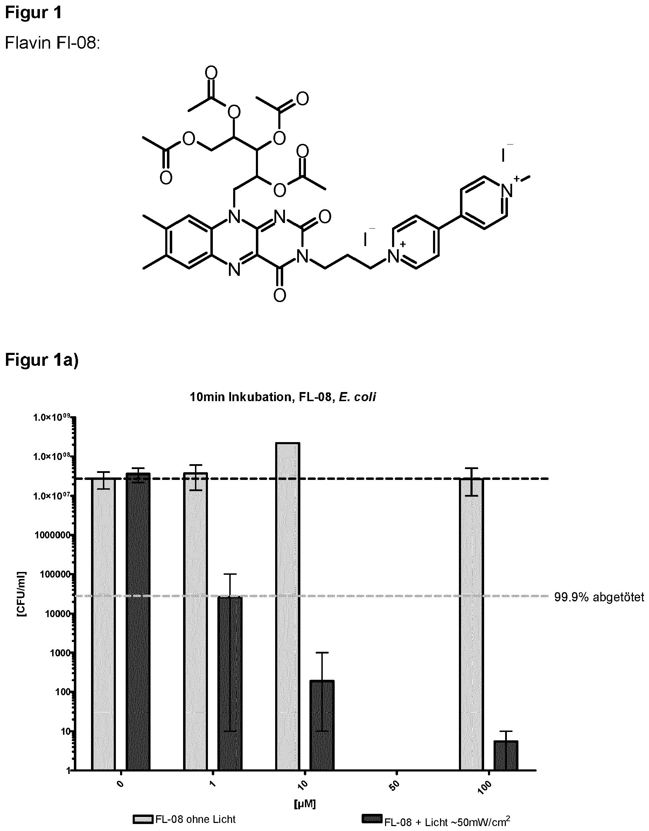

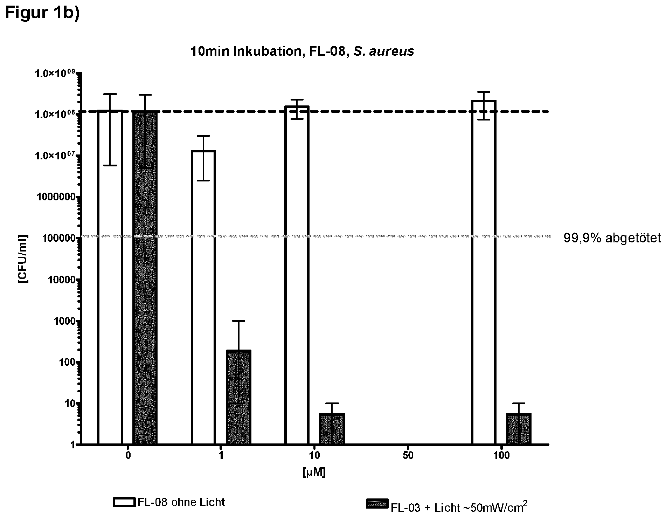

Die Ergebnisse der Phototoxizitätsexperimente sind in den Figuren 1-11 dargestellt.

Die Figuren 1-12 zeigen die logarithmische Abnahmen der CFU/ml 24 Std nach Bestrahlung sowie die dazugehörigen Kontrollen (nur bestrahlte Bakterien; mit Photosensibilisator inkubierte Bakterien, aber nicht bestrahlt; unbehandelte Bakterien) für den jeweils angegeben Photosensibilisator.

Die angegebenen koloniebildenden Einheiten (KBE bzw. CFU) pro ml sind jeweils der Median aus drei Versuchen.
Figur 1 zeigt die Wirkung von Flavin FL-08 (Diiodid der Verbindung mit der Formel (47)) auf E. coli und S. aureus.
   Figur 1a: E. coli Proben wurden mit verschiedenen Konzentrationen (0 µM, 1 µM, 10 µM, 100 µM) von Flavin FL-08 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-08, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 1b: S. aureus Proben wurden mit verschiedenen Konzentrationen (0 µM, 1 µM, 10 µM, 100 µM) von Flavin FL-08 für 10 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-08, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

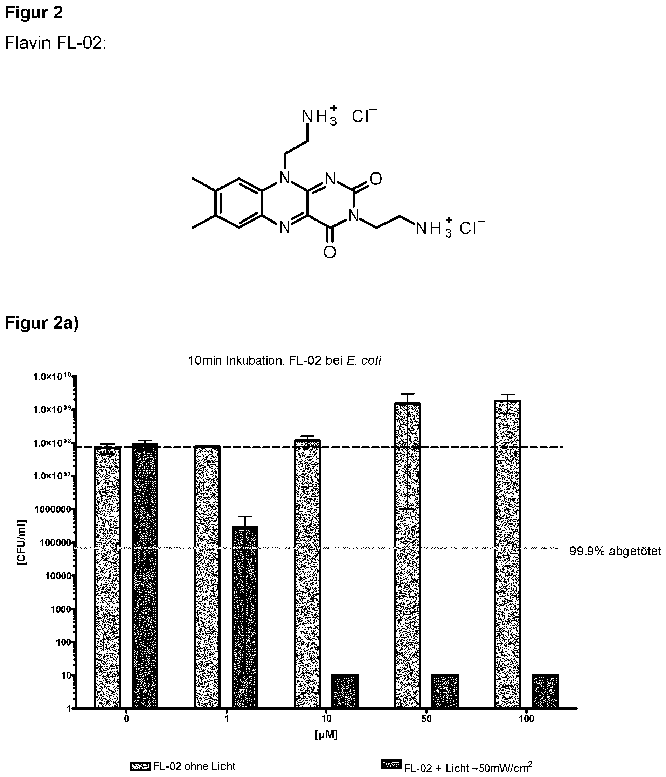

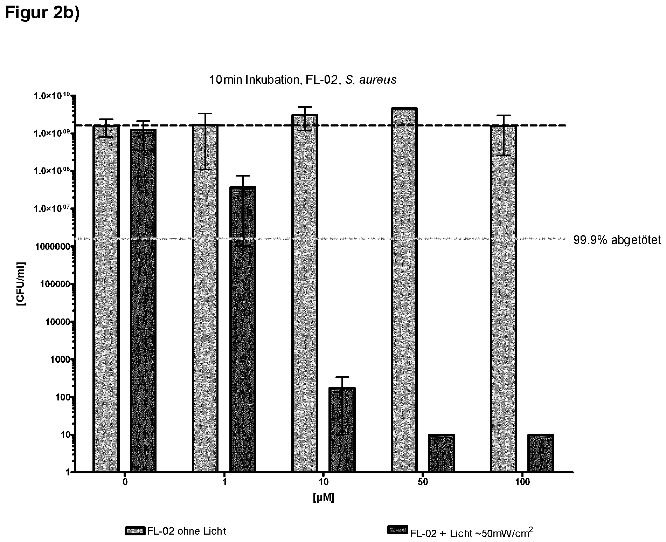

Figur 2 zeigt die Wirkung von Flavin FL-02 (Dichlorid der Verbindung mit der Formel (20)) auf E. coli und S. aureus.
   Figur 2a: E. coli Proben wurden mit verschiedenen Konzentrationen (0 µM, 1 µM, 10 µM, 50 µM, 100 µM) von Flavin FL-02 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-02, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 2b: S. aureus Proben wurden mit verschiedenen Konzentrationen (0 µM, 1 µM, 10 µM, 50 µM, 100 µM) von Flavin FL-02 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-02, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

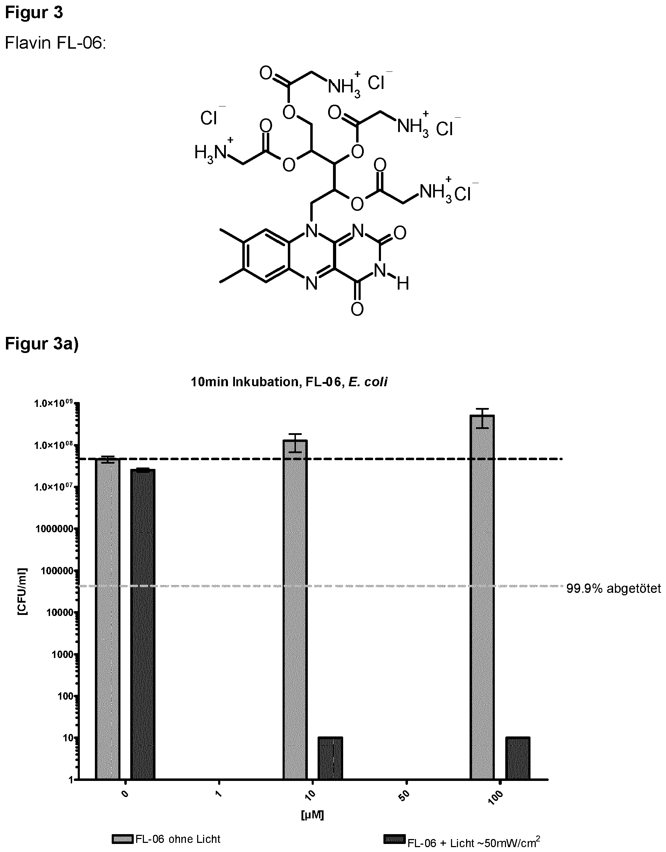

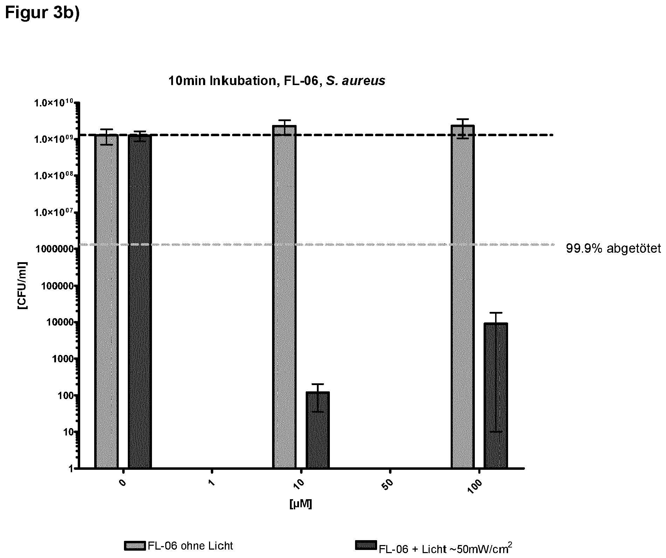

Figur 3 zeigt die Wirkung von Flavin FL-06 (Iodid der Verbindung mit der Formel (37)) auf E. coli und S. aureus.
   Figur 3a: E. coli Proben wurden mit verschiedenen Konzentrationen (0 µM, 10 µM, 100 µM) von Flavin FL-06 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-06, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 3b: S. aureus Proben wurden mit verschiedenen Konzentrationen (0 µM, 10 µM, 100 µM) von Flavin FL-06 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-06, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

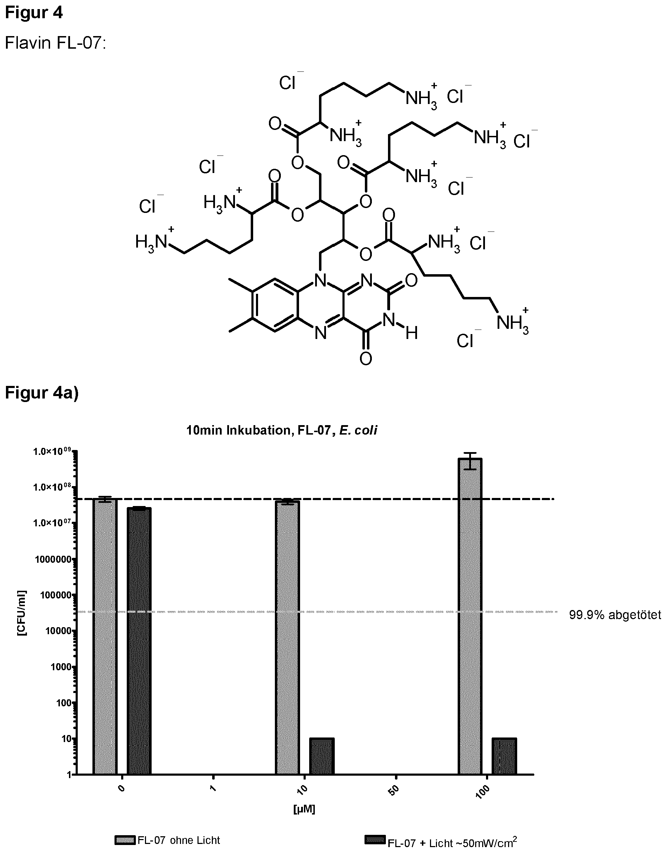

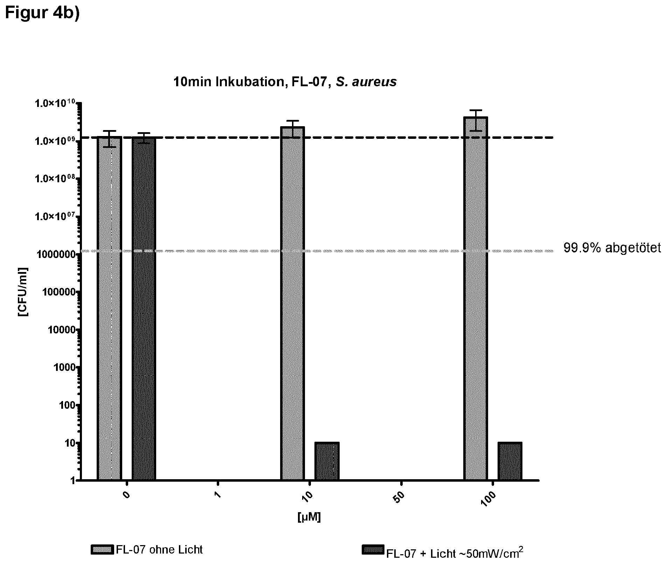

Figur 4 zeigt die Wirkung von Flavin FL-07 (Octachlorid der Verbindung mit der Formel (49)) auf E. coli und S. aureus.
   Figur 4a: E. coli Proben wurden mit verschiedenen Konzentrationen (0 µM, 10 µM, 100 µM) von Flavin FL-07 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-07, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 4b: S. aureus Proben wurden mit verschiedenen Konzentrationen (0 µM, 10 µM, 100 µM) von Flavin FL-07 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-07, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

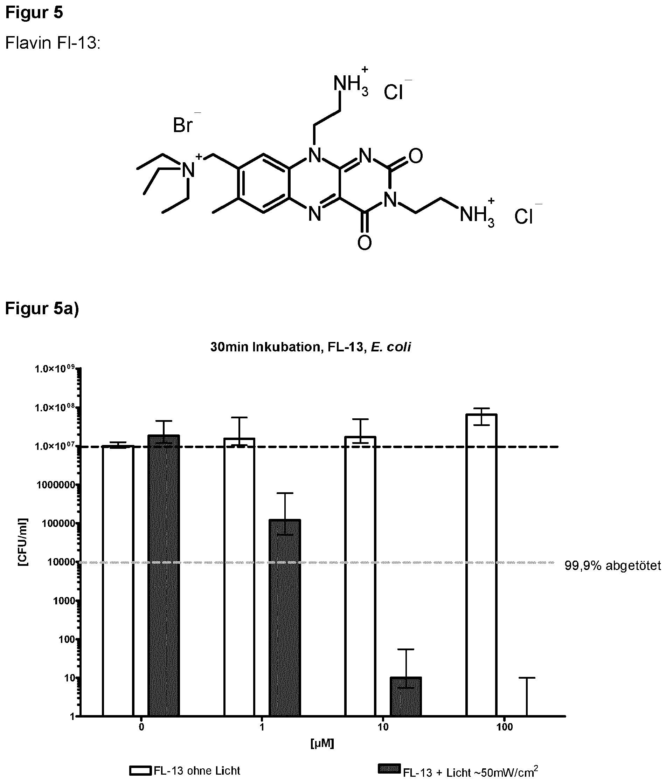

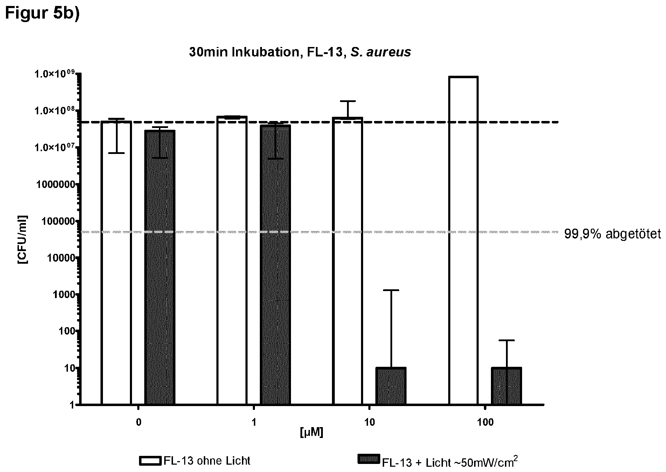

Figur 5 zeigt die Wirkung von Flavin FL-13 (Bromid-dichlorid der Verbindung mit der Formel (41)) auf E. coli und S. aureus.
   Figur 5a: E. coli Proben wurden mit verschiedenen Konzentrationen (0 µM, 1 µM, 10 µM, 100 µM) von Flavin FL-13 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-13, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 5b: S. aureus Proben wurden mit verschiedenen Konzentrationen (0 µM, 1 µM, 10 µM, 100 µM) von Flavin FL-13 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-13, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

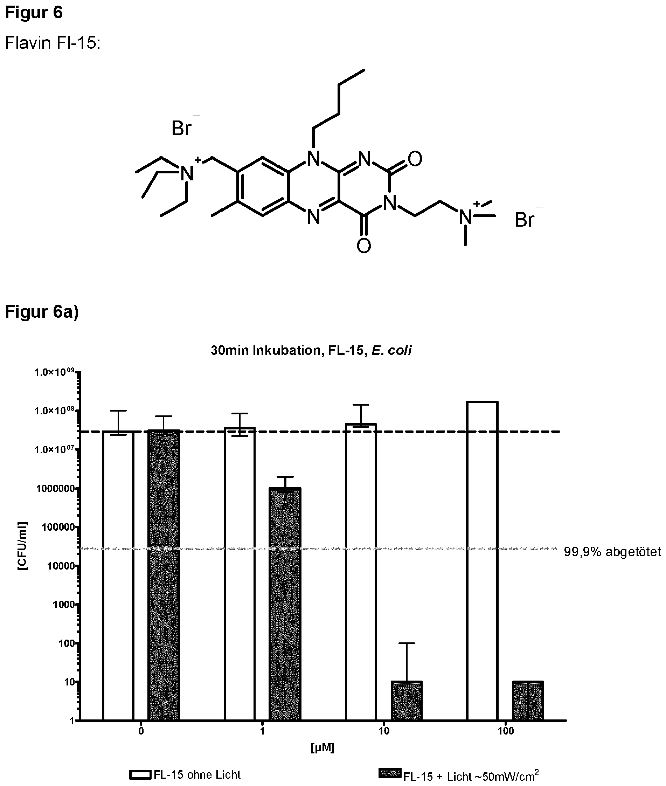

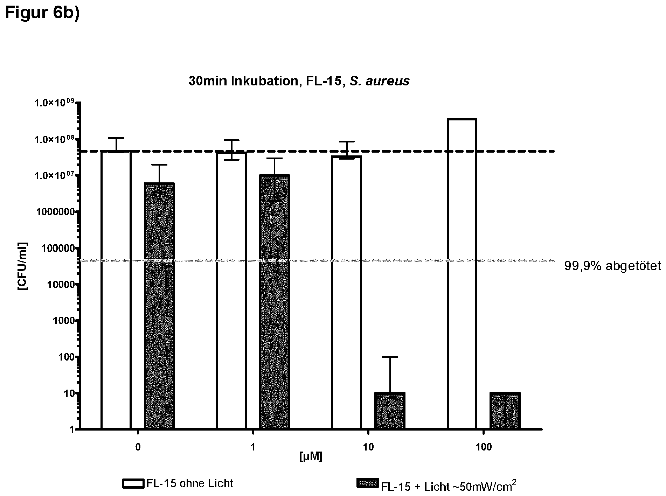

Figur 6 zeigt die Wirkung von Flavin FL-15 (Dibromid der Verbindung mit der Formel (42)) auf E. coli und S. aureus.
   Figur 6a: E. coli Proben wurden mit verschiedenen Konzentrationen (0 µM, 1 µM, 10 µM, 100 µM) von Flavin FL-15 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-15, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 6b: S. aureus Proben wurden mit verschiedenen Konzentrationen (0 µM, 1 µM, 10 µM, 100 µM) von Flavin FL-15 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-15, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

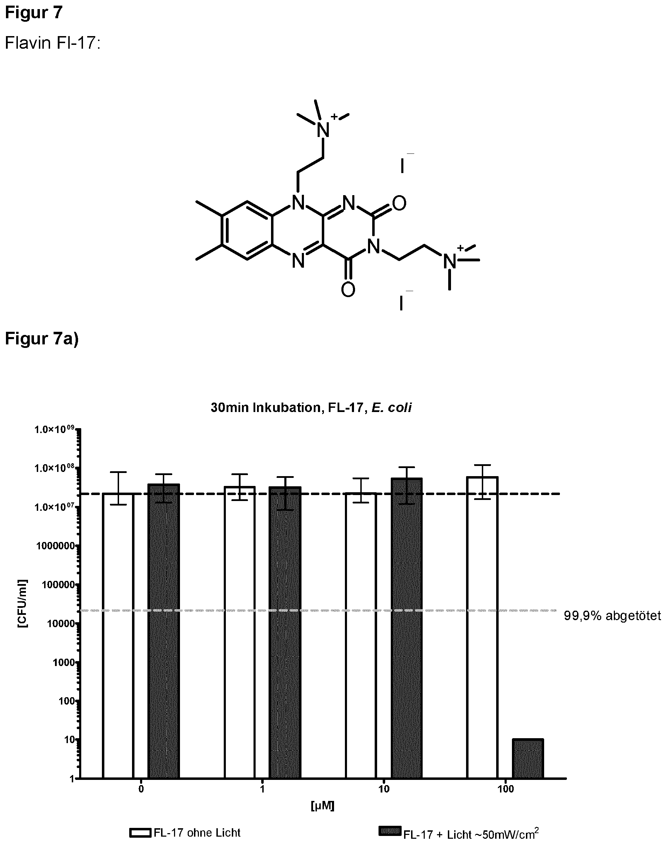

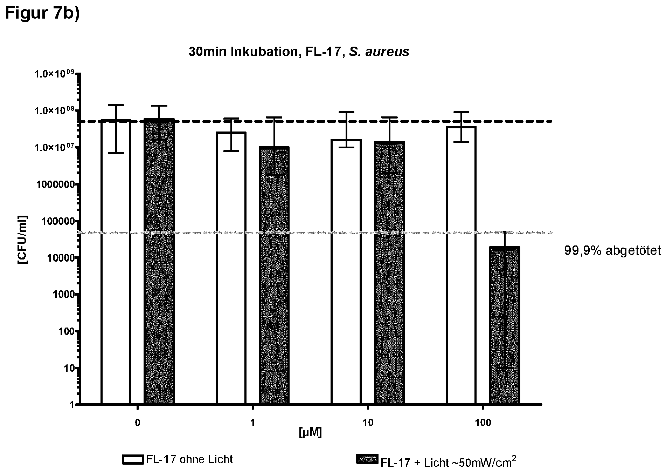

Figur 7 zeigt die Wirkung von Flavin FL-17 (Diiodid der Verbindung mit der Formel (40)) auf E. coli und S. aureus.
   Figur 7a: E. coli Proben wurden mit verschiedenen Konzentrationen (0 µM, 1 µM, 10 µM, 100 µM) von Flavin FL-17 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-17, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 7b: S. aureus Proben wurden mit verschiedenen Konzentrationen (0 µM, 1 µM, 10 µM, 100 µM) von Flavin FL-17 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-17, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

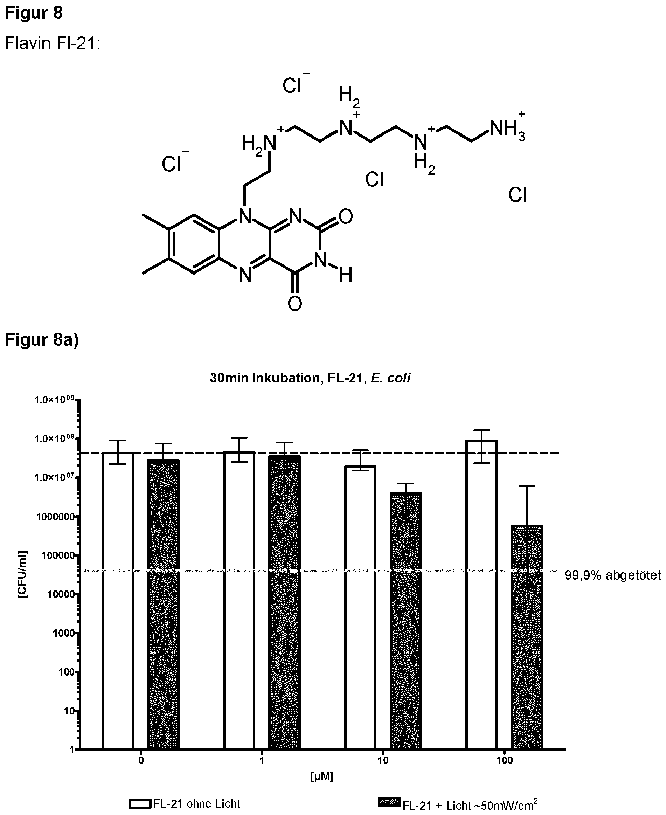

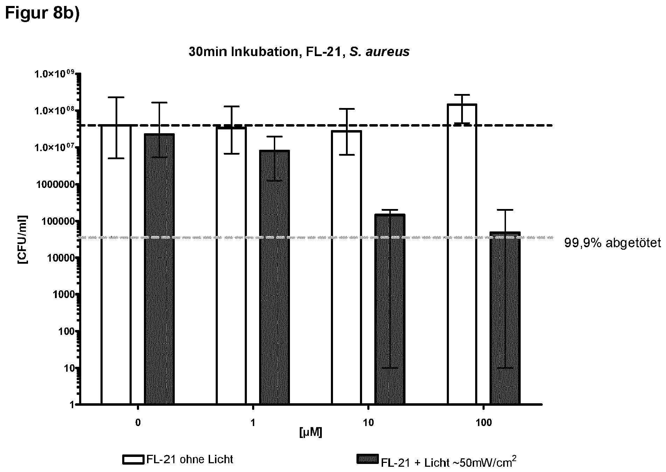

Figur 8 zeigt die Wirkung von Flavin FL-21 (Iodid der Verbindung mit der Formel (46)) auf E. coli und S. aureus.
   Figur 8a: E. coli Proben wurden mit verschiedenen Konzentrationen (0 µM, 1 µM, 10 µM, 100 µM) von Flavin FL-21 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-21, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 8b: S. aureus Proben wurden mit verschiedenen Konzentrationen (0 µM, 1 µM, 10 µM, 100 µM) von Flavin FL-21 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-21, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

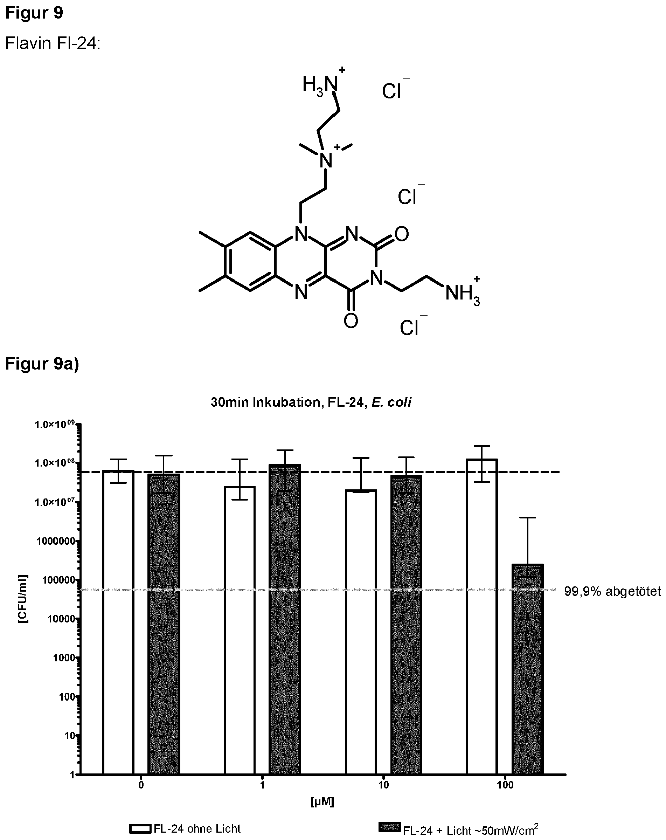

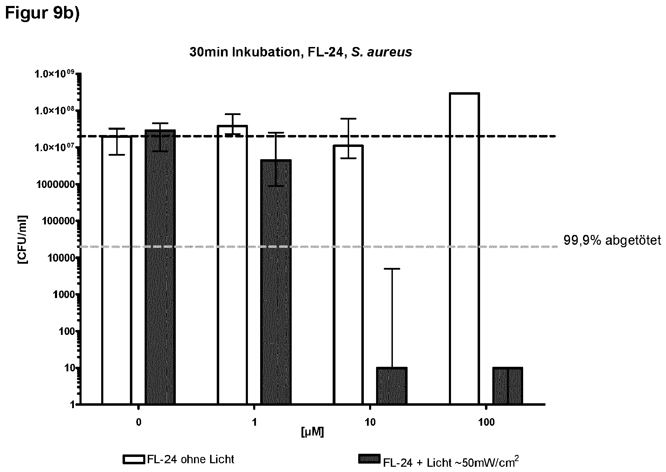

Figur 9 zeigt die Wirkung von Flavin FL-24 (Trichlorid der Verbindung mit der Formel (44)) auf E. coli und S. aureus.
   Figur 9a: E. coli Proben wurden mit verschiedenen Konzentrationen (0 µM, 1 µM, 10 µM, 100 µM) von Flavin FL-24 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-24, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 9b: S. aureus Proben wurden mit verschiedenen Konzentrationen (0 µM, 1 µM, 10 µM, 100 µM) von Flavin FL-24 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-24, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

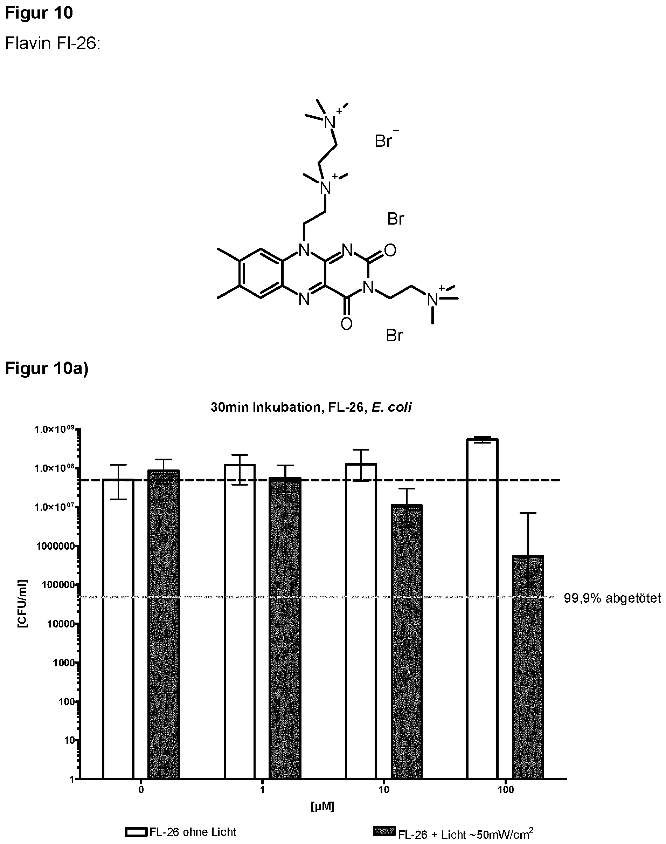

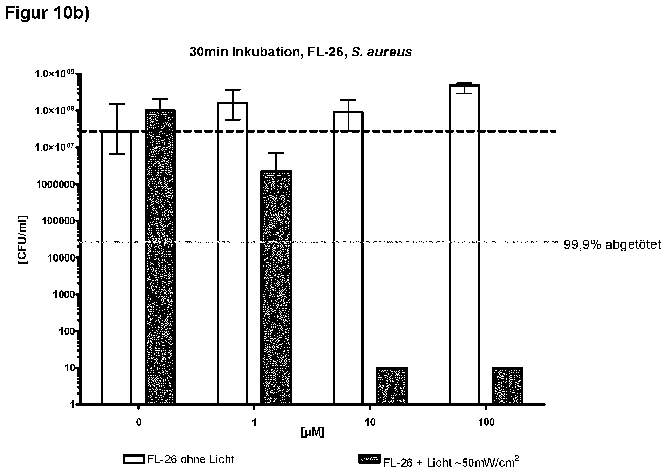

Figur 10 zeigt die Wirkung von Flavin FL-26 (Tribromid der Verbindung mit der Formel (43)) auf E. coli und S. aureus.
   Figur 10a: E. coli Proben wurden mit verschiedenen Konzentrationen (0 µM, 1 µM, 10 µM, 100 µM) von Flavin FL-26 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-26, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 10b: S. aureus Proben wurden mit verschiedenen Konzentrationen (0 µM, 1 µM, 10 µM, 100 µM) von Flavin FL-26 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-26, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

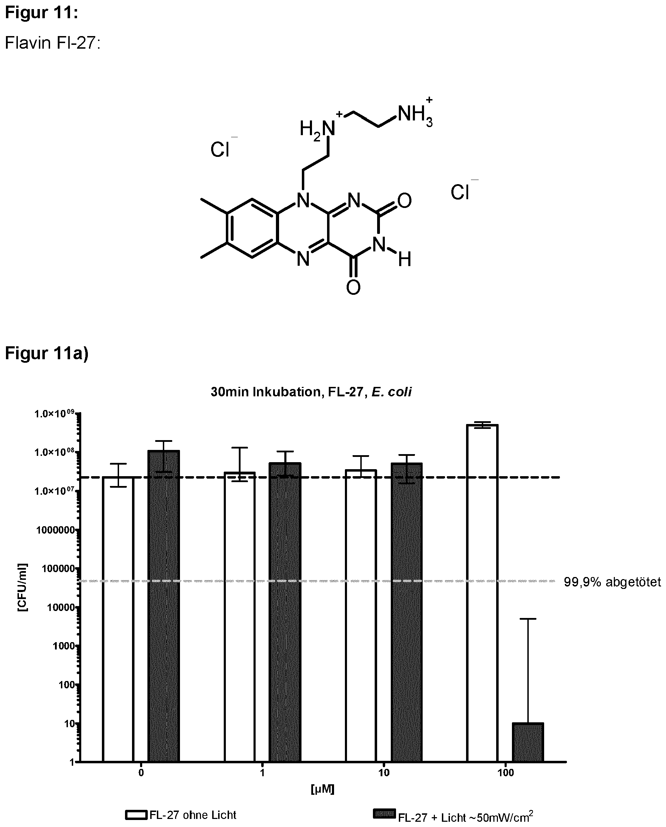

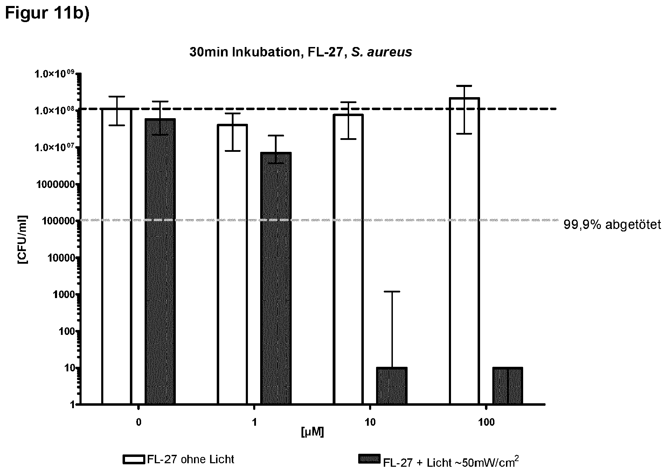

Figur 11 zeigt die Wirkung von Flavin FL-27 (Dichlorid der Verbindung mit der Formel (45)) auf E. coli und S. aureus.
   Figur 11a: E. coli Proben wurden mit verschiedenen Konzentrationen (0 µM, 1 µM, 10 µM, 100 µM) von Flavin FL-27 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-27, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.
   Figur 11b: S. aureus Proben wurden mit verschiedenen Konzentrationen (0 µM, 1 µM, 10 µM, 100 µM) von Flavin FL-27 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-27, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

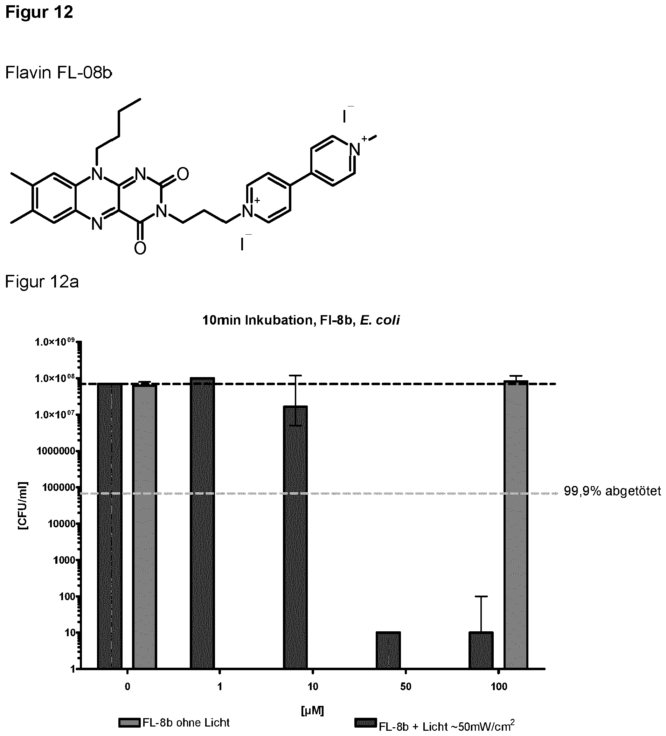

Figur 12 zeigt die Wirkung von Flavin FL-27 (Dichlorid der Verbindung mit der Formel (51)) auf E. coli und S. aureus.
   Figur 12a: E. coli wurde mit verschiedenen Konzentrationen von FL-08b [µM] für 10 min inkubiert, anschließend wurden die Proben mit 50mW/cm2 (210sec; 10.5J/cm2) bestrahlt. 24h später wurde die überlebenden Kolonien ausgezählt (CFU/ml). Graue Balken: Dunkelkontrolle ohne Licht. Rote Balken: mit Photosensibilisator inkubiert und bestrahlt. Die schwarze Linie kennzeichnet die Referenz der Dunkelkontrolle (kein Photosensibilisatorinkubation, kein Licht). Die grüne Linie kennzeichnet eine Abnahme der CFU/ml um 3-log10-Stufen; dies entspricht eine Abnahme um 99.9% ("antibakterielle Wirkung"). N= 3; Median CFU/ml ± SEM.
   Figur 12b: S. aureus wurde mit verschiedenen Konzentrationen von FL-08b [µM] für 10 min inkubiert, anschließend wurden die Proben mit 50mW/cm2 (210sec; 10.5J/cm2) bestrahlt. 24h später wurde die überlebenden Kolonien ausgezählt (CFU/ml). Graue Balken: Dunkelkontrolle ohne Licht. Rote Balken: mit Photosensibilisator inkubiert und bestrahlt. Die schwarze Linie kennzeichnet die Referenz der Dunkelkontrolle (kein Photosensibilisatorinkubation, kein Licht). Die grüne Linie kennzeichnet eine Abnahme der CFU/ml um 3-log10-Stufen; dies entspricht eine Abnahme um 99.9% ("antibakterielle Wirkung"). N= 3; Median CFU/ml ± SEM.

Wie aus den Figuren 1-12 ersichtlich, hat eine Bestrahlung der verwendeten Mikroorganismen *Staphylococcus aureus* (*S*. *aureus*) und *Escherichia coli* (*E*. *coli)* mit einer Lichtdosis von 10.5J/cm² mit blauem Licht (390nm - 500nm) in Abwesenheit eines Photosensibilisators (0µM des jeweiligen Flavins) keinen Einfluss auf die Anzahl der überlebenden Mikroorganismen im Vergleich zur unbelichteten Kontrolle.

Darüber hinaus zeigen die in den Figuren 1-12 dargestellten Ergebnisse, dass die Inkubation (10min bzw. 30min) des jeweiligen Photosensibilisators mit den Mikroorganismen ohne nachfolgende Belichtung ebenfalls keinen oder nur einen geringen Einfluss auf die Anzahl der überlebenden Mikroorganismen hat.

Wie aus den Figuren 1-12 ersichtlich erfolgt nach Inkubation (10min bzw. 30min) der Mikroorganismen in Abhängigkeit der verwendeten Konzentration der jeweiligen Photosensibilisatoren und nachfolgender Bestrahlung mit einer Lichtdosis von 10.5J/cm² eine Abnahme der KBE/ml und somit eine Inaktivierung von *E*. *coli* und *S*. *aureus.*

Die Effektivität der Phototoxizität gegenüber Bakterien nach Bestrahlung wurde nach folgenden Richtlinen für Handhygiene im Gesundheitswesen festgelegt (Boyce, J. M., and D. Pittet. 2002. Guideline for Hand Hygiene in Health-Care Settings: recommendations of the Healthcare Infection Control Practices Advisory Committee and the HICPAC/SHEA/APIC/IDSA Hand Hygiene Task Force. Infect Control Hosp Epidemiol 23:S3-40.):

| | | |
|---|---|---|
| - Reduktion der KBE/ml um 1 log₁₀-Stufe | 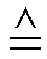 | 90% Effektivität |
| - Reduktion der KBE/ml um 3 log₁₀-Stufen | 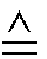 | 99,9% Effektivität |
| - Reduktion der KBE/ml um 5 log₁₀-Stufen | 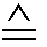 | 99,999% Effektivität. |

Für eine effektive Inaktivierung kann daher die Abnahme von ≥3log₁₀ Stufen angenommen werden, wobei *S*. *aureus* und *E*. *coli* als Beispiele für Vertreter aus der Gruppe der Gram-positiven und Gram-negativen Bakterien ausgewählt wurden (siehe Boyce J.M and D. Pittet 2009).

Die benötigte Konzentration, um jeweils eine Reduktion um ≥3log₁₀ Stufen zu erzielen, ist in Tabelle 2 dargestellt.

**Tabelle 2: Zusammenfassung photodynamische Inaktivierung**

| **Photosensibilisator** | **Benötigte Konzentration [µM], um eine Reduktion um ≥ 3log₁₀ Stufen zu erzielen (Abnahme um 99.9%), Bestrahlung mit 10.5 J/cm²** | |
|---|---|---|
| | ***E.coli*** | ***S*. *aureus*** |
| **FL-02** | 10 | 10 |
| **FL-06** | 10 | 10 |
| **FL-07** | 10 | 10 |
| **FL-08** | 1 | 1 |
| **FL-08b** | 50 | 10 |
| **FL-13** | 10 | 10 |
| **FL-15** | 10 | 10 |
| **FL-17** | 100 | 100 |
| **FL-21** | >100 ^{(*)} | 100 |
| **FL-24** | >100 ^{(*)} | 10 |
| **FL-26** | >100 ^{(*)} | 10 |
| **FL-27** | 100 | 10 |

| | | |
|---|---|---|
| (*) bisher konnte nur eine Reduktion von weniger als 3-log₁₀-Stufen erzielt werden (99%), bei einer Konzentration von 100µM und nachfolgender Bestrahlung | | |

### Bevorzugte Aspekte:

Aspekt 1 betrifft eine Verbindung mit der Formel (1): 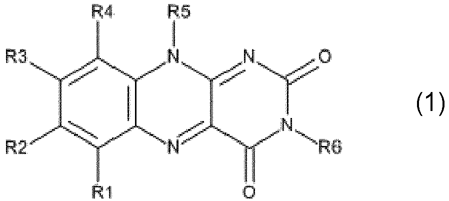
   wobei I) nur 1 Rest R1, R2, R3, R4, R5 oder R6 ein organischer Rest mit:
      a) wenigstens zwei neutralen, protonierbaren Stickstoffatomen, die nicht direkt an den Isoalloxazinring gebunden sind, oder
      b) wenigstens zwei positiv geladenen, vorzugsweise quartären, Stickstoffatomen, die nicht direkt an den Isoalloxazinring gebunden sind, bedeutet
      und wobei jeder der Reste R1, R2, R3 oder R4, der kein organischer Rest mit a) wenigstens zwei neutralen, protonierbaren Stickstoffatomen, die nicht direkt an den Isoalloxazinring gebunden sind, oder b) wenigstens zwei positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
      wobei jeder der Reste R5 oder R6, der kein organischer Rest mit a) wenigstens zwei neutralen, protonierbaren Stickstoffatomen, die nicht direkt an den Isoalloxazinring gebunden sind, oder b) wenigstens zwei positiv geladenen, vorzugsweise quartären, Stickstoffatomen, die nicht direkt an den Isoalloxazinring gebunden sind, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
      mit der Maßgabe, dass der Rest R3 kein Aminomethyl-Rest, wobei das Stickstoffatom unsubstituiert oder substituiert sein kann, bedeutet, und
      wobei Verbindungen ausgenommen sind, bei denen die Reste R1, R4 und R10 Wasserstoff sind, der Rest R2 Wasserstoff, Alkyl mit 1 bis 8 C-Atomen oder Cycloalkyl mit 3 bis 7 C-Atomen bedeutet, der Rest R3 Wasserstoff, Halogen, Alkyl mit 1 bis 8 C-Atomen, O-Alkyl mit 1 bis 8 C-Atomen oder ein heterocyclischer Rest mit 4 bis 7 C-Atomen bedeutet und der Rest R5 ein Alkylrest mit 1 bis 8 C-Atomen ist, der mit wenigstens mit einem Rest der Formel -N(R³⁵)(R³⁶) oder einem Rest der Formel -OR³⁵ substituiert ist, wobei der Rest R³⁵ ein Rest der Formel -C₁₋₈Alkyl(amin)-OR³⁷ oder ein 7,8-dimethyl-isoalloxazin-10-yl-C₁₋₈alkylrest bedeutet und wobei der Rest R³⁶ Wasserstoff oder ein Rest der Formel -C₁₋₈Alkyl, der unsubstituiert oder substituiert sein kann, bedeutet und wobei der Rest R³⁷ Wasserstoff, Aryl mit 6 bis 7 C-Atomen oder Alkyl, das unsubstituiert oder substituiert sein kann, mit 1 bis 8 C-Atomen bedeutet, und
      wobei weiterhin Verbindungen ausgenommen sind, bei denen die Reste R1 und R4 Wasserstoff bedeuten, der Rest R10 Wasserstoff oder ein Rest mit der allgemeinen Formel -C₁₋₄Alkyl-OC(O)CH₃ bedeutet, der Rest R2 Wasserstoff oder Alkyl mit 8 bis 8 C-Atomen bedeutet und der Rest R5 ein Rest mit der allgemeinen Formel -C₁₋₆Alkyl-N(R³¹)-C₀₋₃Alkyl-(R³²) bedeutet, wobei R³¹ Wasserstoff oder ein Rest mit der Formel -C₁₋₄Alkyl bedeutet und wobei R³² ein Rest mit der Formel -C₁₋₄Alkyl-N(R³³)(R³⁴), ein Rest mit der Formel -C₀₋₄Alkyl-Aryl, ein Rest mit der Formel -C₀₋₄Alkyl-Heterocycloalkyl oder ein Rest mit der Formel -C₀₋₄Alkyl-Heteroaryl bedeutet, und wobei die Reste R³³ und R³⁴ unabhängig voneinander Wasserstoff oder -C₁₋₄Alkyl bedeuten, und
      wobei 10-Butyl-7,8-dimethyl-3-[2-oxo-2-(1,4,7,10-tetrazacyclododec-1-yl)ethyl]benzo[g]pteridin-2,4-dion und 10-[2-(2-Methoxyethoxy)ethyl]-7,8-dimethyl-3-[2-oxo-2-(1,4,7,10-tetrazacyclododec-1-yl)ethyl]benzo[g]pteridin-2,4-dion ausgenommen sind,
      oder
   wobei II) nur 1 Rest R1, R2, R3, R4, R5 oder R6 ein organischer Rest mit:
      a) wenigstens einem neutralen, protonierbaren Stickstoffatom, das nicht direkt an den Isoalloxazinring gebunden ist, und
      b) wenigstens einem positiv geladenen, vorzugsweise quartären, Stickstoffatom, das nicht direkt an den Isoalloxazinring gebunden ist, bedeutet
      und wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
      wobei jeder der Reste R5 oder R6, der kein organischer Rest mit a) wenigstens einem neutralen, protonierbaren Stickstoffatom, das nicht direkt an den Isoalloxazinring gebunden ist, und b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
   wobei III) wenigstens 2 Reste R1, R2, R3, R4, R5 oder R6 ein organischer Rest mit:
      a) wenigstens einem neutralen, protonierbaren Stickstoffatom, das nicht direkt an den Isoalloxazinring gebunden ist, und/oder
      b) wenigstens einem positiv geladenen, vorzugsweise quartären, Stickstoffatom, das nicht direkt an den Isoalloxazinring gebunden ist, bedeutet und
      wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und/oder b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
      wobei jeder der Reste R5 oder R6, der kein organischer Rest mit a) wenigstens einem neutralen, protonierbaren Stickstoffatom, das nicht direkt an den Isoalloxazinring gebunden ist, und/oder b) wenigstens einem positiv geladenen, vorzugsweise quartären, Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet
      und wobei die Verbindung mit der Formel (20): 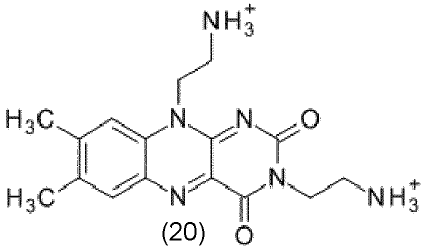 ausgenommen ist.
Aspekt 2 betrifft eineVerbindung nach Aspekt 1, wobei der organische Rest mit
   a) wenigstens einem neutralen, protonierbaren Stickstoffatom, das nicht direkt an den Isoalloxazinring gebunden ist, und/oder
   b) wenigstens einem positiv geladenen, vorzugsweise quartären, Stickstoffatom, das nicht direkt an den Isoalloxazinring gebunden ist, ein Rest der Formel (2), (3) oder (4): 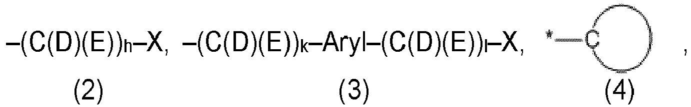 ist,
      wobei h eine ganze Zahl von 1 bis 20 und k und l jeweils unabhängig voneinander eine ganze Zahl von 0 bis 6 bedeuten und wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest ist, der a) wenigstens ein neutrales, protonierbares Stickstoffatom und/oder b) wenigstens ein positiv geladenes Stickstoffatom enthält, und der Rest Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
      wobei der Rest mit der Formel (4) einen Heteroaromaten, der über ein C-Atom des Heteroaromaten an den Isoalloxazinring gebunden ist und der a) wenigstens ein neutrales, protonierbares Stickstoffatom und/oder b) wenigstens ein positiv geladenes Stickstoffatom enthält, bedeutet.
Aspekt 3 betrifft eine Verbindung nach einem der Aspekte 1 oder 2, wobei die Reste R1 und R4, die jeweils unabhängig voneinander gleich oder voneinander verschieden sein können, Wasserstoff oder Methyl sind und wobei 2 der Reste R2, R3, R5 oder R6 ein organischer Rest der allgemeinen Formel -(C(D)(E))ₕ-X oder - (C(D)(E))ₖ-Aryl-(C(D)(E))ₗ-X sind.
Aspekt 4 betrifft eine Verbindung nach einem der Aspekte 1 bis 3, wobei X einen Rest der allgemeinen Formel (2): 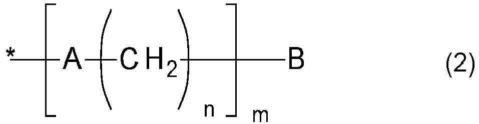 wobei A ein Sauerstoff- oder ein Schwefelatom ist und wobei n eine ganze Zahl von 1 bis 8 und m eine ganze Zahl von 0 bis 100 ist und wobei B ein Rest der Formel (3a),(3b), (4a), (4b), (5a) oder (5b): 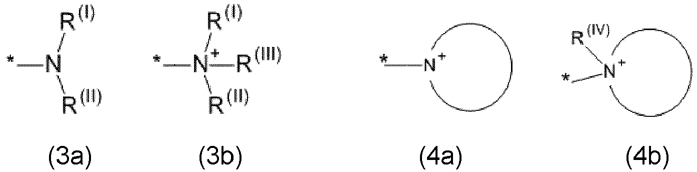 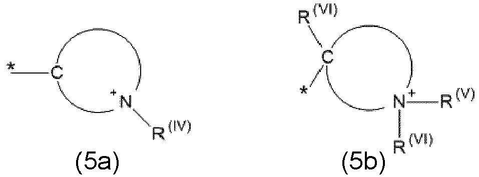 ist und wobei der Reste R^{(I)} ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist, wobei jeder der Reste R^{(II)}, R^{(III)}, R^{(IV)}, R^{(V)} und R^{(VI)} unabhängig voneinander Wasserstoff, ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist und wobei jeder der vorgenannten Arylreste, heterocyclische Reste, Alkylreste, Alkenylreste, Hydroxyalkylreste, Etherreste, Thioetherreste und Alkylaminoreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert ist,
   und wobei der Rest mit der Formel (4a) und der Rest mit der Formel (5a): 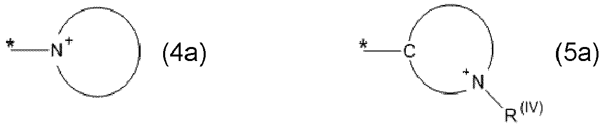 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 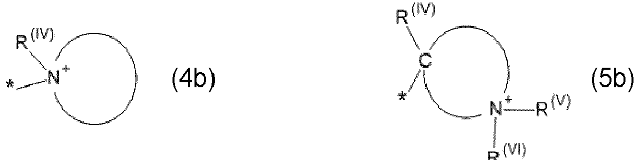 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, darstellt.
Aspekt 5 betrifft eine Verbindung nach einem der Aspekte 2 bis 4, wobei der organischer Rest mit:
   a) wenigstens zwei neutralen, protonierbaren Stickstoffatomen, die nicht direkt an den Isoalloxazinring gebunden sind, oder
   b) wenigstens zwei positiv geladenen, vorzugsweise quartären, Stickstoffatomen, die nicht direkt an den Isoalloxazinring gebunden sind, jeweils aus der Gruppe, die aus den Resten der Formel (30a) bis (36) besteht, 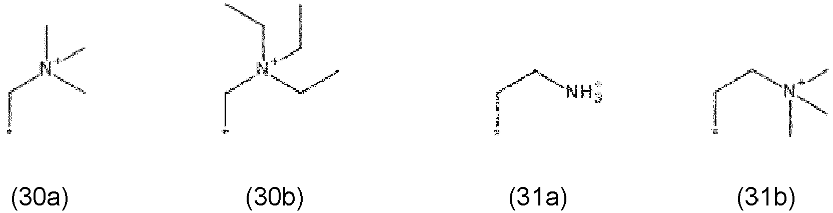 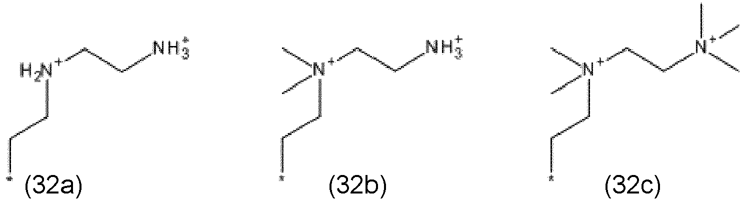 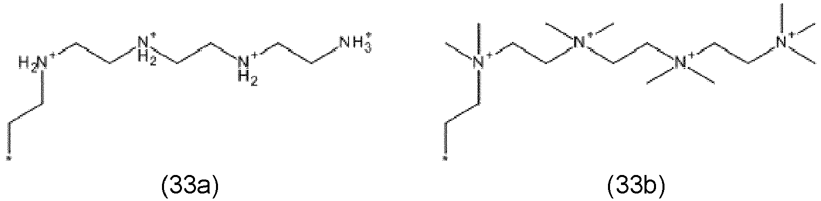 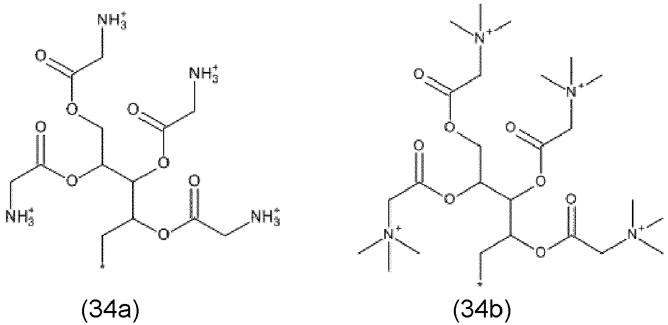 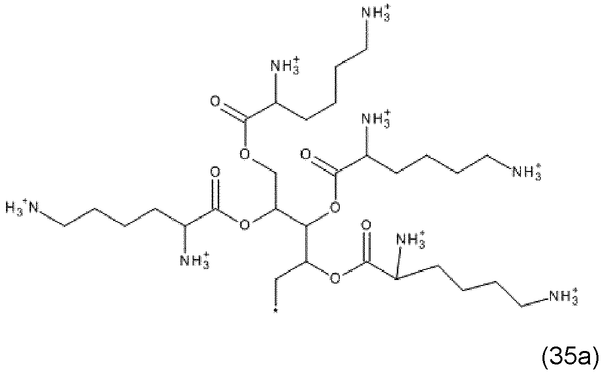 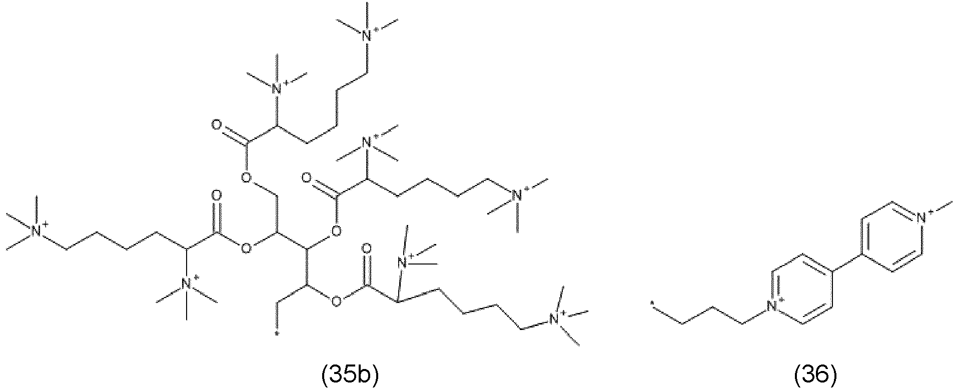
   ausgewählt wird.
Aspekt 6 betrifft eine Verbindung nach einem der Aspekte 1 bis 5, wobei das 10H-Benzo[g]pteridin-2,4-dion-Derivats der Formel (1) aus der Gruppe, die aus Verbindungen mit den Formeln (40) bis (49) und (115) bis (117) besteht, ausgewählt: 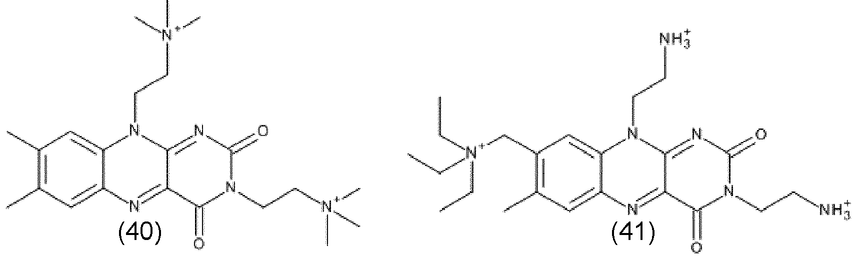 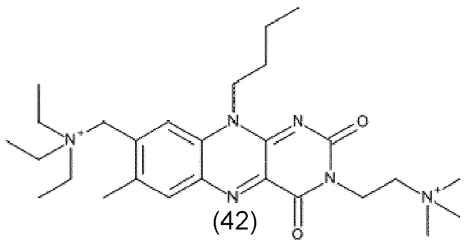 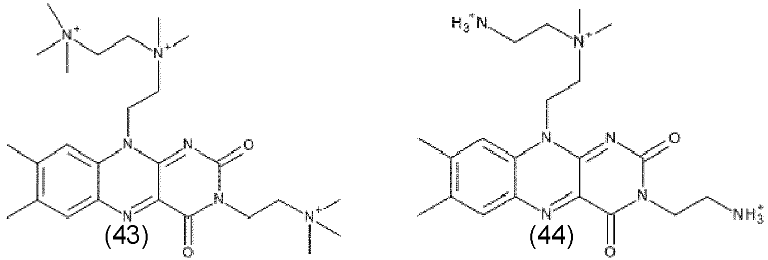 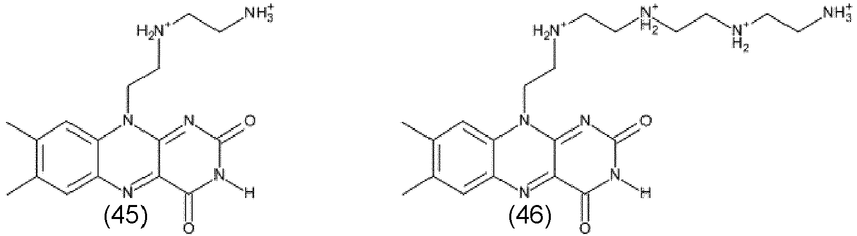 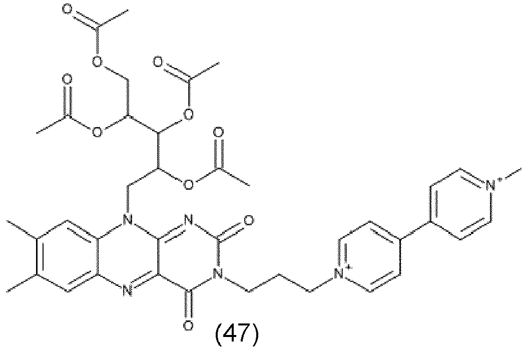 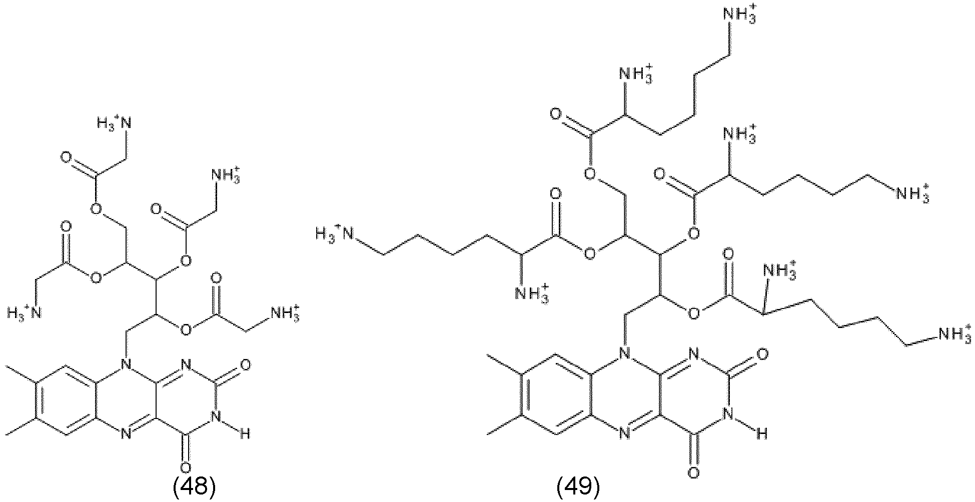 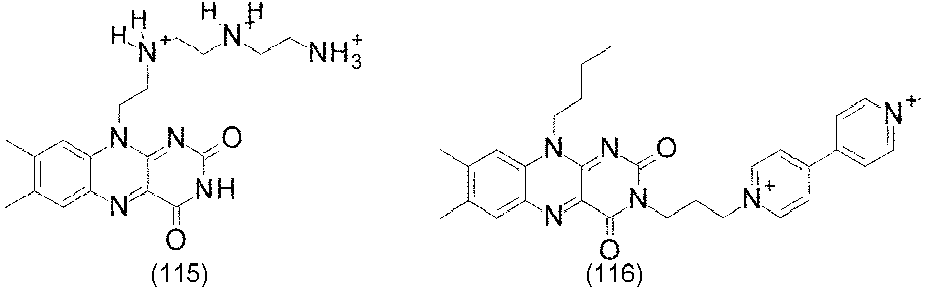 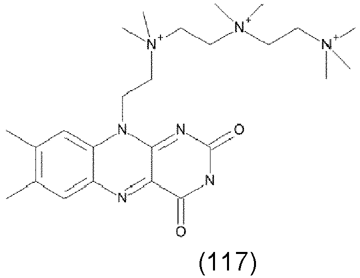
Aspekt 7 betrifft eine Verbindung nach einem der Aspekte 1 bis 6 zur Verwendung als Photosensibilisator, vorzugsweise bei der photodynamischen Therapie.
Aspekt 8. betrifft eine Verbindung nach einem der Aspekte 1 bis 7 zur Verwendung bei der Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden.
Aspekt 9 betrifft eineVerbindung nach einem der Aspekte 1 bis 8 zur Verwendung bei der Reinigung von Zähnen, Zahnersatz und/oder Zahnspangen und/oder Prophylaxe und/oder Behandlung einer Erkrankung des Zahngewebes und/oder des Zahnhalteapparates.
Aspekt 10 betrifft eine Verbindung nach einem der Aspekte 1 bis 8, zur Verwendung bei der Prophylaxe und/oder Behandlung einer infektiösen Hautkrankheit
Aspekt 11 betrifft eine Verbindung nach einem der Aspekte 1 bis 8, zur Verwendung bei der Oberflächenreinigung und/oder -beschichtung.
Aspekt 12 betrifft eine Verbindung nach Aspekt 10, zur Verwendung bei der Oberflächenreinigung und/oder -beschichtung von Medizinprodukten, Lebensmittelverpackungen oder Hygieneartikeln.
Aspekt 13 betrifft eine pharmazeutische Zusammensetzung enthaltend wenigstens eine Verbindung nach einem der Aspekte 1 bis 6 oder einem pharmakologisch verträglichen Salz und/oder Ester und/oder Komplex davon und wenigstens einen pharmakologisch verträglichen Exzipienten.
Aspekt 14 betrifft einen beschichteten Gegenstand dadurch gekennzeichnet dass die Oberfläche des Gegenstandes wenigstens eine Verbindung nach einem der Aspekte 1 bis 5 aufweist.
Aspekt 15 betrifft ein Verfahren zum Herstellen einer Verbindung einem der Aspekte 1 bis 5, wobei das Verfahren folgende Schritte umfasst:
   (A) Reduzieren eines substituierten Nitroanilins der Formel (9) zu einem substituierten o-Phenlendiamin der Formel (10), 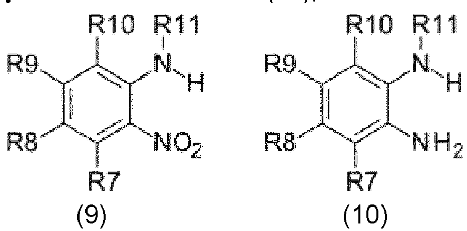
      wobei jeder der Reste R7 bis R10 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet und
      wobei der Rest R11 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
   (B) Kondensieren des in Schritt (A) erhaltenen substituierten o-Phenylendiamin der Formel (10) mit Alloxan oder dessen Hydrat unter Erhalt einer Verbindung mit der Formel (11): 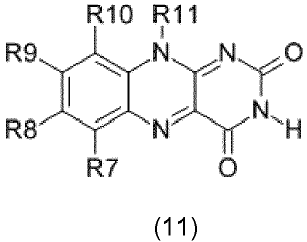
   (C) Optional Umsetzen der in Schritt (B) erhaltenen Verbindung der Formel (11) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(C(D)(E))ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (12): 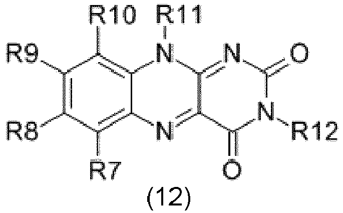 wobei der Rest R12 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das kein Stickstoffatom enthält, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
   (D) Optional Umsetzen der in Schritt (B) erhaltenen Verbindung der Formel (11) oder der in Schritt (C) erhaltenen Verbindung der Formel (12) mit Tosylchlorid, Mesylchlorid oder Iod, optional in Gegenwart eines Katalysators, und nachfolgend mit einer organischen Verbindung, die wenigstens ein neutrales, protonierbares Stickstoffatom und/oder b) wenigstens ein positiv geladenes Stickstoffatom enthält, wenn mindestes 1 Rest R7, R8, R9, R10, R11 oder R12 -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
      mit der Maßgabe, dass wenigstens 2 Reste R1, R2, R3, R4, R5 oder R6 ein organischer Rest mit
      a) wenigstens einem neutralen, protonierbaren Stickstoffatom, das nicht direkt an den Isoalloxazinring gebunden ist, und/oder
      b) wenigstens einem positiv geladenen Stickstoffatom, das nicht direkt an den Isoalloxazinring gebunden ist, bedeutet und
         wobei h jeweils eine ganze Zahl von 1 bis 20 und k, l jeweils unabhängig voneinander eine ganze Zahl von 0 bis 6 wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeutet bedeuten und wobei X jeweils ein organischer Rest mit a) wenigstens einem neutralen, protonierbaren Stickstoffatom, das nicht direkt an den Isoalloxazinring gebunden ist, und/oder b) wenigstens einem positiv geladenen Stickstoffatom, das nicht direkt an den Isoalloxazinring gebunden ist, bedeutet und Aryl jeweils einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet.
Aspekt 16 betrifft ein Verfahren zur Herstellung einer Verbindung nach einem der Aspekte 1 bis 5, wobei das Verfahren folgende Schritte umfasst:
   (A) Kondensieren eines Amins mit der Formel R11-NH₂ mit einem Chloruracil-Derivate der Formel (13), optional in Gegenwart eines Katalysators, vorzugsweise Lewis-Säure oder Broenstedt-Säure, unter Erhalt einer Verbindung mit der Formel (14): 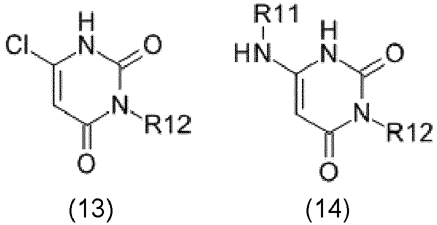 wobei jeder der Reste R11 oder R12 jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet,
   (B) Umsetzen der in Schritt (A) erhaltenen Verbindung der Formel (14) mit einer Nitrosoverbindung der Formel (15) unter Erhalt einer Verbindung der Formel (12): 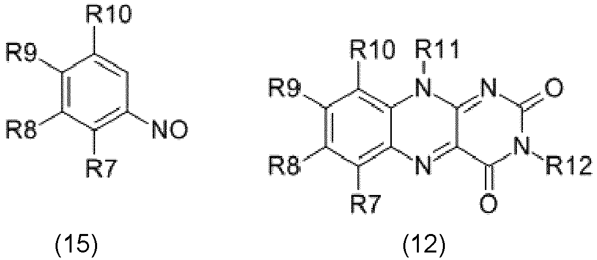 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
   (C) Optional Umsetzen der in Schritt (B) erhaltenen Verbindung der Formel (12) mit Tosylchlorid, Mesylchlorid oder Iod, optional in Gegenwart eines Katalysators, und nachfolgend mit einer organischen Verbindung, die wenigstens ein tertiäres Stickstoffatom enthält, umgesetzt wurde, wenn mindestes 1 Rest R7, R8, R9, R10, R11 oder R12 -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
      mit der Maßgabe, dass wenigstens 2 Reste R1, R2, R3, R4, R5 oder R6 ein organischer Rest mit
      a) wenigstens einem neutralen, protonierbaren Stickstoffatom, das nicht direkt an den Isoalloxazinring gebunden ist, und/oder
      b) wenigstens einem positiv geladenen Stickstoffatom, das nicht direkt an den Isoalloxazinring gebunden ist, bedeutet und
   wobei h jeweils eine ganze Zahl von 1 bis 20 und k, l jeweils unabhängig voneinander eine ganze Zahl von 0 bis 6 bedeuten wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X jeweils ein organischer Rest mit a) wenigstens einem neutralen, protonierbaren Stickstoffatom, das nicht direkt an den Isoalloxazinring gebunden ist, und/oder b) wenigstens einem positiv geladenen Stickstoffatom, das nicht direkt an den Isoalloxazinring gebunden ist, bedeutet und Aryl jeweils einen substituierten oder unsubstituierten Aromat mit 5 bis 20 C-Atomen oder einen substituierten oder unsubstituierten Heteroaromat, der kein Stickstoffatom enthält, mit 4 bis 20 C-Atomen bedeutet.
Aspekt 17 betrifft ein Verfahren zur Herstellung einer Verbindung nach einem der Aspekte 1 bis 5, wobei das Verfahren folgende Schritte umfasst:
   (A) Herstellen eines substituierten Anilins mit der Formel (122a) oder (122b) durch (a) Peptidkupplung/Reduktion an ein Anilin mit der Formel (121) oder (b) reduktive Aminierung eines Anilins mit der Formel (121) mit Aldehyden oder (c) Pd-katalysierte Kupplung von einem Halogenid der Formel (124) an ein Amine der Formel R11-NH₂ oder (d) Pd-katalysierte Kupplung von einem Amin der Formel (120) an ein Halogenid der Formel R11-NH₂
      a) 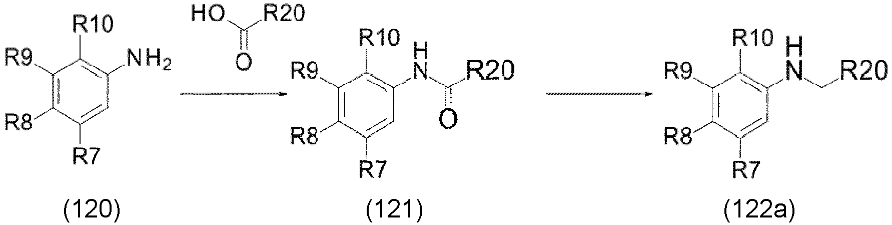
      b) 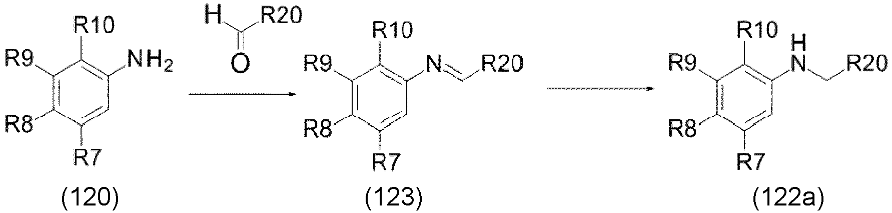
      c) 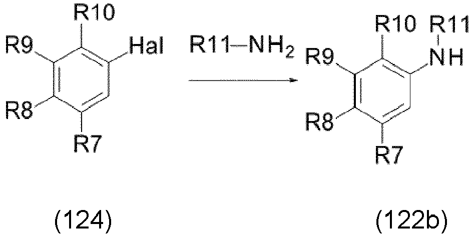
      d) 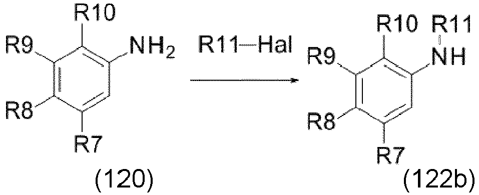
         wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))h-OH, -(C(D)(E))k-Aryl-(C(D)(E))l-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
         wobei der Rest R11 Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl, das kein Stickstoffatom enthält, mit 4 bis 20 C-Atomen, -(C(D)(E))ₕ-OH, -(C(D)(E))ₖ-Aryl-(C(D)(E))ₗ-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
         wobei der Rest R20 Wasserstoff, Alkyl mit 1 bis 19 C-Atomen, Alkenyl mit 2 bis 19 C-Atomen, Ether mit 1 bis 19 C-Atomen, Thioether mit 1 bis 19 C-Atomen, Cycloalkyl mit 3 bis 19 C-Atomen, Cycloalkenyl mit 3 bis 19 C-Atomen, Aryl mit 5 bis 19 C-Atomen, Heteroaryl mit 4 bis 19 C-Atomen, das keine N-Atome enthält, -(C(D)(E))ₕ₋₁-OH, -(C(D)(E))ₖ₋₁-Aryl-(C(D)(E))ₗ₋₁-OH oder einen organischen Rest, der a) wenigstens ein neutrales, protonierbares Stickstoffatom, das nicht direkt an den Isoalloxazinring gebunden ist, und/oder b) wenigstens ein positiv geladenes Stickstoffatom, das nicht direkt an den Isoalloxazinring gebunden ist, enthält, bedeutet, und
         wobei der Rest Hal Fluor, Chlor, Brom oder Iod bedeutet,
   (B) Umsetzen des in Schritt (A) erhaltenen substituierten Anilins mit der Formel (122a) mit Violursäure unter Erhalt einer Verbindung der Formel (11z): 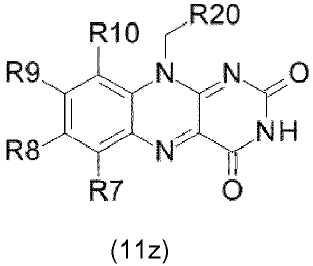 oder
      Umsetzen des in Schritt (A) erhaltenen substituierten Anilins mit der Formel (122b) mit Violursäure unter Erhalt einer Verbindung der Formel (11): 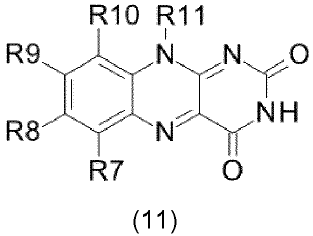
   (C) Optional Umsetzen der in Schritt (B) erhaltenen Verbindung der Formel (11z) mit Tosylchlorid, Mesylchlorid oder Iod, optional in Gegenwart eines Katalysators, und nachfolgend mit einer organischen Verbindung, die wenigstens ein tertiäres Stickstoffatom enthält, umgesetzt wird, wenn mindestes 1 Rest R7, R8, R9, R10, R11 oder R20 -(C(D)(E))ₕ-OH oder -(C(D)(E))ₖ-Aryl-(C(D)(E))ₗ-OH ist, unter Erhalt des 10H-Benzo[g]pteridin-2,4-dion-Derivats der Formel (12z): 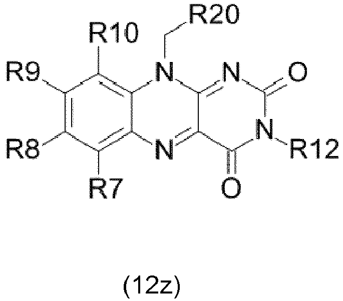 wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeuten,
      oder
      Umsetzen der in Schritt (B) erhaltenen Verbindung der Formel (11) mit Tosylchlorid, Mesylchlorid oder Iod, optional in Gegenwart eines Katalysators, und nachfolgend mit einer organischen Verbindung, die wenigstens ein tertiäres Stickstoffatom enthält, umgesetzt wird, wenn mindestes 1 Rest R7, R8, R9, R10, R11 oder R20 -(C(D)(E))ₕ-OH oder -(C(D)(E))ₖ-Aryl-(C(D)(E))ₗ-OH ist, unter Erhalt des 10H-Benzo[g]pteridin-2,4-dion-Derivats der Formel (12), 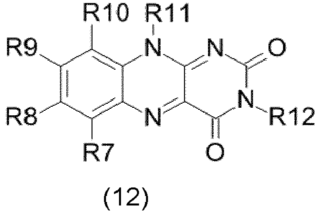 wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R(IX) Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, und
   mit der Maßgabe, dass
   I) nur 1 Rest R1, R2, R3, R4, R5 oder R6 ein organischer Rest mit:
      a) wenigstens zwei, vorzugsweise wenigstens drei, vorzugsweise wenigstens vier, weiter bevorzugt wenigstens fünf, neutralen, protonierbaren Stickstoffatomen, die nicht direkt an den Isoalloxazinring gebunden sind, oder
      b) wenigstens zwei, vorzugsweise wenigstens drei, vorzugsweise wenigstens vier, weiter bevorzugt wenigstens fünf, positiv geladenen, vorzugsweise quartären, Stickstoffatomen, die nicht direkt an den Isoalloxazinring gebunden sind, bedeutet oder
   II) nur 1 Rest R1, R2, R3, R4, R5 oder R6 ein organischer Rest mit:
      a) wenigstens einem, vorzugsweise wenigstens zwei, vorzugsweise wenigstens drei, vorzugsweise wenigstens vier, weiter bevorzugt wenigstens fünf, neutralen, protonierbaren Stickstoffatom(en), das (die) nicht direkt an den Isoalloxazinring gebunden ist (sind), und
      b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeutet
      oder
   III) wenigstens 2 Reste R1, R2, R3, R4, R5 oder R6 ein organischer Rest mit:
      a) wenigstens einem, vorzugsweise wenigstens zwei, vorzugsweise wenigstens drei, vorzugsweise wenigstens vier, weiter bevorzugt wenigstens fünf,neutralen, protonierbaren Stickstoffatom(en), das (die) nicht direkt an den Isoalloxazinring gebunden ist (sind), und/oder
      b) wenigstens einem, vorzugsweise wenigstens zwei, vorzugsweise wenigstens drei, vorzugsweise wenigstens vier, weiter bevorzugt wenigstens fünf, positiv geladenen, vorzugsweise quartären, Stickstoffatom(en), das (die) nicht direkt an den Isoalloxazinring gebunden ist (sind), bedeuten.
Aspekt 18 betrifft eine Verwendung einer Verbindung der Formel (1): 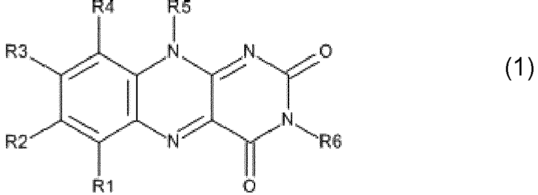 als Photosensibilisator, vorzugsweise bei der photodynamischen Inaktivierung von Mikroorganismen,
   wobei I) nur 1 Rest R1, R2, R3, R4, R5 oder R6 ein organischer Rest mit:
      a) wenigstens zwei neutralen, protonierbaren Stickstoffatomen, die nicht direkt an den Isoalloxazinring gebunden sind, oder
      b) wenigstens zwei positiv geladenen Stickstoffatomen, die nicht direkt an den Isoalloxazinring gebunden sind, bedeutet
      und wobei jeder der Reste R1, R2, R3 oder R4, der kein organischer Rest mit a) wenigstens zwei neutralen, protonierbaren Stickstoffatomen, die nicht direkt an den Isoalloxazinring gebunden sind, oder b) wenigstens zwei positiv geladenen Stickstoffatomen, die nicht direkt an den Isoalloxazinring gebunden sind, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
      wobei jeder der Reste R5 oder R6, der kein organischer Rest mit a) wenigstens zwei neutralen, protonierbaren Stickstoffatomen, die nicht direkt an den Isoalloxazinring gebunden sind, oder b) wenigstens zwei positiv geladenen Stickstoffatomen, die nicht direkt an den Isoalloxazinring gebunden sind, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
   wobei II) nur 1 Rest R1, R2, R3, R4, R5 oder R6 ein organischer Rest mit:
      a) wenigstens einem neutralen, protonierbaren Stickstoffatom, das nicht direkt an den Isoalloxazinring gebunden ist, und
      b) wenigstens einem positiv geladenen Stickstoffatom, das nicht direkt an den Isoalloxazinring gebunden ist, bedeutet
      und wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
      wobei jeder der Reste R5 oder R6, der kein organischer Rest mit a) wenigstens einem neutralen, protonierbaren Stickstoffatom, das nicht direkt an den Isoalloxazinring gebunden ist, und b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet oder
   wobei III) wenigstens 2 Reste R1, R2, R3, R4, R5 oder R6 ein organischer Rest mit:
      a) wenigstens einem neutralen, protonierbaren Stickstoffatom, das nicht direkt an den Isoalloxazinring gebunden ist, und/oder
      b) wenigstens einem positiv geladenen Stickstoffatom, das nicht direkt an den Isoalloxazinring gebunden ist, bedeutet und
      wobei jeder der Reste R1, R2, R3 oder R4, der kein organischer Rest mit a) wenigstens einem neutralen, protonierbaren Stickstoffatom, das nicht direkt an den Isoalloxazinring gebunden ist, und/oder b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet und
      wobei jeder der Reste R5 oder R6, der kein organischer Rest mit a) wenigstens einem neutralen, protonierbaren Stickstoffatom, das nicht direkt an den Isoalloxazinring gebunden ist, und/oder b) wenigstens einem positiv geladenen Stickstoffatom, das nicht direkt an den Isoalloxazinring gebunden ist, bedeutet, jeweils unabhängig voneinander gleich oder verschieden ist und Wasserstoff, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet.

## Patentansprüche

1. Verbindung mit der Formel (1): 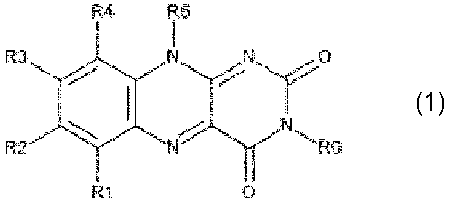 wobei nur 1 Rest R5 oder R6 ein organischer Rest mit:
a) wenigstens zwei neutralen, protonierbaren Stickstoffatomen, die nicht direkt an den Isoalloxazinring gebunden sind, oder
b) wenigstens zwei positiv geladenen, quartären, Stickstoffatomen, die nicht direkt an den Isoalloxazinring gebunden sind, bedeutet, wobei entweder
A) der Reste R5 ein nichtcyclischer Polyolrest der allgemeinen Formel -CH₂(CH(OZ))_{f}CH₍₃₋ₑ₎(OZ)ₑ bedeutet, wobei e 1, oder 2 ist und f eine ganze Zahl von 1 bis 10, vorzugsweise 1 bis 4, bedeutet und wobei Z entweder
ein Ester einer Am inocarbonsäure, vorzugsweise alpha-Aminocarbonsäure, mit 1 bis 20 C-Atomen, wobei jeweils wenigstes eine Aminogruppe mit Methylresten substituiert sein kann, oder
ein organischer Rest der Formel (2), (3) oder (4): 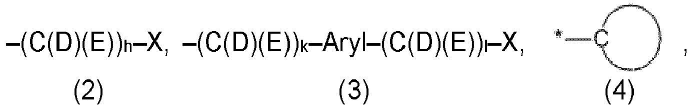 bedeutet,
wobei h eine ganze Zahl von 1 bis 20 und k und l jeweils unabhängig voneinander eine ganze Zahl von 0 bis 6 bedeuten und wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest ist, der a) wenigstens ein neutrales, protonierbares Stickstoffatom oder b) wenigstens ein positiv geladenes, quartäres Stickstoffatom enthält, und der Rest Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
wobei der Rest mit der Formel (4) einen Heteroaromaten, der über ein C-Atom des Heteroaromaten an den Isoalloxazinring gebunden ist und der a) wenigstens ein neutrales, protonierbares Stickstoffatom oder b) wenigstens ein positiv geladenes, quartäres Stickstoffatom enthält, bedeutet, und
wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet,
und wobei jeder der Reste R1, R2, R3 oder R4 jeweils unabhängig voneinander gleich oder verschieden ist und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl mit 4 bis 20 C-Atomen, das keine N-Atome enthält, bedeutet,
oder
B) wobei der Reste R6 ein organischer Rest der allgemeinen Formel (2), (3) oder (4): 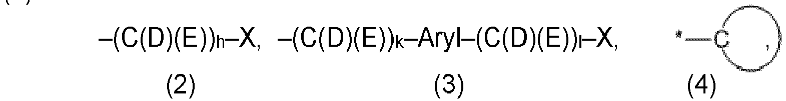 bedeutet,
wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet, und
wobei X ein Rest der allgemeinen Formel (2): 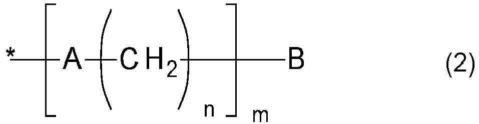 bedeutet, wobei A ein Sauerstoff- oder ein Schwefelatom ist und wobei n eine ganze Zahl von 1 bis 8 und m eine ganze Zahl von 0 bis 100 ist und wobei B ein Rest der Formel (3a), (3b), (4a), (4b), (5a) oder (5b): 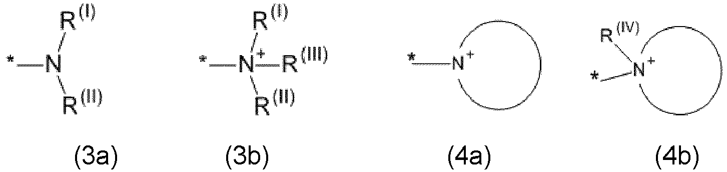 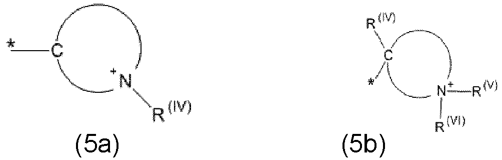 bedeutet und wobei der Reste R^{(I)} ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist, wobei jeder der Reste R^{(II)}, R^{(III)}, R^{(IV)}, R^{(V)} und R^{(VI)} unabhängig voneinander Wasserstoff, ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist und wobei jeder der vorgenannten Arylreste, heterocyclische Reste, Alkylreste, Alkenylreste, Hydroxyalkylreste, Etherreste, Thioetherreste und Alkylaminoreste mindestens mit einem Aminorest und/oder Alkylaminorest, der geradkettig oder verzweigt sein kann, mit 1 bis 20 C-Atomen und/oder Guanidinorest substituiert ist,
und wobei der Rest mit der Formel (4a) und der Rest mit der Formel (5a): 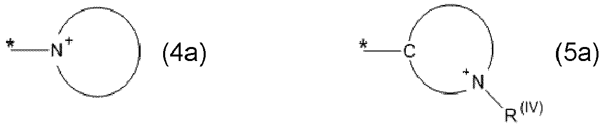 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 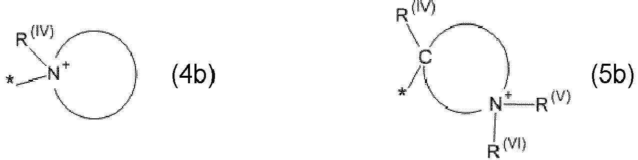 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, darstellt.
und wobei der Rest mit der Formel (4) ein Heteroaromat, der über ein C-Atom des Heteroaromaten an den Isoalloxazinring gebunden ist und der a) wenigstens zwei, vorzugsweise wenigstens drei, neutrale, protonierbare Stickstoffatome, die nicht direkt an den Isoalloxazinring gebunden sind, oder b) wenigstens zwei, vorzugsweise wenigstens drei, positiv geladene, vorzugsweise quartären, Stickstoffatome, die nicht direkt an den Isoalloxazinring gebunden sind, enthält, bedeutet und
wobei der Rest R5 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet oder
wobei der Rest R5 ein nichtcyclischer Polyolrest der allgemeinen Formel -CH₂(CH(OH))_{g}CH₂OH oder ein Ether, Ester oder Acetal davon bedeutet, wobei g eine ganze Zahl von 1 bis 10, vorzugsweise 1 bis 4, bedeutet, und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

2. Verbindung nach Anspruch 1, wobei Z ein Ester einer Aminocarbonsäure ist, der sich von einer Aminocarbonsäure mit 1 bis 20 C-Atomen ableitet, die aus der Gruppe, die aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin, L-Homoserin, Ornithin, N5-(Aminocarbonyl)-L-ornithin, L-(-)-Carnitin, alpha-Phenylglycin, L-3,4-Dihydroxyphenylalanin, 3,6-Diaminohexansäure, N-(Aminoiminomethyl)-N-methyl-glycin, gamma-Aminobuttersäure, L-5-Hydroxytryptophan, Norleucin und 2,6-Diaminopimelinsäure, weiter bevorzugt Arginin, Glycin, Lysin, Ornithin, 3,6-Diaminohexansäure und gamma-Aminobuttersäure, noch weiter bevorzugt Glycin und Lysin, besteht, ausgewählt wird, wobei vorzugsweise jeweils wenigstes eine Aminogruppe mit Methylresten substituiert sein kann.

3. Verbindung nach Anspruch 1, wobei
A) der Reste R5 ein nichtcyclischer Polyolrest der allgemeinen Formel -CH₂(CH(OZ))_{f}CH₍₃₋ₑ₎(OZ)ₑ bedeutet, wobei e 1, oder 2 ist und f eine ganze Zahl von 1 bis 10, vorzugsweise 1 bis 4, bedeutet und wobei Z ein organischer Rest der Formel (2), (3) oder (4): 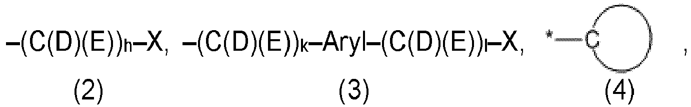 ist,
wobei h eine ganze Zahl von 1 bis 20 und k und I jeweils unabhängig voneinander eine ganze Zahl von 0 bis 6 bedeuten und wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C (=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und
wobei X einen Rest der allgemeinen Formel (2): 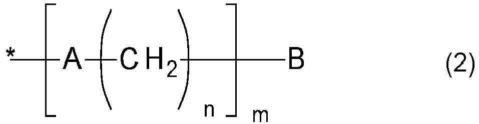 bedeutet, wobei A ein Sauerstoff- oder ein Schwefelatom ist und wobei n eine ganze Zahl von 1 bis 8 und m eine ganze Zahl von 0 bis 100 ist und wobei B ein Rest der Formel (3a),(3b), (4a), (4b), (5a) oder (5b): 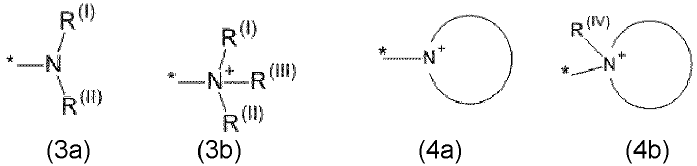 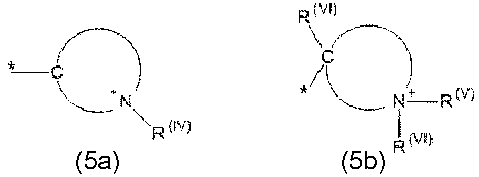 ist und wobei der Reste R^{(I)} ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist, wobei jeder der Reste R^{(II)}, R^{(III)}, R^{(IV)}, R^{(V)} und R^{(VI)} unabhängig voneinander Wasserstoff, ein Arylrest mit 5 bis 20 C-Atomen, ein heterocyclischer Rest mit 5 bis 20 C-Atomen, ein Alkylrest, der geradkettig oder verzweigt sein kann, mit 1 bis 20 C-Atomen, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, ein Etherrest, der geradkettig oder verzweigt sein kann, mit 2 bis 20 C-Atomen, ein Thioetherrest, der geradkettig oder verzweigt sein kann, mit 2 bis 20 C-Atomen, oder ein Alkylaminorest, der geradkettig oder verzweigt sein kann, mit 1 bis 20 C-Atomen ist und wobei jeder der vorgenannten Arylreste, heterocyclische Reste, Alkylreste, Alkenylreste, Hydroxyalkylreste, Etherreste, Thioetherreste und Alkylaminoreste mindestens mit einem, vorzugsweise mindestens zwei, vorzugsweise mindestens drei, vorzugsweise mindestens vier, weiter bevorzugt mindestens fünf, Aminorest(en) und/oder Alkylaminorest(en), der (die) geradkettig oder verzweigt sein kann (können), mit 1 bis 20 C-Atomen, beispielsweise Aminomethyl, 2-Aminoethyl, Dimethylaminomethyl oder (Trimethylammonio)methyl, und/oder Guanidinorest substituiert ist,
und wobei der Rest mit der Formel (4a) und der Rest mit der Formel (5a): 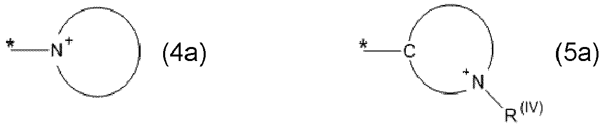 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, wobei 1 Stickstoffatom eine Doppelbindung ausbildet, darstellt und wobei der Rest mit der Formel (4b) und der Rest mit der Formel (5b): 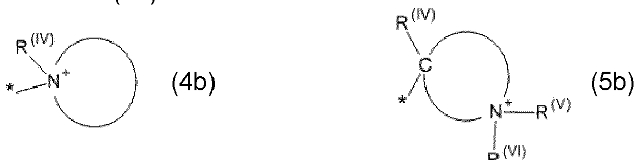 einen substituierten oder unsubstituierten heterocyclischen Rest mit 5 bis 7 Ringatomen, die 2 bis 4 Stickstoffatome und mindestens 1 Kohlenstoffatom sowie optional 1 oder 2 Sauerstoff- oder Schwefelatom umfassen, darstellt.

4. Verbindung nach einem der Ansprüche 1 oder 3, wobei die Reste R^{(II)}, R^{(III)}, R^{(IV)}, R^{(V)} und R^{(VI)} unabhängig voneinander Wasserstoff oder ein Rest der Formel (6) bis (9): 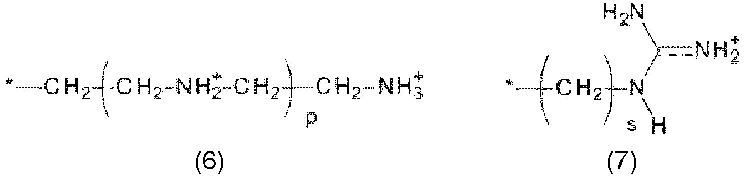 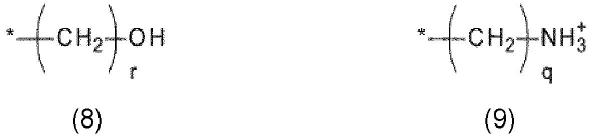 sind und wobei der Rest R^{(I)} ein Rest der Formel (6) bis (9) ist, wobei jeweils p eine ganze Zahl von 1 bis 10, vorzugsweise von 1 bis 7, weiter bevorzugt von 1 bis 3, bedeutet und wobei s, r, q jeweils unabhängig voneinander eine ganze Zahl von 1 bis 20, vorzugsweise von 1 bis 8, weiter bevorzugt von 1 bis 4, bedeutet.

5. Verbindung nach einem der Ansprüche 1 oder 3, wobei die Reste R^{(II)}, R^{(III)}, R^{(IV)}, R^{(V)} und R^{(VI)} unabhängig voneinander Wasserstoff oder ein Polyaminrest der allgemeinen Formel -[(CH₂)ₐ-N(R^{(VII)})-(CH₂)_{b}-]_{c}NH_{(3-g)}(R^{(VIII)})_{g}⁺ sind, und der Rest R^{(I)} ein Polyaminrest der allgemeinen Formel -[(CH₂)ₐ-N(R^{(VII)}-(CH₂)_{b}-]_{c}NH_{(3-g)}(R^{(VIII)})_{g}⁺ ist, wobei jeweils c eine ganze Zahl von 1 bis 10, vorzugsweise von 1 bis 7, weiter bevorzugt von 1 bis 3, bedeutet, a und b jeweils unabhängig voneinander eine ganze Zahl von 1 bis 10, vorzugsweise von 1 bis 4, weiter bevorzugt von 2 bis 3, bedeuten, g 0, 1, 2 oder 3 ist und R^{(VII)} und R^{(VIII)} unabhängig voneinander Wasserstoff, Methyl oder Ethyl bedeuten.

6. Verbindung nach einem der Ansprüche 1 bis 5 oder ein pharmakologisch verträgliches Salz und/oder Ester und/oder Komplex davon zur Anwendung als Photosensibilisator zur photodynamischen Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden, bei der photodynamischen Therapie.

7. Verbindung zur Anwendung nach Anspruch 6 bei der Desinfektion und/oder Dekolonisierung von Haut- oder Weichteiloberflächen.

8. Verbindung zur Anwendung nach Anspruch 6 bei der Prophylaxe und/oder Behandlung einer infektiösen Erkrankung.

9. Verbindung zur Anwendung nach Anspruch 8 bei der Prophylaxe und/oder Behandlung einer infektiösen Erkrankung des Ohrs, der oberen Luftwege, der Mundhöhle, des Rachens, des Kehlkopfes, der unteren Luftwege und/oder der Speiseröhre.

10. Verbindung zur Anwendung nach einem der Ansprüche 8 oder 9 bei der Prophylaxe und/oder Behandlung einer infektiösen Erkrankung des Zahngewebes und/oder des Zahnhalteapparates.

11. Verbindung zur Anwendung nach Anspruch 8 bei der bei der Prophylaxe und/oder Behandlung einer infektiösen Hautkrankheit

12. Nicht-medizinische Verwendung einer Verbindung nach einem der Ansprüche 1 bis 5 bei der Oberflächenreinigung und/oder -beschichtung.

13. Nicht-medizinische Verwendung einer Verbindung nach einem der Ansprüche 1 bis 5 bei der Reinigung von Zahnersatz und/oder Zahnspangen.

14. Nicht-medizinische Verwendung einer Verbindung nach einem der Ansprüche 1 bis 5 bei der Oberflächenreinigung und/oder -beschichtung von Medizinprodukten, Lebensmittelverpackungen oder Hygieneartikeln.

15. Pharmazeutische Zusammensetzung enthaltend wenigstens eine Verbindung nach einem der Ansprüche 1 bis 5 oder einem pharmakologisch verträglichen Salz und/oder Ester und/oder Komplex davon und wenigstens einen pharmakologisch verträglichen Exzipienten.

16. Beschichteter Gegenstand **dadurch gekennzeichnet, dass** die Oberfläche des Gegenstandes wenigstens eine Verbindung nach einem der Ansprüche 1 bis 5 aufweist.
